# EUROPÄISCHE PATENTSCHRIFT

(11) **EP 3 484 857 B1**
(45) Veröffentlichungstag und Bekanntmachung des Hinweises auf die Patenterteilung: **03.03.2021**
(21) Anmeldenummer: 17737619.1
(22) Anmeldetag: 17.07.2017
(51) Int. Cl.: C07D 403/06, C07D 403/14, C09K 3/10, C07D 251/04

(54) **KATALYSATOR FÜR HÄRTBARE ZUSAMMENSETZUNGEN ENTHALTEND HEXAHYDROTRIAZIN-STRUKTUREINHEITEN**
CATALYST FOR CURABLE COMPOSITIONS COMPRISING HEXAHYDROTRIAZIN STRUCTURAL UNITS
CATALYSEUR POUR COMPOSITIONS DURCISSABLES CONTENANT DES UNITES DE STRUCTURE D'HEXAHYDROTRIAZINE

(30) Priorität: 18.07.2016 EP 16179945
(43) Veröffentlichungstag der Anmeldung: 22.05.2019
(73) Patentinhaber: Sika Technology AG, 6340 Baar (CH)
(72) Erfinder: CANNAS, Rita, 8600 Dübendorf (CH); BURCKHARDT, Urs, 8049 Zürich (CH)
(74) Vertreter: Sika Patent Attorneys
(86) Internationale Anmeldenummer: PCT/EP2017/068032
(87) Internationale Veröffentlichungsnummer: WO 2018/015344

(56) Entgegenhaltungen:
- EP-A1- 0 007 440
- EP-A2- 2 268 743
- DE-A1- 3 140 635

## Beschreibung

### Technisches Gebiet

Die Erfindung betrifft Amidin- oder Guanidingruppen aufweisende Hexahydrotriazine und deren Verwendung als Katalysator für härtbare Zusammensetzungen, insbesondere auf der Basis von Silangruppen aufweisenden Polymeren.

### Stand der Technik

Härtbare Zusammensetzungen spielen eine bedeutende Rolle in vielen technischen Anwendungen, beispielsweise als Klebstoffe, Dichtstoffe oder Beschichtungen. Ihre Aushärtung wird durch Vernetzungsreaktionen bewirkt, welche über Reaktivgruppen wie beispielsweise Silangruppen, Isocyanatgruppen, Epoxidgruppen, Hydroxylgruppen oder Aminogruppen ablaufen, wobei diese nach einem Mischvorgang, durch Erwärmen oder durch Kontakt mit Feuchtigkeit mit sich selbst oder untereinander reagieren und so die in der Zusammensetzung enthaltenen Aufbaukomponenten kovalent zu einem polymeren Netzwerk verbinden. Zur Beschleunigung solcher Vernetzungsreaktionen werden häufig Katalysatoren eingesetzt. Sehr oft handelt es sich dabei um toxikologisch bedenkliche Stoffe, welche eine potentielle Gefährdung von Verarbeiter und Umwelt darstellen, insbesondere nach der Aushärtung der Zusammensetzung, wenn der Katalysator oder dessen Abbauprodukte durch Ausgasen, Migration oder Auswaschen freigesetzt werden.

Bei Raumtemperatur härtbare Zusammensetzungen auf Basis von Silangruppen aufweisenden Polymeren sind ausgeprägt mit dieser Problemstellung konfrontiert. Silangruppen aufweisende Polymere sind dabei insbesondere Polyorganosiloxane, welche gemeinhin als "Silicone" bzw. "Siliconkautschuke" bezeichnet werden, und Silangruppen aufweisende organische Polymere, welche auch als "silanfunktionelle Polymere", "silanmodifizierte Polymere" (SMP) oder "silanterminierte Polymere" (STP) bezeichnet werden. Ihre Vernetzung verläuft über die Kondensation von Silanolgruppen unter Ausbildung von Siloxanbindungen und wird klassischerweise mittels Organozinn-Verbindungen, wie insbesondere Dialkylzinn(IV)carboxylaten, katalysiert. Diese zeichnen sich durch eine sehr hohe Aktivität in Bezug auf die Silanol-Kondensation aus und sind sehr hydrolysebeständig; allerdings sind sie gesundheitsschädlich und stark wassergefährdend. Oft werden sie mit weiteren Katalysatoren kombiniert, hauptsächlich mit basischen Verbindungen wie insbesondere Aminen, welche vor allem die vorgeschaltete Hydrolyse der Silangruppen beschleunigen. Aufgrund einer stärkeren Gewichtung von EHS-Aspekten durch Berufsverbände und Verbraucher sowie schärferer staatlicher Regulierung werden seit einiger Zeit vermehrt Anstrengungen unternommen, die Organozinn-Verbindungen durch andere, weniger toxische Katalysatoren zu ersetzen. So wurden etwa Organotitanate, -zirkonate und -aluminate als alternative Metallkatalysatoren beschrieben. Diese haben aber zumeist eine geringere katalytische Aktivität in Bezug auf die Silanol-Kondensation und bewirken eine deutlich langsamere Vernetzung. Wegen ihrer mangelnden Hydrolysestabilität können sie beim Lagern der Zusammensetzung durch Restfeuchte der Inhaltsstoffe einen Grossteil ihrer Aktivität verlieren, wodurch sich die Aushärtung stark verlangsamt oder ganz zum Erliegen kommt.

Eine weitere bekannte Alternative zu Organozinn-Verbindungen stellen stark basische Stickstoff-Verbindungen aus der Klasse der Amidine und Guanidine dar, welche in Kombination mit den erwähnten Metall-Katalysatoren oder auch allein eingesetzt werden können. Viele der gebräuchlichen Amidin- und Guanidin-Katalysatoren, wie insbesondere 1,8-Diazabicyclo[5.4.0]undec-7-en (DBU) und 1,1,3,3-Tetramethylguanidin (TMG), sind allerdings leichtflüchtige und geruchsintensive sowie ebenfalls gesundheitsschädliche und umweltgefährdende Stoffe. Ausserdem neigen sie dazu, aufgrund geringer Verträglichkeit in der Zusammensetzung zu migrieren und dadurch Separation, Ausschwitzen oder Substratverschmutzung zu verursachen.

Weitere Amidin- und/oder Guanidin-Katalysatoren sind bekannt aus WO 2009/118307, WO 2010/043353, WO 2015/158859, WO 2015/158860, WO 2015/158863 und WO 2015/193208. Diese Katalysatoren sind aber bezüglich katalytischer Aktivität und/oder Verträglichkeit in unterschiedlichen PolymerSystemen und der Variabilität ihrer Eigenschaften noch verbesserungsfähig. Aus DE 1 769 043 sind Hexahydrotriazin-Katalysatoren für die Herstellung von Polyurethanschäumen bekannt.

Aus EP 0 872 505 sind Hexahydrotriazin-Katalysatoren für Epoxidharz-Zusammensetzungen bekannt EP 0 007 440 offenbart Hexahydrotriazincarboxylate, Verfahren zu ihrer Herstellung und ihre Verwendung als Katalysatoren zur Herstellung von Polyisocyanuratkunststoffen.

Diese bekannten Hexahydrotriazin-Katalysatoren weisen aber eine vergleichsweise geringe Aktivität auf, insbesondere für die Vernetzung von Silangruppen.

### Darstellung der Erfindung

Aufgabe der vorliegenden Erfindung ist es daher, einen Katalysator für die Vernetzung von härtbaren Zusammensetzungen, insbesondere auf der Basis von Silangruppen aufweisenden Polymeren, bereitzustellen, welcher eine hohe katalytische Aktivität für die Vernetzungsreaktion besitzt, einen geringen Dampfdruck und eine hohe Verträglichkeit mit der Zusammensetzung aufweist und möglichst geruchsarm und wenig toxisch ist.

Diese Aufgabe wird durch eine Verbindung enthaltend mindestens eine Hexahydrotriazin-Einheit der Formel (I), wie in Anspruch 1 beschrieben, gelöst. Sie enthält mindestens eine Amidin- oder Guanidin-Gruppe und ist bei Raumtemperatur geruchlos, nicht flüchtig und weitgehend untoxisch. Sie zeigt eine hohe katalytische Aktivität für die Reaktion vieler Verbindungen mit polymerisationsfähigen Reaktivgruppen, insbesondere für Zusammensetzungen auf der Basis von Silangruppen aufweisenden Polymeren. Die hohe Aktivität ist dabei besonders überraschend, würde man doch aufgrund des relativ hohen Molekulargewichtes und der starken intermolekularen Wechselwirkungen über Wasserstoffbrücken eine verminderte Aktivität im Vergleich mit kleineren, unpolareren und somit mobileren Amidinen oder Guanidinen erwarten.

Die Verbindung enthaltend mindestens eine Hexahydrotriazin-Einheit der Formel (I) ist in einem einfachen Verfahren aus gut erhältlichen Grundstoffen herstellbar, wobei auf überraschend einfache Weise sehr unterschiedliche Strukturelemente - wie zum Beispiel Polyalkylenoxid-Ketten, Polysiloxan-Ketten, Silangruppen, Guanidin- und/oder Amidin-Gruppen von gleicher oder verschiedener Struktur - über den Hexahydrotriazin-Ring miteinander verbunden werden können. Somit lassen sich auf einfache Weise massgeschneiderte Amidin- und/oder Guanidin-Katalysatoren herstellen, welche bezüglich Verträglichkeit und katalytischer Aktivität auf unterschiedliche funktionelle Polymere abgestimmt sind.

Härtbare Zusammensetzungen enthaltend die Verbindung mit mindestens einer Hexahydrotriazin-Einheit der Formel (I) sind wenig toxisch und neigen weder vor noch nach der Aushärtung zu migrationsbedingten Fehlern wie Separation, Ausschwitzen oder Substratverschmutzung. Sie zeigen eine gute Lagerstabilität und sind dank geringem Geruch angenehm applizierbar. Nach der Applikation bauen sie überraschend schnell Festigkeit auf und bilden mechanisch hochwertige, beständige sowie emissionsarme Materialien aus. Weitere Aspekte der Erfindung sind Gegenstand weiterer unabhängiger Ansprüche. Besonders bevorzugte Ausführungsformen der Erfindung sind Gegenstand der abhängigen Ansprüche.

### Wege zur Ausführung der Erfindung

Gegenstand der Erfindung ist eine Verbindung enthaltend mindestens eine Hexahydrotriazin-Einheit der Formel (I), 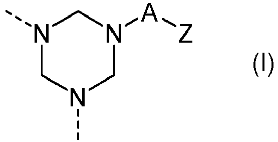 wobei
A für einen zweiwertigen Kohlenwasserstoff-Rest, welcher gegebenenfalls Heteroatome enthält, steht, und
Z für eine über ein Stickstoffatom gebundene Amidin- oder Guanidingruppe steht,
wobei der Hexahydrotriazin-Ring und die Gruppe Z durch eine Kette von mindestens zwei C-Atomen voneinander getrennt sind.

Eine gestrichelte Linie in den Formeln in diesem Dokument stellt jeweils die Bindung zwischen einem Substituenten und dem zugehörigen Molekülrest dar. Als "primäre Aminogruppe" bzw. "primärer Amin-Stickstoff" wird eine Aminogruppe bzw. deren Stickstoffatom bezeichnet, die bzw. das an einen einzigen organischen Rest gebunden ist und zwei Wasserstoffatome trägt; als "sekundäre Aminogruppe" bzw. "sekundärer Amin-Stickstoff" wird eine Aminogruppe bzw. deren Stickstoffatom bezeichnet, die bzw. das an zwei organische Reste, welche auch gemeinsam Teil eines Rings sein können, gebunden ist und ein Wasserstoffatom trägt; und als "tertiäre Aminogruppe" bzw. "tertiärer Amin-Stickstoff" wird eine Aminogruppe bzw. deren Stickstoffatom bezeichnet, die bzw. das an drei organische Reste, welche auch zu zweit oder zu dritt Teil eines oder mehrerer Ringe sein können, gebunden ist und kein Wasserstoffatom trägt.

Als "Siloxan-Rest" wird ein Rest enthaltend mindestens eine Siloxanbindung Si-O-Si bezeichnet.

Als "Polysiloxan-Rest" wird ein Siloxan-Rest enthaltend mehrere Siloxanbindungen in Folge, also Si-(O-Si)ₛ-Einheiten mit s = 2 oder mehr, bezeichnet. Dabei wird eine (O-Si)-Einheit als "Siloxan-Einheit" bezeichnet.

Der Begriff "Silangruppe" bezeichnet eine an einen organischen Rest oder an einen Polysiloxan-Rest gebundene Silylgruppe mit einer bis drei, insbesondere zwei oder drei, hydrolysierbaren Substituenten am Siliciumatom. Besonders gebräuchliche hydrolysierbare Substituenten sind Alkoxy-Reste. Diese Silangruppen werden auch als "Alkoxysilangruppen" bezeichnet. Silangruppen können auch in partiell oder vollständig hydrolysierter Form vorhanden sein.

Als "Hydroxysilan", "Isocyanatosilan", "Aminosilan" bzw. "Mercaptosilan" werden Organoalkoxysilane (Silane) bezeichnet, die am organischen Rest zusätzlich zur Silangruppe eine oder mehrere Hydroxyl-, Isocyanato-, Amino- bzw. Mercaptogruppen aufweisen.

Der Begriff "organisches Polymer" umfasst ein Kollektiv von chemisch einheitlichen, sich aber in Bezug auf Polymerisationsgrad, Molmasse und Kettenlänge unterscheidenden Makromolekülen, das durch eine Polyreaktion (Polymerisation, Polyaddition, Polykondensation) hergestellt wurde und im PolymerRückgrat mehrheitlich Kohlenstoffatome aufweist, sowie Umsetzungsprodukte eines solchen Kollektivs von Makromolekülen. Polymere mit einem Polyorganosiloxan-Rückgrat (gemeinhin als "Silicone" bezeichnet) stellen keine organischen Polymere im Sinne des vorliegenden Dokuments dar.

Der Begriff "Silangruppen aufweisender Polyether" umfasst auch Silangruppen aufweisende organische Polymere, welche zusätzlich zu Polyether-Einheiten auch Urethangruppen, Harnstoffgruppen oder Thiourethangruppen enthalten können. Solche Silangruppen aufweisende Polyether können auch als "Silangruppen aufweisende Polyurethane" bezeichnet werden.

Unter "Molekulargewicht" versteht man im vorliegenden Dokument die molare Masse (in Gramm pro Mol) eines Moleküls oder eines Teils eines Moleküls, auch als "Rest" bezeichnet. Als "mittleres Molekulargewicht" wird das Zahlenmittel Mₙ einer oligomeren oder polymeren Mischung von Molekülen oder Resten bezeichnet, welches üblicherweise mittels Gelpermeationschromatographie (GPC) gegen Polystyrol als Standard bestimmt wird.

Als "lagerstabil" oder "lagerfähig" wird eine Substanz oder eine Zusammensetzung bezeichnet, wenn sie bei Raumtemperatur in einem geeigneten Gebinde während längerer Zeit, typischerweise mindestens 3 Monaten bis zu 6 Monaten und mehr, aufbewahrt werden kann, ohne dass sie sich in ihren Anwendungs- oder Gebrauchseigenschaften, insbesondere der Viskosität und der Vernetzungsgeschwindigkeit, durch die Lagerung in einem für ihren Gebrauch relevanten Ausmass verändert.

Als "Raumtemperatur" wird eine Temperatur von ca. 23°C bezeichnet.

Die Verbindung enthaltend mindestens eine Hexahydrotriazin-Einheit der Formel (I) kann bezüglich ihrer Amidin- bzw. Guanidingruppen auch in tautomerer Form vorliegen. Alle möglichen Tautomerformen werden als gleichwertig angesehen. Weiterhin kann sie auch in protonierter Form vorliegen. Ebenfalls kann sie in komplexierter Form vorliegen, insbesondere mit Kationen von Zink, Eisen oder Molybdän.

Bevorzugt steht A für einen zweiwertigen Kohlenwasserstoff-Rest mit 2 bis 50, insbesondere 2 bis 20, C-Atomen, welcher gegebenenfalls Heteroatome in Form von Ether-Sauerstoff oder sekundärem oder tertiärem Amin-Stickstoff oder Siloxan-Einheiten enthält.

Besonders bevorzugt ist A ausgewählt aus der Gruppe bestehend aus 1,2-Ethylen, 1,2-Propylen, 1,3-Propylen, 1,3-Pentylen, 1,5-Pentylen, 2-Methyl-1,5-pentylen, 1,6-Hexylen, 2,2(4),4-Trimethyl-1,6-hexamethylen, 1,8-Octylen, 1,10-Decylen, 1,12-Dodecylen, (1,5,5-Trimethylcyclohexan-1-yl)methan-1,3, 1,3-Cyclohexylen-bis(methylen), 1,4-Cyclohexylen-bis(methylen), 1,3-Phenylen- bis(methylen), 2- und/oder 4-Methyl-1,3-cyclohexylen, N-Methyl-4-aza-1,7-heptylen, N-Ethyl-4-aza-1,7-heptylen, Piperazin-1,4-diyl-bis(1,2-ethylen), Piperazin-1,4-diyl-bis(1,3-propylen), 3-Oxa-1,5-pentylen, 3,6-Dioxa-1,8-octylen, 4,7-Dioxa-1,10-decylen, α,ω-Polyoxypropylen mit einem mitleren Molekulargewicht im Bereich von etwa 180 bis 500 g/mol und α,ω-(1,3-Propylen)polydimethylsiloxan mit einem mittleren Molekulargewicht im Bereich von etwa 350 bis 2'000 g/mol.

Ganz besonders bevorzugt steht A für einen zweiwertigen Kohlenwasserstoff-Rest mit 2 bis 20 C-Atomen, welcher gegebenenfalls Heteroatome in Form von Ether-Sauerstoff oder tertiärem Amin-Stickstoff oder Siloxan-Einheiten enthält.

Insbesondere bevorzugt steht A für 1,2-Ethylen, 1,2-Propylen, 1,3-Propylen, 2-Methyl-1,5-pentylen, 1,6-Hexylen, 2,2(4),4-Trimethyl-1,6-hexamethylen, 1,8-Octylen, 1,10-Decylen, 1,12-Dodecylen, (1,5,5-Trimethylcyclohexan-1-yl)methan-1,3, 1,3-Cyclohexylen-bis(methylen), 1,4-Cyclohexylen-bis(methylen), 1,3-Phenylen-bis(methylen) oder 2- und/oder 4-Methyl-1,3-cyclohexylen. Eine solche Verbindung enthaltend mindestens eine Hexahydrotriazin-Einheit der Formel (I) ist besonders gut zugänglich und besonders aktiv als Katalysator.

Weiterhin insbesondere bevorzugt steht A für α,ω-Polyoxypropylen mit einem mittleren Molekulargewicht im Bereich von etwa 180 bis 500 g/mol. Eine solche Verbindung enthaltend mindestens eine Hexahydrotriazin-Einheit der Formel (I) ist besonders gut verträglich in härtbaren Zusammensetzungen basierend auf Polyether-Polymeren, insbesondere Silangruppen aufweisenden Polyethern.

Weiterhin insbesondere bevorzugt steht A für α,ω-(1,3-Propylen)polydimethylsiloxan mit einem mittleren Molekulargewicht im Bereich von etwa 350 bis 2'000 g/mol. Eine solche Verbindung enthaltend mindestens eine Hexahydrotriazin-Einheit der Formel (I) ist besonders gut verträglich in härtbaren Zusammensetzungen basierend auf Polyorganosiloxan-Polymeren.

Z steht bevorzugt für 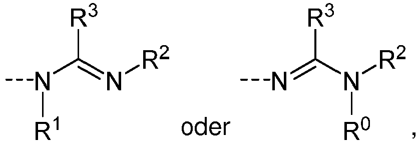 wobei
R⁰ für einen Wasserstoff-Rest oder für einen Alkyl- oder Cycloalkyl- oder Aralkyl-Rest mit 1 bis 8 C-Atomen steht,
R¹ für einen Wasserstoff-Rest oder für einen Alkyl- oder Cycloalkyl- oder Aralkyl-Rest mit 1 bis 8 C-Atomen oder zusammen mit R² für R⁶ steht,
R² für einen Wasserstoff-Rest oder für einen Alkyl-, Cycloalkyl- oder Aralkyl-Rest mit 1 bis 18 C-Atomen, welcher gegebenenfalls Ether-Sauerstoff oder tertiären Amin-Stickstoff enthält, oder zusammen mit R¹ für R⁶ steht,
R³ für -NR⁴R⁵ oder für einen Wasserstoff-Rest oder für einen Alkyl- oder Cycloalkyl- oder Aralkyl-Rest mit 1 bis 12 C-Atomen steht,
wobei
R⁴ und R⁵ unabhängig voneinander jeweils für einen Wasserstoff-Rest oder für einen Alkyl-, Cycloalkyl- oder Aralkyl-Rest mit 1 bis 18 C-Atomen, welcher gegebenenfalls Ether-Sauerstoff oder tertiären Amin-Stickstoff enthält, stehen,
R⁶ für einen gegebenenfalls substituierten 1,2-Ethylen-, 1,3-Propylen- oder 1,4-Butylen-Rest mit 2 bis 12 C-Atomen steht,
R² und R⁰ auch zusammen für einen Alkylen-Rest mit 3 bis 6 C-Atomen, welcher gegebenenfalls Ether-Sauerstoff oder tertiären Amin-Stickstoff enthält, stehen können,
R² und R³ auch zusammen für einen Alkylen-Rest mit 3 bis 6 C-Atomen stehen können,
R⁴ und R⁵ auch zusammen für einen Alkylen-Rest mit 4 bis 7 C-Atomen, welcher gegebenenfalls Ether-Sauerstoff oder tertiären Amin-Stickstoff enthält enthält, stehen können, und
R² und R⁵ auch zusammen für einen Alkylen-Rest mit 2 bis 12 C-Atomen stehen können.
R¹ steht bevorzugt für einen Wasserstoff-Rest oder für einen Alkyl-Rest mit 1 bis 4 C-Atomen oder zusammen mit R² für R⁶.
R² steht bevorzugt für einen Alkyl-, Cycloalkyl- oder Aralkyl-Rest mit 1 bis 12 C-Atomen, insbesondere 1 bis 8 C-Atomen, welcher gegebenenfalls einen Ether-Sauerstoff oder tertiären Amin-Stickstoff enthält, oder zusammen mit R¹ für R⁶.

In einer Ausführungsform steht Z für eine Amidin-Gruppe. In diesem Fall steht R³ für einen Wasserstoff-Rest oder für einen Alkyl- oder Cycloalkyl- oder Aralkyl-Rest mit 1 bis 12 C-Atomen.

Eine Amidin-Gruppen aufweisende Verbindung enthaltend mindestens eine Hexahydrotriazin-Einheit weist den Vorteil auf, dass sie weniger empfindlich ist auf Hydrolyse durch vorhandene Feuchtigkeit, bei der Verwendung als Katalysator eine nicht ganz so hohe katalytische Aktivität besitzt und deshalb in etwas höherer Menge eingesetzt werden kann und somit weniger anfällig ist für Störungen durch andere Bestandteile einer Zusammensetzung, insbesondere den darin enthaltenen Verunreinigungen.

Dabei steht R³ bevorzugt für einen Wasserstoff-Rest oder für einen Alkyl-Rest mit 1 bis 4 C-Atomen, besonders bevorzugt für einen Wasserstoff-Rest oder Methyl-Rest.

Dabei steht R¹ bevorzugt für einen Alkyl-Rest mit 1 bis 4 C-Atomen oder zusammen mit R² für R⁶. Besonders bevorzugt stehen R¹ und R² zusammen für R⁶.

Besonders bevorzugt steht Z somit für eine Amidingruppe, bei welcher R³ für einen Wasserstoff-Rest oder für einen Alkyl-Rest mit 1 bis 4 C-Atomen, insbesondere für einen Wasserstoff-Rest oder Methyl-Rest, steht, und R¹ und R² zusammen für R⁶ stehen.

Z stellt somit besonders bevorzugt eine Amidin-Gruppe der Formel 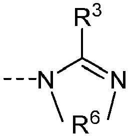 dar. Eine solche Amidingruppe ist besonders einfach zugänglich und zeigt eine hohe katalytische Aktivität.

R⁶ weist bevorzugt 2 bis 6 C-Atome auf.

R⁶ steht bevorzugt für 1,2-Ethylen, 1,2-Propylen, 1,3-Propylen, 2-Methyl-1,2-propylen, 2,2-Dimethyl-1,3-propylen, 1,3-Butylen, 1,4-Butylen, 1,3-Pentylen, 1,2-Cyclohexylen, 1,3-Cyclohexylen oder 2(4)-Methyl-1,3-cyclohexylen, insbesondere für 1,2-Ethylen, 1,2-Propylen, 1,3-Propylen, 2-Methyl-1,2-propylen, 2,2-Dimethyl-1,3-propylen, 1,3-Butylen oder 1,3-Pentylen, besonders bevorzugt für 1,2-Ethylen oder 1,3-Propylen, am meisten bevorzugt für 1,3-Propylen.

Meist bevorzugt stehen R³ für Methyl und R¹ und R² zusammen für 1,3-Propylen. Eine solche Amidingruppe weist eine besonders hohe katalytische Aktivität auf und ist besonders einfach herstellbar.

In einer weiteren Ausführungsform steht Z für eine aliphatische Guanidin-Gruppe. In diesem Fall steht R³ für -NR⁴R⁵.

Ein Guanidin-Gruppen aufweisende Verbindung enthaltend mindestens eine Hexahydrotriazin-Einheit weist den Vorteil auf, dass sie bei der Verwendung als Katalysator für die Vernetzung von Silangruppen eine ganz besonders hohe katalytische Aktivität besitzt.

Dabei stehen R¹ und R⁰ jeweils bevorzugt für einen Wasserstoff-Rest. R⁴ steht bevorzugt für einen Wasserstoff-Rest.

R⁵ steht bevorzugt für einen Alkyl-, Cycloalkyl- oder Aralkyl-Rest mit 1 bis 12 C-Atomen, insbesondere 1 bis 8 C-Atomen, welcher gegebenenfalls einen Ether-Sauerstoff oder tertiären Amin-Stickstoff enthält.

Besonders bevorzugt steht Z somit für eine Guanidingruppe, bei welcher R³ für-NR⁴R⁵ steht, R¹, R⁰ und R⁴ jeweils für einen Wasserstoff-Rest stehen und R² und R⁵ unabhängig voneinander jeweils für einen Alkyl-, Cycloalkyl- oder Aralkyl-Rest mit 1 bis 12 C-Atomen, welcher gegebenenfalls einen Ether-Sauerstoff oder tertiären Amin-Stickstoff enthält, stehen.

Z stellt somit besonders bevorzugt eine Guanidin-Gruppe der Formel 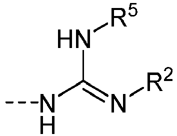 dar. Eine solche Guanidingruppe ist besonders einfach und in hoher Reinheit zugänglich.

Dabei stehen R² und R⁵ bevorzugt unabhängig voneinander jeweils für Ethyl, Isopropyl, tert.Butyl, 3-(Dimethylamino)propyl oder Cyclohexyl, insbesondere für Isopropyl oder Cyclohexyl.

Die Verbindung enthaltend mindestens eine Hexahydrotriazin-Einheit der Formel (I) ist bevorzugt ausgewählt aus Verbindungen der Formel (II) und Verbindungen der Formel (III), 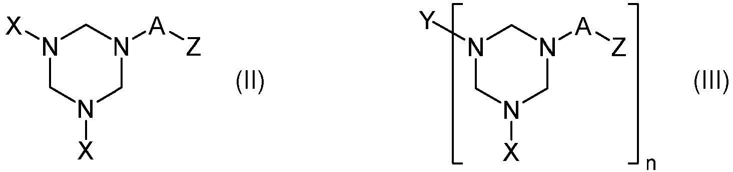 wobei
X für -A-Z oder für einen einwertigen Kohlenwasserstoff-Rest mit 1 bis 30 C-Atomen, welcher gegebenenfalls Heteroatome enthält, steht,
Y für einen n-wertigen Kohlenwasserstoff-Rest mit 2 bis 30 C-Atomen, welcher gegebenenfalls Heteroatome enthält, steht, und
n für 2 oder 3 steht,
wobei im Fall von Verbindungen der Formel (III) die Hexahydrotriazin-Ringe jeweils durch eine Kette von mindestens zwei C-Atomen voneinander getrennt sind.

Bevorzugt steht X für -A-Z oder für einen Alkyl- oder Cycloalkyl- oder Aralkyl-Rest mit 1 bis 20 C-Atomen, welcher gegebenenfalls Ethergruppen, Hydroxylgruppen, Aminogruppen, Silangruppen oder Siloxan-Einheiten aufweist.

In einer bevorzugten Ausführungsform enthält die Verbindung der Formel (II) oder (III) mindestens einen Rest X, welcher für einen mindestens eine tertiäre Aminogruppe aufweisenden Alkyl- oder Cycloalkyl- oder Aralkyl-Rest mit 1 bis 20 C-Atomen steht, insbesondere 3-(Dimethylamino)propyl. Eine solche Verbindung ist besonders geeignet als Katalysator für härtbare Polyurethanzusammensetzungen.

Besonders bevorzugt steht X für einen Rest ausgewählt aus -A-Z, Methyl, Ethyl, Propyl, Isopropyl, Butyl, Isobutyl, sec-Butyl, tert.Butyl, Hexyl, Octyl, 2-Ethylhexyl, Cyclohexyl, Benzyl, 3-(Dimethylamino)propyl, Methoxyethyl, Methoxyethoxyethyl, Trimethoxysilylpropyl, Triethoxysilylpropyl und ω-Alkoxy-poly(di-methylsiloxan)prop-3-yl mit einem mittleren Molekulargewicht im Bereich von etwa 350 bis 2'000 g/mol, wobei Alkoxy insbesondere für Methoxy, Ethoxy, Propoxy, Isopropoxy, Butoxy, Isobutoxy, tert.Butoxy oder die isomeren Pentoxy- oder Hexyloxy-Reste steht.

Bevorzugt steht n für 2.

Bevorzugt steht Y für einen Alkylen-Rest mit 2 bis 20 C-Atomen, welcher gegebenenfalls Heteroatome in Form von Ether-Sauerstoff, Amin-Stickstoff oder Siloxan-Einheiten aufweist.

Besonders bevorzugt ist Y ausgewählt aus der Gruppe bestehend aus 2-Methyl-1,5-pentylen, 1,6-Hexylen, 2,2(4),4-Trimethyl-1,6-hexamethylen, 1,8-Octylen, 1,10-Decylen, 1,12-Dodecylen, (1,5,5-Trimethylcyclohexan-1-yl)methan-1,3, 1,3-Cyclohexylen-bis(methylen), 1,4-Cyclohexylen-bis(methylen), 1,3-Phenylen-bis(methylen), 2- und/oder 4-Methyl-1,3-cyclohexylen, N-Methyl-4-aza-1,7-heptylen, N-Ethyl-4-aza-1,7-heptylen, Piperazin-1,4-diyl-bis(1,2-ethylen), Piperazin-1,4-diyl-bis(1,3-propylen), 3-Oxa-1,5-pentylen, 3,6-Dioxa-1,8-octylen, 4,7-Dioxa-1,10-decylen, α,ω-Polyoxypropylen mit einem mittleren Molekulargewicht im Bereich von etwa 180 bis 500 g/mol und α,ω-(1,3-Propylen)-polydimethylsiloxan mit einem mittleren Molekulargewicht im Bereich von etwa 350 bis 2'000 g/mol.

Davon bevorzugt ist α,ω-Polyoxypropylen mit einem mittleren Molekulargewicht im Bereich von etwa 180 bis 500 g/mol. Diese Verbindungen der Formel (III) sind besonders geeignet als Katalysator für funktionelle Polyether-Polymere.

Davon weiterhin bevorzugt ist α,ω-(1,3-Propylen)polydimethylsiloxan mit einem mittleren Molekulargewicht im Bereich von etwa 350 bis 2'000 g/mol. Diese Verbindungen der Formel (III) sind besonders geeignet als Katalysator für funktionelle Polyorganosiloxan-Polymere.

Bevorzugt sind Verbindungen der Formel (II), welche im Mittel eine oder zwei Guanidingruppen aufweisen. Solche Verbindungen sind bei Raumtemperatur typischerweise flüssig und somit leicht handhabbar, und bei der Verwendung als Katalysator ermöglichen sie härtbare Zusammensetzungen mit guter Lagerstabilität und schneller Vernetzung.

Bevorzugt sind weiterhin Verbindungen der Formel (II), welche im Mittel eine Guanidingruppe und eine oder zwei Amidingruppen aufweisen. Solche Verbindungen sind bei Raumtemperatur typischerweise flüssig und somit leicht handhabbar, und bei der Verwendung als Katalysator ermöglichen sie härtbare Zusammensetzungen mit guter Lagerstabilität und schneller Vernetzung.

Bevorzugt sind weiterhin Verbindungen der Formel (II) oder der Formel (III), welche im Mittel zwei oder drei oder vier Amidingruppen aufweisen. Solche Verbindungen sind bei Raumtemperatur oft flüssig und somit leicht handhabbar, und bei der Verwendung als Katalysator ermöglichen sie härtbare Zusammensetzungen mit guter Lagerstabilität und schneller Vernetzung.

Verbindungen der Formel (II) oder (III), welche Siloxan-Einheiten enthalten, sind besonders gut verträglich mit Polyorganosiloxan-Polymeren und somit besonders geeignet als Katalysator für solche Systeme.

Verbindungen der Formel (II) oder (III), welche Polyoxyalkylen-Einheiten enthalten, sind besonders gut verträglich mit Polyether-Polymeren und somit besonders geeignet als Katalysator für solche Systeme.

Verbindungen der Formel (II) oder (III), welche neben dem Hexohydrotriazin-Ring weitere tertiäre Aminogruppen aufweisen, sind besonders aktiv als Katalysator für Isocyanatgruppen aufweisende Zusammensetzungen.

Ein weiterer Gegenstand der Erfindung ist ein Verfahren zur Herstellung der Verbindung enthaltend mindestens eine Hexahydrotriazin-Einheit der Formel (I), indem mindestens ein Amin der Formel H₂N-A-Z und gegebenenfalls mindestens ein weiteres primäres Amin mit Formaldehyd oder einer Formaldehyd freisetzenden Verbindung, insbesondere Paraformaldehyd oder 1,3,5-Trioxan, unter Entfernung von Wasser umgesetzt wird.

Das Verfahren ist überraschend schnell und einfach durchführbar, insbesondere ohne den Einsatz von Hilfsstoffen und ohne eine aufwendige Reinigung des Reaktionsprodukts zu erfordern, und geht von kommerziell zugänglichen, preisgünstigen Ausgangsmaterialien aus.

Das Verfahren wird bevorzugt bei einer Temperatur im Bereich von 0 bis 60°C, insbesondere 20 bis 40°C, durchgeführt.

Bevorzugt werden die Reaktanden in beliebiger Reihenfolge miteinander vermischt, gegebenenfalls in Gegenwart eines organischen Lösemittels, und reagieren lassen. Das Reaktionsprodukt kann mit Wasser gewaschen und anschliessend getrocknet werden, beispielsweise über Magnesiumsulfat. Vorhandene flüchtige Bestandteile werden bevorzugt unter Vakuum abdestilliert.

Ein solches Reaktionsprodukt kann ohne weitere Aufarbeitung oder Reinigung als Katalysator für funktionelle Polymere oder Verbindungen bzw. als Katalysator für härtbare Zusammensetzungen verwendet werden.

Als Amin der Formel H₂N-A-Z für das Verfahren geeignet sind Aminoamidine oder Aminoguanidine, welche eine primäre Aminogruppe aufweisen.

Ein für das Verfahren bevorzugtes Aminoamidin der Formel H₂N-A-Z weist die Formel 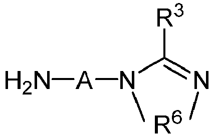 auf.

Es ist insbesondere ausgewählt aus der Gruppe bestehend aus 1-(2-Aminoethyl)-2-methyl-imidazolin, 1-(3-Aminopropyl)-2-methyl-imidazolin, 1-(2-Aminoethyl)-2-methyl-1,4,5,6-tetrahydropyrimidin und 1-(3-Aminopropyl)-2-methyl-1,4,5,6-tetrahydropyrimidin.

Davon bevorzugt sind 1-(2-Aminoethyl)-2-methyl-1,4,5,6-tetrahydropyrimidin oder 1-(3-Aminopropyl)-2-methyl-1,4,5,6-tetrahydropyrimidin. Diese Aminoamidine ermöglichen eine vergleichsweise hohe katalytische Aktivität.

Solche Aminoamidine sind ihrerseits insbesondere herstellbar aus der Umsetzung von mindestens einem Polyalkylenamin der Formel H₂N-A-NH-R₆-NH₂, wie insbesondere Diethylentriamin, Dipropylentriamin oder N-(2-Aminoethyl)-1,3-propandiamin (N3-Amin), mit mindestens einem Reagens zur Einführung von Amidin-Gruppen, insbesondere ein Orthoester, ein 1,3-Ketoester oder ein Nitril, bevorzugt ausgewählt aus der Gruppe bestehend aus Trimethylorthoformiat, Triethylorthoformiat, Trimethylorthoacetat, Triethylorthoacetat, Methylacetoacetat, Ethylacetoacetat, Isopropylacetoacetat, tert.Butylacetoacetat und Acetonitril.

Die Umsetzung wird bevorzugt bei erhöhter Temperatur, gegebenenfalls unter erhöhtem Druck und gegebenenfalls in Gegenwart eines Katalysators, durchgeführt, wobei aus dem Reagens freigesetzte Abspalter wie Alkohole, Ester oder Amine während oder nach der Umsetzung bevorzugt entfernt werden, insbesondere mittels Destillation, gegebenenfalls unter Vakuum.

Wird Trimethylorthoformiat, Triethylorthoformiat, Trimethylorthoacetat, Triethylorthoacetat oder ein anderer Orthoester der Formel R³-C(OR^{a})₃ eingesetzt, so erfolgt die Umsetzung bevorzugt bei einer Temperatur von 40 bis 160°C, insbesondere 60 bis 140°C, wobei der freigesetzte Alkohol R^{a}OH bevorzugt destillativ entfernt wird. Gegebenenfalls wird dabei ein Katalysator eingesetzt, insbesondere eine Säure.

Wird Methylacetoacetat, Ethylacetoacetat, Isopropylacetoacetat, tert.Butyl-acetoacetat oder ein anderer 1,3-Ketoester der Formel R³-C(O)CH₂C(O)OR^{a} eingesetzt, so erfolgt die Umsetzung bevorzugt bei einer Temperatur im Bereich von 20 bis 100°C, insbesondere 40 bis 80°C, wobei der freigesetzte Ester CH₃C(O)OR^{a} bevorzugt destillativ entfernt wird. Bevorzugt wird dabei ein Katalysator eingesetzt, insbesondere eine Säure, bevorzugt eine Sulfonsäure. Wird Acetonitril oder ein anderes Nitril der Formel R³-CN eingesetzt, so erfolgt die Umsetzung bevorzugt bei einer Temperatur von 60 bis 180°C, insbesondere 80 bis 160°C, gegebenenfalls unter erhöhtem Druck, wobei der freigesetzte Ammoniak bevorzugt destillativ entfernt wird. Bevorzugt wird dabei ein Katalysator eingesetzt, insbesondere eine Lewissäure, bevorzugt Bortrifluorid-Etherat, Lithiumperchlorat, Zinkchlorid, Zink(III)-trifluormethansulfonat oder Lanthan(III)trifluormethansulfonat.

Ein für das Verfahren bevorzugtes Aminoguanidin der Formel H₂N-A-Z weist die Formel 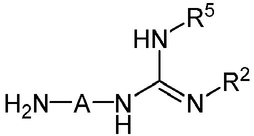 auf.

Es ist insbesondere ausgewählt aus der Gruppe bestehend aus 1-(2-Aminoethyl)-2,3-dicyclohexylguanidin, 1-(2-Amino-2(1)-methylethyl)-2,3-dicyclohexyl-guanidin, 1-(3-Aminopropyl)-2,3-dicyclohexylguanidin, 1-(5-Amino-4(2)-methylpentyl)-2,3-dicyclohexylguanidin, 1-(6-Aminohexyl)-2,3-dicyclohexylguanidin, 1-(6-Amino-2,2(4),4-trimethylhexyl)-2,3-dicyclohexylguanidin, 1-(6-Amino-3,3(5),5-trimethylhexyl)-2,3-dicyclohexylguanidin, 1-(8-Aminooctyl)-2,3-dicyclo-hexylguanidin, 1-(10-Aminodecyl)-2,3-dicyclohexylguanidin, 1-(12-Aminodode-cyl)-2,3-dicyclohexylguanidin, 1-(3-Aminomethyl-3,5,5-trimethylcyclohexyl)-2,3-dicyclohexylguanidin, 1-(3-Amino-1,5,5-trimethylcyclohexylmethyl)-2,3-dicyclo-hexylguanidin, 1-(3-Aminomethylcyclohexyl)-2,3-dicyclohexylguanidin, 1-(4-Aminomethylcyclohexyl)-2,3-dicyclohexylguanidin, 1-(3-Aminomethylbenzyl)-2,3-dicyclohexylguanidin, 1-(3-Amino-2(4)-methylcyclohexyl)-2,3-dicyclohexyl-guanidin, 1-(ω-2-Aminopropyl-polyoxypropylen)-2,3-dicyclohexylguanidin mit einem mittleren Molekulargewicht im Bereich von etwa 400 bis 500 g/mol und 1-(ω-3-Aminopropyl-α-1,3-propylen-poly(dimethylsiloxan))-2,3-dicyclohexylgua-nidin mit einem mittleren Molekulargewicht im Bereich von etwa 550 bis 2'250 g/mol, und den entsprechenden Verbindungen mit 2,3-Diisopropyl- anstelle der 2,3-Dicyclohexyl-Gruppen.

Solche Aminoguanidine sind ihrerseits insbesondere herstellbar aus der Umsetzung von mindestens einem primären Polyamin der Formel H₂N-A-NH₂ mit mindestens einem Carbodiimid der Formel R⁵-N=C=N-R², wobei R² und R⁵ die beschriebenen Bedeutungen aufweisen.

Der Umsetzung wird bevorzugt bei erhöhter Temperatur, gegebenenfalls unter erhöhtem Druck und gegebenenfalls in Gegenwart eines Katalysators, durchgeführt. Bevorzugt werden das primäre Polyamin und das Carbodiimid im Molverhältnis von ungefähr 1:1 umgesetzt.

Besonders geeignet als Carbodiimid ist N,N'-Diisopropylcarbodiimid (DIC), N,N'-Di-tert.butylcarbodiimid, N,N'-Dicyclohexylcarbodiimid (DCC) oder N-Ethyl-N'-(3-dimethylaminopropyl)carbodiimid (EDC), insbesondere N,N'-Diisopropylcarbodiimid (DIC) oder N,N'-Dicyclohexylcarbodiimid (DCC). Diese Reagentien sind einfach verfügbar und lassen sich gut zu Guanidinen umsetzen.

Besonders geeignet als primäres Polyamin der Formel H₂N-A-NH₂ sind handelsübliche Amine mit zwei primären Aminogruppen, wie insbesondere die folgenden:
- aliphatische, cycloaliphatische oder arylaliphatische primäre Diamine, insbesondere Ethylendiamin, 1,2- oder 1,3-Propandiamin, 2-Methyl-1,2-propandiamin, 2,2-Dimethyl-1,3-propandiamin, 1,3- oder 1,4-Butandiamin, 1,3-Pentandiamin (DAMP), 1,5-Pentandiamin, 1,5-Diamino-2-methylpentan (MPMD), 2-Butyl-2-ethyl-1,5-pentandiamin (C11-Neodiamin), 1,6-Hexandiamin, 2,5-Dimethyl-1,6-hexandiamin, 2,2,4- und 2,4,4-Trimethylhexamethylendiamin (TMD), 1,7-Heptandiamin, 1,8-Octandiamin, 1,9-Nonandiamin, 1,10-Decandiamin, 1,11-Undecandiamin, 1,12-Dodecandiamin, 1,2-, 1,3- oder 1,4-Diaminocyclohexan, Bis(4-aminocyclohexyl)methan, Bis(4-amino-3-methyl-cyclohexyl)methan, Bis(4-amino-3-ethylcyclohexyl)methan, Bis(4-amino-3,5-dimethylcyclohexyl)methan, Bis(4-amino-3-ethyl-5-methylcyclohexyl)methan, 1-Amino-3-aminomethyl-3,5,5-trimethylcyclohexan (Isophorondiamin oder IPD), 2- und/oder 4-Methyl-1,3-diaminocyclohexan, 1,3- oder 1,4-Bis-(aminomethyl)cyclohexan, 2,5(2,6)-Bis(aminomethyl)bicyclo[2.2.1]heptan (NBDA), 3(4),8(9)-Bis(aminomethyl)tricyclo[5.2.1.0^{2,6}]decan, 1,4-Diamino-2,2,6-trimethylcyclohexan (TMCDA), 1,8-Menthandiamin, 3,9-Bis(3-aminopropyl)-2,4,8,10-tetraoxaspiro[5.5]undecan, 1,3-Bis(aminomethyl)benzol, 1,4-Bis(aminomethyl)benzol, 4-Aminomethyl-1,8-octandiamin, N,N'-Bis(2-aminoethyl)piperazin, N,N'-Bis(3-aminopropyl)piperazin, N,N-Bis(3-aminopropyl)methylamin, N,N-Bis(3-aminopropyl)ethylamin, N,N-Bis(3-aminopropyl)propylamin, N,N-Bis(3-aminopropyl)cyclohexylamin, N,N-Bis(3-aminopropyl)-2-ethylhexylamin, oder Produkte aus der doppelten Cyanoethylierung und nachfolgenden Reduktion von Fettaminen, welche abgeleitet sind von natürlichen Fettsäuren, wie N,N-Bis(3-aminopropyl)dodecylamin oder N,N-Bis(3-aminopropyl)talgalkylamin, erhältlich als Triameen® Y12D oder Triameen® YT (von Akzo Nobel);
- Ethergruppenhaltige aliphatische oder cycloaliphatische primäre Diamine, insbesondere Bis(2-aminoethyl)ether, 3,6-Dioxaoctan-1,8-diamin, 4,7-Dioxa-decan-1,10-diamin, 4,7-Dioxadecan-2,9-diamin, 4,9-Dioxadodecan-1,12-diamin, 5,8-Dioxadodecan-3,10-diamin, 4,7,10-Trioxatridecan-1,13-diamin, cycloaliphatische ethergruppenhaltige Diamine aus der Propoxylierung und nachfolgenden Aminierung von 1,4-Dimethylolcyclohexan, erhältlich insbesondere als Jeffamine® RFD-270 (von Huntsman), Polyoxyalkylenamine mit einem mittleren Molekulargewicht im Bereich von 200 bis 500 g/mol, wie sie im Handel beispielsweise unter den Handelsnamen Jeffamine® (von Huntsman), Polyetheramine (von BASF) und PC Amine® (von Nitroil) erhältlich sind, dadurch gekennzeichnet, dass sie 2-Aminopropyl- oder 2-Aminobutyl-Endgruppen tragen, insbesondere Jeffamine® D-230, Jeffamine® D-400, Jeffamine® XTJ-582, Jeffamine® HK-511 oder Jeffamine® XTJ-566 (alle von Huntsman), oder dazu analoge Typen von BASF und Nitroil;
- Polyalkylenamine, insbesondere Diethylentriamin (DETA), Triethylentetramin (TETA), Tetraethylenpentamin (TEPA), Pentaethylenhexamin (PEHA), Polyethylenpolyamin mit 5 bis 7 Ethylenamin-Einheiten (sogenanntes "higher ethylenepolyamine", HEPA), Dipropylentriamin (DPTA), N-(2-Aminoethyl)-1,3-propandiamin (N3-Amin), N,N'-Bis(3-aminopropyl)ethylendiamin (N4-Amin), Bis-hexamethylentriamin (BHMT), N3-(3-Aminopentyl)-1,3-pentandiamin, N5-(3-Aminopropyl)-2-methyl-1,5-pentandiamin oder N5-(3-Amino-1 -ethylpropyl)-2-methyl-1,5-pentandiamin.

Davon bevorzugt sind Ethylendiamin, 1,2-Propandiamin, 1,3-Propandiamin, DAMP, 1,5-Pentandiamin, MPMD, 1,6-Hexandiamin, TMD, 1,8-Octandiamin, 1,10-Decandiamin, 1,12-Dodecandiamin, IPD, 1,3-Bis(aminomethyl)cyclohexan, 1,4-Bis(aminomethyl)cyclohexan, 1,3-Bis(aminomethyl)benzol, 2-und/oder 4-Methyl-1,3-diaminocyclohexan, N,N-Bis(3-aminopropyl)methylamin, N,N-Bis(3-aminopropyl)ethylamin, 1,4-Bis(aminoethyl)piperazin, 1,4-Bis(aminopropyl)piperazin, Bis-(2-aminoethyl)ether, 3,6-Dioxaoctan-1,8-diamin, 4,7-Di-oxadecan-1,10-diamin, Polyoxypropylendiamine mit einem mittleren Molekulargewicht im Bereich von etwa 220 bis 500 g/mol oder α,ω-Bis(3-aminopropyl)-polydimethylsiloxane mit einem mittleren Molekulargewicht im Bereich von etwa 350 bis 2'000 g/mol.

Für das Verfahren zur Herstellung einer Verbindung enthaltend mindestens eine Hexahydrotriazin-Einheit der Formel (I) wird neben mindestens einem Amin der Formel H₂N-A-Z gegebenenfalls mindestens ein weiteres primäres Amin eingesetzt.

Das weitere primäre Amin ist insbesondere ein Amin mit einer oder zwei oder drei primären Aminogruppen, welches 1 bis 30 C-Atome aufweist und gegebenenfalls Heteroatome enthält.

Dafür geeignet sind einerseits Amine mit einer primären Aminogruppe, wie insbesondere
- aliphatische, cycloaliphatische oder arylaliphatische gegebenenfalls Ethergruppen aufweisende Monoamine, insbesondere Methylamin, Ethylamin, Propylamin, Isopropylamin, n-Butylamin, Isobutylamin, sec.Butylamin, tert.Butylamin, n-Pentylamin, Isopentylamin, 3-Methyl-2-butylamin, n-Hexylamin, n-Octylamin, 2-Ethylhexylamin, n-Decylamin, Laurylamin, Myristylamin, Palmitylamin, Stearylamin, Cyclohexylamin, Benzylamin, oder von natürlichen Fettsäuregemischen abgeleitete Fettamine, wie zum Beispiel Cocoalkylamin, C₁₆-C₂₂-Alkylamin, Soyaalkylamin, Oleylamin oder Talgalkylamin, erhältlich beispielsweise unter den Handelsnamen Armeen® (von Akzo Nobel) oder Rofamin® (von Ecogreen Oleochemicals), 2-(Diethylami-no)ethylamin, 2-(Diisopropylamino)ethylamin, 3-(Dimethylamino)propylamin, 3-(Diethylamino)propylamin, 1-Diethylamino-4-aminopentan, 2-Methoxyethylamin, 2-Ethoxyethylamin, 2-Butoxyethylamin, 2-Cyclohexyloxyethylamin, 2-Benzyloxyethylamin, 3-Methoxypropylamin, 3-Ethoxypropylamin, 3-Butoxypropylamin, 3-Hexyloxypropylamin, 3-(2-Ethylhexyloxy)propylamin, 3-Cyclohexyloxypropylamin, 3-Phenyloxypropylamin, 2-(2-Methoxyethoxy)-ethylamin, 3-(2-Methoxyethoxy)propylamin, 2(4)-Methoxyphenylethylamin, oder Polyetheramine, insbesondere Polyoxyalkylenamine, insbesondere im Handel erhältliche Typen wie insbesondere Jeffamine® XTJ-581, Jeffamine® M-600, Jeffamine® M-1000, Jeffamine® M-2005, Jeffamine® M-2070 (alle von Huntsman), oder Amine von Fettalkohol- oder Alkylphenolalkoxylaten wie insbesondere Jeffamine® XTJ-247, Jeffamine® XTJ-248, Jeffamine® XTJ-249, Jeffamine® XTJ-435 oder Jeffamine® XTJ-436 (alle von Huntsman);
- Aminosilane, insbesondere 3-Aminopropyltrimethoxysilan, 3-Aminopropyldimethoxymethylsilan, N-(2-Aminoethyl)-3-aminopropyltrimethoxysilan, N-(2-Aminoethyl)-3-aminopropylmethyldimethoxysilan, N-(2-Aminoethyl)-N'-[3-(tri-methoxysilyl)propyl]ethylendiamin, 4-Aminobutyltrimethoxysilan, 4-Aminobutyldimethoxymethylsilan, 4-Amino-3-methylbutyltrimethoxysilan, 4-Amino-3-methylbutyldimethoxymethylsilan, 4-Amino-3,3-dimethylbutyltrimethoxysilan, 4-Amino-3,3-dimethylbutyldimethoxymethylsilan, sowie deren Analoga mit Ethoxy- anstelle der Methoxygruppen am Silicium;
- Polyorganosiloxane mit einer primären Aminogruppe, wie insbesondere α-(3-Aminopropyl)-ω-Alkoxy-poly(dimethylsiloxan) mit einem mittleren Molekulargewicht im Bereich von 350 bis 2'000 g/mol.

Davon bevorzugt ist Methylamin, Ethylamin, Propylamin, Isopropylamin, n-Butylamin, Isobutylamin, sec.Butylamin, tert.Butylamin, n-Hexylamin, n-Octylamin, 2-Ethylhexylamin, Cyclohexylamin, Benzylamin, 3-(Dimethylamino)propylamin, 2-Methoxyethylamin, 2-(2-Methoxyethoxy)ethylamin, 3-Aminopropyltrimethoxysilan, 3-Aminopropyltriethoxysilan oder α-(3-Aminopropyl)-ω-alkoxy-poly(dimethylsiloxan) mit einem mittleren Molekulargewicht im Bereich von 350 bis 2'000 g/mol.

Dafür geeignet sind weiterhin Amine mit zwei oder drei, insbesondere zwei, primären Aminogruppen, wie insbesondere die bereits genannten Polyamine der Formel H₂N-A-NH₂. Diese Amine sind geeignet für die Herstellung von Verbindungen der Formel (III).

Bevorzugt sind MPMD, 1,6-Hexandiamin, TMD, 1,8-Octandiamin, 1,10-Decandiamin, 1,12-Dodecandiamin, IPD, 1,3-Bis(aminomethyl)cyclohexan, 1,4-Bis-(aminomethyl)cyclohexan, 1,3-Bis(aminomethyl)benzol, 2- und/oder 4-Methyl-1,3-diaminocyclohexan, N,N-Bis(3-aminopropyl)methylamin, N,N-Bis(3-aminopropyl)ethylamin, 1,4-Bis(2-aminoethyl)piperazin1,4-Bis(3-aminopropyl)piperazin, Bis-(2-aminoethyl)ether, 3,6-Dioxaoctan-1,8-diamin, 4,7-Dioxadecan-1,10-diamin, Polyoxypropylendiamine mit einem mittleren Molekulargewicht im Bereich von etwa 220 bis 500 g/mol oder α,ω-Bis(3-aminopropyl)polydimethylsiloxane mit einem mittleren Molekulargewicht im Bereich von etwa 350 bis 2'000 g/mol.

Besonders bevorzugt sind MPMD, 1,6-Hexandiamin, TMD, 1,8-Octandiamin, 1,10-Decandiamin, 1,12-Dodecandiamin, IPD, 1,3-Bis(aminomethyl)cyclohexan, 1,4-Bis(aminomethyl)cyclohexan, 1,3-Bis(aminomethyl)benzol, 2- und/oder 4-Methyl-1,3-diaminocyclohexan, Polyoxypropylendiamine mit einem mittleren Molekulargewicht im Bereich von etwa 220 bis 500 g/mol oder α,ω-Bis(3-aminopropyl)polydimethylsiloxane mit einem mittleren Molekulargewicht im Bereich von etwa 350 bis 2'000 g/mol.

In einer bevorzugten Ausführungsform des Verfahrens wird ein Amin der Formel H₂N-A-Z mit Formaldehyd oder einer Formaldehyd freisetzenden Verbindung im Molverhältnis von ungefähr 1:1 umgesetzt. Dabei entstehen Verbindungen der Formel (II), bei welchen X für -A-Z stehen.

Es ist möglich und kann bevorzugt sein, dabei eine Mischung von verschiedenen Aminen der Formel H₂N-A-Z einzusetzten, beispielsweise eine Mischung aus Aminoamidinen und Aminoguanidinen, und/oder eine Mischung, bei welchen A für verschiedene Reste steht, beispielsweise eine Mischung enthaltend Polyoxypropylen-Reste und Kohlenwasserstoffreste ohne Heteroatome, oder ein Mischung enthaltend Amine mit Siloxaneinheiten und Amine ohne Heteroatome im Rest A.

In einer weiteren bevorzugten Ausführungsform des Verfahrens wird eine Mischung aus Amin der Formel H₂N-A-Z und einem Amin mit nur einer primären Aminogruppe mit Formaldehyd oder einer Formaldehyd freisetzenden Verbindung so umgesetzt, dass das Molverhältnis zwischen den primären Aminogruppen und Formaldehyd ungefähr 1:1 beträgt. Dabei entstehen Verbindungen der Formel (II), bei welchen ein Teil der Reste X nicht für -A-Z stehen.

In einer weiteren bevorzugten Ausführungsform des Verfahrens werden mindestens ein Amin der Formel H₂N-A-Z, mindestens ein Polyamin mit zwei oder drei, insbesondere zwei, primären Aminogruppen und gegebenenfalls mindestens ein Amin mit nur einer primären Aminogruppe mit Formaldehyd oder einer Formaldehyd freisetzenden Verbindung umgesetzt, so dass das Molverhältnis zwischen den primären Aminogruppen und Formaldehyd ungefähr 1:1 beträgt. Dabei entstehen Verbindungen der Formel (III).

Eine alternative Möglichkeit, eine Verbindung enthaltend mindestens eine Hexahydrotriazin-Einheit der Formel (I) herzustellen, besteht darin,
- zuerst ein Amin der Formel H₂N-A-NH-R₆-NH₂ oder H₂N-A-NH₂ mit Formaldehyd oder einer Formaldehyd freisetzenden Verbindung zu einer Verbindung mit mindestens einer Hexahydrotriazin-Einheit der Formel (Ia) oder (Ib) 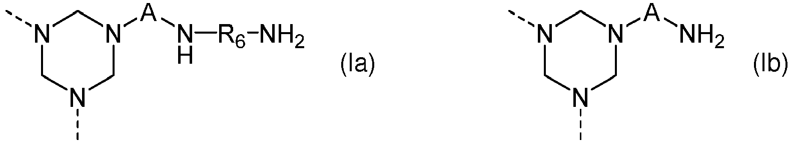 umzusetzen,
- und diese anschliessend mit dem Reagens zur Einführung von Amidin- oder Guanidin-Gruppen zur gewünschten Verbindung enthaltend mindestens eine Hexahydrotriazin-Einheit der Formel (I) umzusetzen.

Diese alternative Möglichkeit der Herstellung ist insbesondere im Fall von Amidinen vorteilhaft, indem beispielsweise eine Hexahydratriazin-Verbindung der Formel (IV) mit einem geeigneten Reagens zur Einführung von Amidingruppen zu einer Verbindung der Formel (IIa) umgesetzt wird.

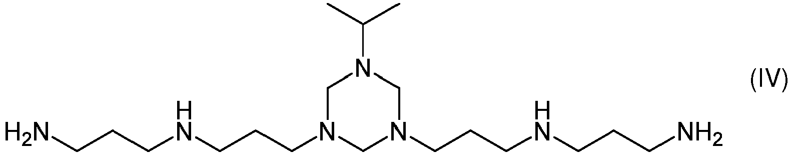

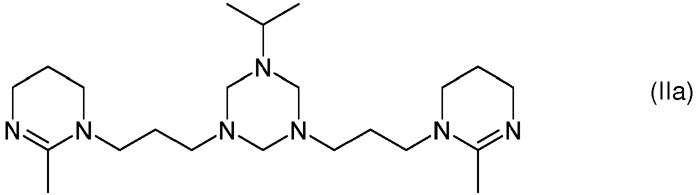

Eine weitere alternative Möglichkeit, eine Verbindung enthaltend mindestens eine Hexahydrotriazin-Einheit der Formel (I) herzustellen, besteht darin, dass mindestens ein Hexahydrotriazin der Formel (V) oder Hexamethylentriamin gemäss Formel (Va) 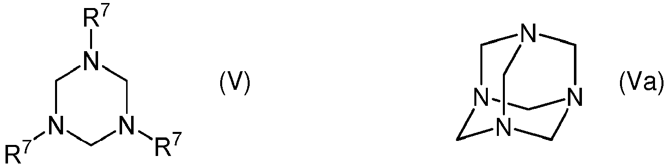 mit mindestens einem Amin der Formel H₂N-A-Z und gegebenenfalls mindestens einem weiteren primären Amin unter Umaminierung und Freisetzung von Amin der Formel H₂N-R⁷ bzw. Ammoniak umgesetzt wird,
wobei R⁷ für einen Alkyl-Rest mit 1 bis 6 C-Atomen, insbesondere für Methyl, Ethyl, Propyl, Isopropyl, Butyl, Isobutyl oder tert.Butyl, steht.

Die Umaminierung wird bevorzugt bei erhöhter Temperatur, insbesondere bei einer Temperatur im Bereich von 60 bis 180°C, durchgeführt. Bevorzugt wird freigesetztes Amin der Formel H₂N-R⁷ bzw. Ammoniak während der Umaminierung fortlaufend destillativ entfernt.

Auch diese Herstellung kann so durchgeführt werden, dass für die Umaminierung anstelle des Amins der Formel H₂N-A-Z das entsprechende Amin der Formel H₂N-A-NH-R₆-NH₂ bzw. H₂N-A-NH₂ eingesetzt wird und die Amidin- oder Guanidin-Gruppen erst nach der Umaminierung durch Umsetzung mit einem geeigneten Reagens eingeführt werden.

Die Herstellung über Umaminierung ist vor allem bei der Verwendung von Aminosilanen als weiteres Amin vorteilhaft, da dabei die Silangruppen nicht mit Wasser in Kontakt kommen.

Insbesondere Silangruppen-haltige Verbindungen, wie beispielsweise die Verbindungen der Formel (IIb) oder (IIc), werden bevorzugt über Umaminierung hergestellt.

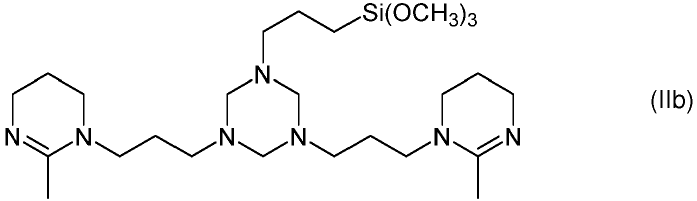

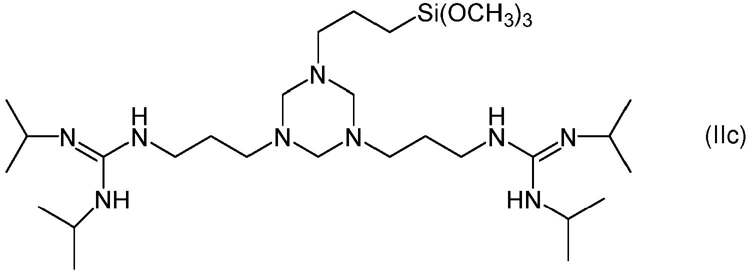

Die Verbindung der Formel (IIb) beispielsweise wird besonders bevorzugt hergestellt, indem 1 mol eines Hexahydrotriazins der Formel (V) mit 2 mol DPTA und 1 mol 3-Aminopropyltrimethoxysilan umaminiert und das erhaltene Produkt anschliessend mit einem geeigneten Reagens zur Einführung von Amidingruppen, beispielsweise Trimethylorthoacetat, umgesetzt wird.

Die Verbindung der Formel (IIc) beispielsweise wird besonders bevorzugt hergestellt, indem 1 mol eines Hexahydrotriazins der Formel (V) mit 2 mol 1-(3-Aminopropyl)-2,3-diisopropylguanidin und 1 mol 3-Aminopropyltrimethoxysilan umaminiert wird.

Ein weiterer Gegenstand der Erfindung ist die Verwendung der Verbindung enthaltend mindestens eine Hexahydrotriazin-Einheit der Formel (I) als Katalysator für die Vernetzung einer funktionellen Verbindung. Dabei beschleunigt die Verbindung enthaltend mindestens eine Hexahydrotriazin-Einheit der Formel (I) die Vernetzung bzw. Aushärtung der funktionellen Verbindung bzw. die Aushärtung einer Zusammensetzung enthaltend die funktionelle Verbindung. Über die Auswahl der Substituenten an der Hexahydrotriazin-Einheit der Verbindung enthaltend mindestens eine Hexahydrotriazin-Einheit der Formel (I) kann diese in ihrer katalytischen Aktivität und ihrer Verträglichkeit auf die jeweilige funktionelle Verbindung bzw. auf die jeweilige Zusammensetzung enthaltend die funktionelle Verbindung optimal abgestimmt werden, so dass sie mit dieser gut mischbar und verträglich ist und nicht zu Separation oder Migration neigt.

Die funktionelle Verbindung weist insbesondere zwei oder mehr funktionelle Gruppen auf, welche mit Feuchtigkeit oder einem geeigneten Härter zu einer polymeren Struktur ausgehärtet werden kann.

Bevorzugt sind die funktionellen Gruppen ausgewählt aus der Gruppe bestehend aus Isocyanatgruppen, Silangruppen, Epoxidgruppen und Cyanatestergruppen.

Als funktionelle Verbindung geeignet ist insbesondere
- ein Polyisocyanat,
- ein Isocyanatgruppen aufweisendes Polymer, insbesondere ein Isocyanatgruppen aufweisendes Polyurethanpolymer,
- ein Silangruppen aufweisendes Polymer,
- eine Glycidoxygruppen aufweisende Verbindung, insbesondere ein di- oder polyfunktionelles Epoxidharz,
- ein Cyanatesterharz, oder
- ein Polymer mit verschiedenen dieser funktionellen Gruppen, insbesondere ein Polymer mit Isocyanat- und Silangruppen oder ein Polymer mit Isocyanat- und Epoxidgruppen.

Eine bevorzugte funktionelle Verbindung, für deren Vernetzung die Verbindung enthaltend mindestens eine Hexahydrotriazin-Einheit der Formel (I) verwendet wird, ist ein Polyisocyanat oder ein Isocyanatgruppen aufweisendes Polyurethanpolymer oder ein Silangruppen aufweisendes Polymer.

Ein geeignetes Polyisocyanat ist insbesondere ein monomeres Diisocyanat, oder ein Oligomer oder ein Polymer oder ein Derivat eines monomeren Diiisocyanats, oder eine beliebige Mischung davon. Unter Oligomeren und Polymeren werden hierbei Homopolymere und -oligomere verstanden, die ausschließlich aus Di- oder Triisocyanatbestandteilen bestehen.

Geeignete monomere Diisocyanate sind insbesondere 2,4- oder 2,6-Toluylendiisocyanat oder beliebige Gemische dieser Isomeren (TDI), 4,4'-, 2,4'- oder 2,2'-Diphenylmethandiisocyanat oder beliebige Gemische dieser Isomeren (MDI), Gemische aus MDI und MDI-Homologen (polymeres MDI oder PMDI), 1,3- oder 1,4-Phenylendiisocyanat, 2,3,5,6-Tetramethyl-1,4-diisocyanatobenzol, Naphthalin-1,5-diisocyanat (NDI), 3,3'-Dimethyl-4,4'-diisocyanatodiphenyl (TODI), Dianisidindiisocyanat (DADI), 1,4-Tetramethylendiisocyanat, 2-Methyl- pentamethylen-1,5-diisocyanat, 1,6-Hexamethylendiisocyanat (HDI), 2,2(4),4-Trimethyl-1,6-hexamethylendiisocyanat (TMDI), 1,10-Decamethylendiisocyanat, 1,12-Dodecamethylendiisocyanat, Lysin- oder Lysinesterdiisocyanat, Cyclohexan-1,3- oder -1,4-diisocyanat, 1-Methyl-2,4- oder -2,6-diisocyanatocyclohexan oder beliebige Gemische dieser Isomeren (HTDI oder H₆TDI), 1-Isocyanato-3,3,5-trimethyl-5-isocyanatomethyl-cyclohexan (Isophorondiisocyanat oder IPDI), Perhydro-2,4'- oder -4,4'-diphenylmethandiisocyanat (HMDI oder H₁₂MDI), 1,4-Diisocyanato-2,2,6-trimethylcyclohexan (TMCDI), 1,3- oder 1,4-Bis-(isocyanatomethyl)cyclohexan, m- oder p-Xylylendiisocyanat (m- oder p-XDI), m- oder p-Tetramethyl-1,3- oder -1,4-xylylendiisocyanat (m- oder p-TMXDI) oder Bis-(1-Isocyanato-1-methylethyl)naphthalin.

Davon bevorzugt ist MDI, TDI, IPDI oder HDI, insbesondere MDI.

Geeignete Oligomere, Polymere oder Derivate von monomeren Diisocyanaten sind insbesondere abgeleitet von MDI, TDI, HDI oder IPDI.

Besonders bevorzugt ist das Polyisocyanat eine bei Raumtemperatur flüssige Form von MDI, welche insbesondere einen hohen Gehalt an 4,4'-Diphenylmethandiisocyanat aufweist. Das sogenannte "flüssige MDI" stellt insbesondere entweder durch partielle chemische Modifizierung - insbesondere Carbodiimidisierung bzw. Uretoniminbildung - verflüssigtes 4,4'-Diphenylmethandiisocyanat dar, oder es ist ein durch Abmischen gezielt herbeigeführtes oder durch den Herstellungsprozess bedingtes Gemisch von 4,4'-Diphenylmethandiisocyanat mit anderen MDI-Isomeren (2,4'-Diphenylmethandiisocyanat und/oder 2,2'-Diphenylmethandiisocyanat), oder mit MDI-Oligomeren oder MDI-Homologen.

Ein geeignetes Isocyanatgruppen aufweisendes Polyurethanpolymer wird insbesondere erhalten aus der Umsetzung von mindestens einem Polyol mit einer überstöchiometrischen Menge von mindestens einem Polyisocyanat, insbesondere einem Diisocyanat. Die Umsetzung wird bevorzugt unter Feuchtigkeitsausschluss bei einer Temperatur im Bereich von 50 bis 160 °C durchgeführt, gegebenenfalls in Anwesenheit geeigneter Katalysatoren. Der Überschuss an Polyisocyanat wird bevorzugt so gewählt, dass im Polyurethanpolymer nach der Umsetzung aller Hydroxylgruppen ein Gehalt an freien Isocyanatgruppen im Bereich von 1 bis 30 Gewichts-%, bevorzugt 1.5 bis 25 Gewichts-%, besonders bevorzugt 2 bis 20 Gewichts-%, verbleibt. Gegebenenfalls kann das Polyurethanpolymer unter Mitverwendung von Weichmachern oder Lösemitteln hergestellt werden, wobei die verwendeten Weichmacher oder Lösemittel keine gegenüber Isocyanaten reaktive Gruppen enthalten.

Dafür geeignete Diisocyanate sind insbesondere MDI, TDI, PMDI, HDI, IPDI, H₁₂MDI, oder Oligomere oder Derivate dieser Diidocyanate.

Dafür geeignete Polyole sind insbesondere Polyetherpolyole, bevorzugt Polyoxyalkylenpolyole, welche Polymerisationsprodukte von Ethylenoxid oder 1,2-Propylenoxid oder 1,2- oder 2,3-Butylenoxid oder Oxetan oder Tetrahydrofuran oder Mischungen davon sind, eventuell polymerisiert mit Hilfe eines Startermoleküls mit zwei oder mehreren aktiven Wasserstoffatomen; Polyesterpolyole, bevorzugt Produkte aus der Polykondensation von Diolen oder Triolen mit Lactonen oder Dicarbonsäuren oder deren Estern oder Anydriden; Polycarbonatpolyole; OH-endständige Blockcopolymere mit mindestens zwei unterschiedlichen Blöcken mit Polyether-, Polyester- oder Polycarbonat-Einheiten; Polyacrylat- oder Polymethacrylat-Polyole; Polyhydroxy-funktionelle Fette oder Öle, insbesondere natürliche Fette oder Öle; oder Polykohlenwasserstoffpolyole wie beispielsweise Polyhydroxy-funktionelle Polyolefine, insbesondere Polybutadienpolyole.

Geeignet sind insbesondere auch Mischungen der genannten Polyole. Geeignet sind insbesondere Diole oder Triole oder Mischungen davon.

Das Isocyanatgruppen aufweisende Polyurethanpolymer weist bevorzugt ein mittleres Molekulargewicht im Bereich von 350 bis 30'000 g/mol, insbesondere 1'000 bis 15'000 g/mol, auf.

Besonders bevorzugt ist funktionelle Verbindung, für deren Vernetzung die Verbindung enthaltend mindestens eine Hexahydrotriazin-Einheit der Formel (I) verwendet wird, ein Silangruppen aufweisendes Polymer.

Die Verbindung enthaltend mindestens eine Hexahydrotriazin-Einheit der Formel (I) wirkt stark katalytisch auf die Hydrolyse- und Kondensationsreaktionen von Silangruppen aufweisenden Polymeren. Ein Silangruppen aufweisendes Polymer härtet deshalb schon mit einer relativ geringen Menge einer Verbindung enthaltend mindestens eine Hexahydrotriazin-Einheit der Formel (I) als Katalysator rasch aus und ist damit besonders wenig anfällig auf migrationsbedingte Fehler wie Separation oder Ausschwitzen und besonders kostengünstig.

Das Silangruppen aufweisende Polymer ist insbesondere ausgewählt aus der Gruppe bestehend aus Polyorganosiloxanen mit endständigen Silangruppen und Silangruppen aufweisenden organischen Polymeren, wie sie im Folgenden genauer beschrieben werden.

Ein Polyorganosiloxan mit endständigen Silangruppen weist den Vorteil auf, dass es besonders wasser- und lichtbeständig ist und besonders weichelastische Eigenschaften ermöglicht.

Ein Silangruppen aufweisendes organisches Polymer weist den Vorteil auf, dass es besonders gute Haftungseigenschaften auf einer Vielzahl von Untergründen aufweist und besonders kostengünstig ist.

Ein weiterer Gegenstand der Erfindung ist eine härtbare Zusammensetzung enthaltend mindestens eine Verbindung enthaltend mindestens eine Hexahydrotriazin-Einheit der Formel (I) als Katalysator. Dabei beschleunigt diese die Vernetzung bzw. Aushärtung der Zusammensetzung.

Bevorzugt enthält die härtbare Zusammensetzung funktionelle Gruppen ausgewählt aus Isocyanatgruppen, Silangruppen, Epoxidgruppen und Cyanatestergruppen.

Besonders bevorzugt enthält die härtbare Zusammensetzung Isocyanatgruppen und/oder Silangruppen, insbesondere Silangruppen.

Eine härtbare Zusammensetzung, welche Isocyanatgruppen enthält, wird auch als härtbare Polyurethanzusammensetzung bezeichnet.

Bevorzugt enthält die härtbare Zusammensetzung mindestens ein Polyisocyanat oder mindestens ein Isocyanatgruppen aufweisendes Polyurethanpolymer oder mindestens ein Silangruppen aufweisendes Polymer, wie vorgängig beschrieben.

Bevorzugt wird die härtbare Zusammensetzung eingesetzt zum Verkleben, Abdichten, Dämmen, Beschichten oder Vorbehandeln in Bau- und Industrieanwendungen, insbesondere als Betonelementklebstoff, Fassadenklebstoff, Parkettklebstoff, Fensterprofilklebstoff, Verankerungsklebstoff, Montageklebstoff, Karosserieklebstoff, Scheibenklebstoff, Sandwichelementklebstoff, Kaschierklebstoff, Laminatklebstoff, Verpackungsklebstoff, Fugendichtstoff, Bodenfuge, Spachtelmasse, Abdichtungsmembran, Bördel- oder Schweissnahtdichtstoff, Hohlraumversiegelung, Bauschaum, Möbelschaum, Filterschaum, Isolationsschaum, Schalldämmschaum, Verpackungsschaum, Karosserieschaum, Bodenbelag, Bodenbeschichtung, Balkonbeschichtung, Dachbeschichtung, Betonschutzbeschichtung, Parkhausbeschichtung, Rohrbeschichtung, Korrosionsschutzbeschichtung, Textilbeschichtung, Primer, Aktivator oder Haftbrücke, oder als Formteil, Halbzeug, Folie oder Faser, insbesondere als Kissen, Polster, Matratze, Schuhsohle, Stossdämpfer, Dämpfungselement, Dichtung, Reifen, Rolle, Lager, Walze, Förderband, Schlauch, Gehäuse, Fensterprofil, Isolationsplatte, Modellbauplatte, Sandwichelement, Faserverbundkörper, Implantat, Verpackungsfolie, Kaschierfolie oder Textilfaser.

Insbesondere stellt die härtbare Zusammensetzung einen Klebstoff oder einen Dichtstoff oder eine Beschichtung dar.

Besonders bevorzugt enthält die härtbare Zusammensetzung mindestens ein Silangruppen aufweisendes Polymer, insbesondere ausgewählt aus der Gruppe bestehend aus Polyorganosiloxanen mit endständigen Silangruppen und Silangruppen aufweisenden organischen Polymeren.

Eine solche Zusammensetzung verfügt über eine gute Lagerstabilität ohne Separationsneigung, erlaubt aufgrund der geringen Toxizität und geringen Flüchtigkeit der Verbindung enthaltend mindestens eine Hexahydrotriazin-Einheit der Formel (I) eine niedrige Gefahreneinstufung und ermöglicht emissions- und geruchsarme Produkte, welche rasch aushärten und dabei ein mechanisch hochwertiges und beständiges Material bilden. Besonders vorteilhaft ist dabei der Umstand, dass dieses Material kaum zu migrationsbedingten Fehlern wie Ausschwitzen oder Substratverschmutzung neigt, im Gegensatz zu Zusammensetzungen mit Katalysatoren nach dem Stand der Technik, wie beispielsweise DBU oder TMG. Zusammensetzungen enthaltend solche aus dem Stand der Technik bekannten Katalysatoren neigen zu Migrationseffekten, was sich vor der Aushärtung durch Separation und nach der Aushärtung durch klebrige und/oder schmierige Oberflächen und/oder Substratverschmutzungen äussern kann. Besonders letztere Effekte sind äusserst unerwünscht, da klebrige und schmierige Oberflächen schnell verschmutzen und schlecht überstreichbar sind, und Substratverunreinigungen zu bleibenden Verfärbungen führen können.

Das Silangruppen aufweisende Polymer ist in einer bevorzugten Ausführungsform ein Polyorganosiloxan mit endständigen Silangruppen.

Ein bevorzugtes Polyorganosiloxan mit endständigen Silangruppen weist die Formel (VI) auf, 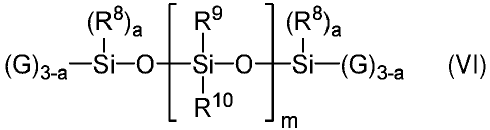 wobei
R⁸, R⁹ und R¹⁰ unabhängig voneinander jeweils für einen einwertigen Kohlenwasserstoff-Rest mit 1 bis 12 C-Atomen stehen,
G für einen Hydroxyl-Rest oder für einen Alkoxy-, Acetoxy-, Ketoximato-, Amido- oder Enoxy-Rest mit 1 bis 13 C-Atomen steht,
a für 0, 1 oder 2 steht, und
m für eine ganze Zahl im Bereich von 50 bis etwa 2'500 steht.
R⁸ steht bevorzugt für Methyl, Vinyl oder Phenyl.
R⁹ und R¹⁰ stehen bevorzugt unabhängig voneinander jeweils für einen Alkyl-rest mit 1 bis 5, bevorzugt 1 bis 3 C-Atomen, insbesondere für Methyl.
G steht bevorzugt für einen Hydroxyl-Rest oder für einen Alkoxy- oder Ketoximato-Rest mit 1 bis 6 C-Atomen, insbesondere für einen Hydroxyl-, Methoxy-, Ethoxy-, Methylethylketoximato- oder Methylisobutylketoximato-Rest. Besonders bevorzugt steht G für einen Hydroxylrest.
a steht bevorzugt für 0 oder 1, insbesondere für 0.

Weiterhin ist m bevorzugt so gewählt, dass das Polyorganosiloxan der Formel (VI) bei Raumtemperatur eine Viskosität im Bereich von 100 bis 500'000 mPa·s, insbesondere von 1'000 bis 100'000 mPa·s, aufweist.

Polyorganosiloxane der Formel (VI) sind gut handhabbar und vernetzen mit Feuchtigkeit und/oder Silanvernetzern zu festen Silicon-Polymeren mit elastischen Eigenschaften.

Geeignete kommerziell erhältliche Polyorganosiloxane der Formel (VI) sind beispielsweise erhältlich von Wacker, Momentive Performance Materials, GE Advanced Materials, Dow Corning, Bluestar Silicones oder Shin-Etsu.

Bevorzugt enthält die Zusammensetzung zusätzlich zum Polyorganosiloxan mit endständigen Silangruppen einen Silanvernetzer, insbesondere ein Silan der Formel (VII),

(R¹¹)_{q}-Si-(G')_{4-q} (VII)

wobei
R¹¹ für einen einwertigen Kohlenwasserstoff-Rest mit 1 bis 12 C-Atomen steht, G' für einen Hydroxyl-Rest oder für einen Alkoxy-, Acetoxy-, Ketoximato-, Amido- oder Enoxy-Rest mit 1 bis 13 C-Atomen steht, und
q für einen Wert von 0, 1 oder 2, insbesondere für 0 oder 1, steht.

Besonders geeignete Silane der Formel (VII) sind Methyltrimethoxysilan, Ethyltrimethoxysilan, Propytrimethoxysilan, Vinyltrimethoxysilan, Phenyltrimethoxysilan, Methyltriethoxysilan, Vinyltriethoxysilan, Phenyltriethoxysilan, Tetramethoxysilan, Tetraethoxysilan, Methyltris(methylethylketoximo)silan, Vinyltris(methylethylketoximo)silan oder Methyltris(isobutylketoximo)silan.

Das Silangruppen aufweisende Polymer ist in einer weiteren bevorzugten Ausführungsform ein Silangruppen aufweisendes organisches Polymer, insbesondere ein Polyolefin, Polyether, Polyester, Polyamid, Poly(meth)acrylat oder eine Mischform dieser Polymere, welches jeweils eine oder bevorzugt mehrere Silangruppen trägt. Die Silangruppen können seitlich in der Kette oder endständig stehen und sind über ein C-Atom an das organische Polymer gebunden.

Besonders bevorzugt ist das Silangruppen aufweisende organische Polymer ein Silangruppen aufweisendes Polyolefin oder ein Silangruppen aufweisender Polyether oder ein Silangruppen aufweisender Polyester oder ein Silangruppen aufweisendes Poly(meth)acrylat oder eine Mischform dieser Polymere.

Am meisten bevorzugt ist das Silangruppen aufweisende organische Polymer ein Silangruppen aufweisender Polyether.

Das Silangruppen aufweisende organische Polymer weist als Silangruppen bevorzugt Alkoxysilangruppen auf, insbesondere Alkoxysilangruppen der Formel (VIII), 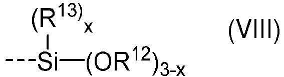 wobei
R¹² für einen linearen oder verzweigten, einwertigen Kohlenwasserstoffrest mit 1 bis 5 C-Atomen, insbesondere für Methyl, Ethyl oder Isopropyl, steht,
R¹³ für einen linearen oder verzweigten, einwertigen Kohlenwasserstoffrest mit 1 bis 8 C-Atomen, insbesondere für Methyl, steht, und
x für einen Wert von 0 oder 1 oder 2, bevorzugt für 0 oder 1, insbesondere für 0, steht.

Besonders bevorzugt steht R¹² für Methyl oder Ethyl.

Besonders bevorzugt sind Trimethoxysilangruppen, Dimethoxymethylsilangruppen oder Triethoxysilangruppen.

Dabei weisen Methoxysilangruppen den Vorteil auf, dass sie besonders reaktiv sind und schnell vernetzen, und Ethoxysilangruppen weisen den Vorteil auf, dass sie besonders lagerstabil sind und bei der Vernetzung wenig toxisches Ethanol freisetzen.

Das Silangruppen aufweisende organische Polymer weist im Mittel bevorzugt 1.3 bis 4, insbesondere 1.5 bis 3, besonders bevorzugt 1.7 bis 2.8, Silangruppen pro Molekül auf. Die Silangruppen sind bevorzugt endständig.

Das Silangruppen aufweisende organische Polymer weist bevorzugt ein mittleres Molekulargewicht im Bereich von 1'000 bis 30'000 g/mol, insbesondere 2'000 bis 20'000 g/mol, auf.

Das Silangruppen aufweisende organische Polymer weist bevorzugt ein Silan-Equivalentgewicht von 300 bis 25'000 g/Eq, insbesondere 500 bis 15'000 g/Eq, auf.

Das Silangruppen aufweisende organische Polymer kann bei Raumtemperatur fest oder flüssig vorliegen. Bevorzugt ist es bei Raumtemperatur flüssig.

Meist bevorzugt handelt es sich beim Silangruppen aufweisenden organischen Polymer um einen bei Raumtemperatur flüssigen Silangruppen aufweisenden Polyether, wobei die Silangruppen insbesondere Dialkoxysilangruppen und/oder Trialkoxysilangruppen, besonders bevorzugt Trimethoxysilangruppen oder Triethoxysilangruppen, sind.

Verfahren zur Herstellung von Silangruppen aufweisenden Polyethern sind dem Fachmann bekannt.

In einem bevorzugten Verfahren sind Silangruppen aufweisende Polyether erhältlich aus der Umsetzung von Allylgruppen-haltigen Polyethern mit Hydrosilanen, gegebenenfalls unter Kettenverlängerung mit beispielsweise Diisocyanaten.

In einem weiteren bevorzugten Verfahren sind Silangruppen aufweisende Polyether erhältlich aus der Copolymerisation von Alkylenoxiden und Epoxysilanen, gegebenenfalls unter Kettenverlängerung mit beispielsweise Diisocyanaten.

In einem weiteren bevorzugten Verfahren sind Silangruppen aufweisende Polyether erhältlich aus der Umsetzung von Polyetherpolyolen mit Isocyanatosilanen, gegebenenfalls unter Kettenverlängerung mit Diisocyanaten.

In einem weiteren bevorzugten Verfahren sind Silangruppen aufweisende Polyether erhältlich aus der Umsetzung von Isocyanatgruppen aufweisenden Polyethern, insbesondere NCO-terminierten Urethan-Polyethern aus der Umsetzung von Polyetherpolyolen mit einer überstöchiometischen Menge an Polyisocyanaten, mit Aminosilanen, Hydroxysilanen oder Mercaptosilanen. Silangruppen aufweisende Polyether aus diesem Verfahren sind besonders bevorzugt. Dieses Verfahren ermöglicht den Einsatz einer Vielzahl von kommerziell gut verfügbaren, kostengünstigen Ausgangsmaterialien, womit unterschiedliche Polymereigenschaften erhalten werden können, beispielsweise eine hohe Dehnbarkeit, eine hohe Festigkeit, ein niedriges Elastizitätsmodul, ein tiefer Glasübergangspunkt oder eine hohe Witterungsbeständigkeit.

Besonders bevorzugt ist der Silangruppen aufweisende Polyether erhältlich aus der Umsetzung von NCO-terminierten Urethan-Polyethern mit Aminosilanen oder Hydroxysilanen. Geeignete NCO-terminierte Urethan-Polyether sind erhältlich aus der Umsetzung von Polyetherpolyolen, insbesondere Polyoxyalkylendiolen oder Polyoxyalkylentriolen, bevorzugt Polyoxypropylendiolen oder Polyoxypropylentriolen, mit einer überstöchiometrischen Menge an Polyisocyanaten, insbesondere Diisocyanaten.

Bevorzugt wird die Umsetzung zwischen dem Polyisocyanat und dem Polyetherpolyol unter Feuchtigkeitsausschluss bei einer Temperatur von 50°C bis 160°C durchgeführt, gegebenenfalls in Anwesenheit geeigneter Katalysatoren, wobei das Polyisocyanat so dosiert ist, dass dessen Isocyanatgruppen im Verhältnis zu den Hydroxylgruppen des Polyols im stöchiometrischen Überschuss vorhanden sind. Insbesondere wird der Überschuss an Polyisocyanat so gewählt, dass im resultierenden Urethan-Polyether nach der Umsetzung aller Hydroxylgruppen ein Gehalt an freien Isocyanatgruppen von 0.1 bis 5 Gewichts- %, bevorzugt 0.2 bis 4 Gewichts-%, besonders bevorzugt 0.3 bis 3 Gewichts- %, verbleibt.

Bevorzugte Diisocyanate sind ausgewählt aus der Gruppe bestehend aus HDI, IPDI, TDI und MDI. Besonders bevorzugt sind IPDI oder TDI. Meist bevorzugt ist IPDI. Damit werden Silangruppen aufweisende Polyether mit besonders guter Lichtechtheit erhalten.

Speziell geeignet als Polyetherpolyole sind Polyoxyalkylendiole oder Polyoxyalkylentriole mit einem Ungesättigtheitsgrad tiefer als 0.02 mEq/g, insbesondere tiefer als 0.01 mEq/g, und einem mittleren Molekulargewicht im Bereich von 400 bis 25'000 g/mol, insbesondere 1'000 bis 20'000 g/mol.

Neben Polyetherpolyolen können anteilig auch andere Polyole eingesetzt werden, insbesondere Polyacrylatpolyole, sowie niedrigmolekulare Diole oder Triole.

Geeignete Aminosilane für die Umsetzung mit einem NCO-terminierten Urethan-Polyether sind primäre und sekundäre Aminosilane. Bevorzugt sind 3-Aminopropyltrimethoxysilan, 3-Aminopropyldimethoxymethylsilan, 4-Aminobutyltrimethoxysilan, 4-Amino-3-methylbutyltrimethoxysilan, 4-Amino-3,3-dimethylbutyltrimethoxysilan, N-Butyl-3-aminopropyltrimethoxysilan, N-Phenyl-3-aminopropyltrimethoxysilan, Addukte aus primären Aminosilanen wie 3-Aminopropyltrimethoxysilan, 3-Aminopropyldimethoxymethylsilan oder N-(2-Aminoethyl)-3-aminopropyltrimethoxysilan und Michael-Akzeptoren wie Acrylnitril, (Meth)acrylsäureestern, (Meth)acrylsäureamiden, Maleinsäure- oder Fumarsäurediestern, Citraconsäurediestern oder Itaconsäurediestern, insbesondere N-(3-Trimethoxysilylpropyl)aminobernsteinsäuredimethyl- oder -diethylester. Ebenfalls geeignet sind Analoga der genannten Aminosilane mit Ethoxy- oder Isopropoxygruppen anstelle der Methoxygruppen am Silicium.

Geeignete Hydroxysilane für die Umsetzung mit einem NCO-terminierten Urethan-Polyether sind insbesondere erhältlich aus der Addition von Aminosilanen an Lactone oder an cyclische Carbonate oder an Lactide.

Dafür geeignete Aminosilane sind insbesondere 3-Aminopropyltrimethoxysilan, 3-Aminopropyltriethoxysilan, 4-Aminobutyltrimethoxysilan, 4-Aminobutyltriethoxysilan, 4-Amino-3-methylbutyltrimethoxysilan, 4-Amino-3-methylbutyltriethoxysilan, 4-Amino-3,3-dimethylbutyltrimethoxysilan, 4-Amino-3,3-dimethylbutyltriethoxysilan, 2-Aminoethyltrimethoxysilan oder 2-Aminoethyltriethoxysilan. Besonders bevorzugt sind 3-Aminopropyltrimethoxysilan, 3-Aminopropyltriethoxysilan, 4-Amino-3,3-dimethylbutyltrimethoxysilan oder 4-Amino-3,3-dimethylbutyltriethoxysilan.

Als Lactone geeignet sind insbesondere γ-Valerolacton, γ-Octalacton, δ-Decalacton oder ε-Decalacton, insbesondere γ-Valerolacton.

Als cyclische Carbonate geeignet sind insbesondere 4,5-Dimethyl-1,3-dioxolan-2-on, 4,4-Dimethyl-1,3-dioxolan-2-on, 4-Ethyl-1,3-dioxolan-2-on, 4-Methyl-1,3-dioxolan-2-on oder 4-(Phenoxymethyl)-1,3-dioxolan-2-on.

Als Lactide geeignet sind insbesondere 1,4-Dioxan-2,5-dion (Lactid aus 2-Hydroxyessigsäure, auch "Glycolid" genannt), 3,6-Dimethyl-1,4-dioxan-2,5-dion (Lactid aus Milchsäure, auch "Lactid" genannt) oder 3,6-Diphenyl-1,4-dioxan-2,5-dion (Lactid aus Mandelsäure).

Bevorzugte Hydroxysilane, welche auf diese Weise erhalten werden, sind N-(3-Triethoxysilylpropyl)-2-hydroxypropanamid, N-(3-Triethoxysilylpropyl)-4-hydroxypentanamid, N-(3-Triethoxysilylpropyl)-4-hydroxyoctanamid, N-(3-Triethoxysilylpropyl)-5-hydroxydecanamid, N-(3-Triethoxysilylpropyl)-2-hydroxypropylcarbamat und die entsprechenden Silane mit Methoxy- anstelle der EthoxyGruppen.

Weiterhin sind geeignete Hydroxysilane auch erhältlich aus der Addition von Aminosilanen an Epoxide oder aus der Addition von Aminen an Epoxysilane. Bevorzugte Hydroxysilane, welche auf diese Weise erhalten werden, sind 2-Morpholino-4(5)-(2-trimethoxysilylethyl)cyclohexan-1-ol, 2-Morpholino-4(5)-(2-triethoxysilylethyl)cyclohexan-1-ol oder 1-Morpholino-3-(3-(triethoxysilyl)propoxy)propan-2-ol.

Als Silangruppen aufweisende Polyether sind auch kommerziell erhältliche Produkte geeignet, insbesondere die Folgenden: MS Polymer™ (von Kaneka Corp.; insbesondere die Typen S203H, S303H, S227, S810, MA903 und S943); MS Polymer™ bzw. Silyl™ (von Kaneka Corp.; insbesondere die Typen SAT010, SAT030, SAT200, SAX350, SAX400, SAX725, MAX450, MAX951); Excestar® (von Asahi Glass Co. Ltd.; insbesondere die Typen S2410, S2420, S3430, S3630); SPUR+* (von Momentive Performance Materials; insbesondere die Typen 1010LM, 1015LM, 1050MM); Vorasil™ (von Dow Chemical Co.; insbesondere die Typen 602 und 604); Desmoseal® (von Covestro AG; insbesondere die Typen S XP 2458, S XP 2636, S XP 2749, S XP 2774 und S XP 2821), TEGOPAC® (von Evonik Industries AG; insbesondere die Typen Seal 100, Bond 150, Bond 250), Polymer ST (von Hanse Chemie AG/Evonik Industries AG, insbesondere die Typen 47, 48, 61, 61LV, 77, 80, 81); Geniosil® STP (von Wacker Chemie AG; insbesondere die Typen E10, E15, E30, E35).

Besonders bevorzugte Silangruppen aufweisende organische Polymere weisen Endgruppen der Formel (IX) auf, 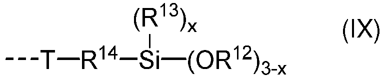 wobei
R¹⁴ für einen zweiwertigen, linearen oder verzweigten Kohlenwasserstoffrest mit 1 bis 12 C-Atomen, welcher gegebenenfalls cyclische und/oder aromatische Anteile und gegebenenfalls ein oder mehrere Heteroatome, insbesondere ein oder mehrere Stickstoffatome, aufweist, steht,
T für einen zweiwertigen Rest ausgewählt aus -O-, -S-, -N(R¹⁵)-, -O-CO-N(R¹⁵)-, -N(R¹⁵)-CO-O- und -N(R¹⁵)-CO-N(R¹⁵)- steht,
   wobei R¹⁵ für einen Wasserstoff-Rest oder für einen linearen oder verzweigten Kohlenwasserstoff-Rest mit 1 bis 20 C-Atomen, welcher gegebenenfalls cyclische Anteile aufweist, und welcher gegebenenfalls eine Alkoxysilan-, Ether- oder Carbonsäureester-Gruppe aufweist, steht, und
R¹², R¹³ und x die bereits genannten Bedeutungen aufweisen.

Bevorzugt steht R¹⁴ für 1,3-Propylen oder für 1,4-Butylen, wobei Butylen mit einer oder zwei Methylgruppen substituiert sein kann.

Besonders bevorzugt steht R¹⁴ für 1,3-Propylen.

Bevorzugt ist die Verbindung enthaltend mindestens eine Hexahydrotriazin-Einheit der Formel (I) in der härtbaren Zusammensetzung in einer solchen Menge vorhanden, dass die Konzentration an Amidin- und/oder Guanidin-Gruppen bezogen auf die Menge des funktionellen Polymers im Bereich von 0.1 bis 50 mmol/100 g, bevorzugt 0.2 bis 50 mmol/100 g, insbesondere 0.5 bis 20 mmol/100 g, liegt.

Eine solche Zusammensetzung weist eine gute Lagerfähigkeit und eine schnelle Aushärtung auf.

Zusätzlich zur Verbindung enthaltend mindestens eine Hexahydrotriazin-Einheit der Formel (I) kann die Zusammensetzung weitere Katalysatoren, welche insbesondere die Vernetzung von Isocyanatgruppen und/oder Silangruppen katalysieren, enthalten. Als weitere Katalysatoren geeignet sind insbesondere Metall-Verbindungen und/oder basische Stickstoff- oder Phosphorverbindungen.

Geeignete Metall-Verbindungen sind insbesondere Verbindungen von Zinn, Titan, Zirkonium, Aluminium oder Zink, insbesondere Diorganozinn(IV)-Verbindungen wie insbesondere Dibutylzinn(IV)-diacetat, Dibutylzinn(IV)-dilaurat, Dibutylzinn(IV)-dineodecanoat oder Dibutylzinn(IV)-bis(acetylacetonat) und Dioctylzinn(IV)-dilaurat, sowie Titan(IV)- oder Zirkonium(IV)- oder Aluminium(III)- oder Zink(II)-Komplexe mit insbesondere Alkoxy-, Carboxylat-, 1,3-Diketonat-, 1,3-Ketoesterat- oder 1,3-Ketoamidat-Liganden.

Geeignete basische Stickstoff- oder Phosphorverbindungen sind insbesondere Imidazole, Pyridine, Phosphazen-Basen oder bevorzugt Amine, weitere Hexahydrotriazine, Biguanide, weitere Guanidine oder weitere Amidine.

Geeignete Amine sind insbesondere Alkyl-, Cycloalkyl- oder Aralkylamine wie Triethylamin, Triisopropylamin, 1-Butylamin, 2-Butylamin, tert.Butylamin, 3-Methyl-1-butylamin, 3-Methyl-2-butylamin, Dibutylamin, Tributylamin, Hexylamin, Dihexylamin, Cyclohexylamin, Dicyclohexylamin, Dimethylcyclohexylamin, Benzylamin, Dibenzylamin, Dimethylbenzylamin, Octylamin, 2-Ethylhexylamin, Di-(2-ethylhexyl)amin, Laurylamin, N,N-Dimethyl-laurylamin, Stearylamin, N,N-Dimethyl-stearylamin; von natürlichen Fettsäuregemischen abgeleitete Fettamine; aliphatische, cycloaliphatische oder araliphatische Diamine wie Ethylendiamin, Butandiamin, Hexamethylendiamin, Dodecandiamin, Neopentandiamin, 2-Methylpentamethylendiamin (MPMD), 2,2(4),4-Trimethylhexamethylendiamin (TMD), Isophorondiamin (IPD), 2,5(2,6)-Bis-(aminomethyl)-bi-cyclo[2.2.1]heptan (NBDA), 1,3-Xylylendiamin (MXDA), N,N'-Di(tert.butyl)ethylendiamin, N,N,N',N'-Tetramethylethylendiamin, N,N,N',N'-Tetramethylpropy-lendiamin, N,N,N',N'-Tetramethylhexamethylendiamin, 3-(Dimethylamino)propylamin, 3-(Methylamino)propylamin, 3-(Cyclohexylamino)propylamin, Piperazin, N-Methylpiperazin, N,N'-Dimethylpiperazin, 1,4-Diazabicyclo[2.2.2]octan (DABCO), Fettpolyamine wie N-Cocoalkyl-1,3-propandiamin; Polyalkylenamine wie Diethylentriamin, Dipropylentriamin, Triethylentetramin (TETA), Tetraethylenpentamin (TEPA), Pentamethylenhexamin (PEHA), 3-(2-Aminoethyl)-aminopropylamin, N,N'-Bis(3-aminopropyl)ethylendiamin, N-(3-Aminopropyl)-N-methylpropandiamin, Bis(3-dimethylaminopropyl)amin, N-(3-Dimethylaminopropyl)-1,3-propylendiamin, N-(2-Aminoethyl)piperazin (N-AEP), N-(2-Aminopropyl)piperazin, N,N'-Di-(2-aminoethyl)piperazin, 1-Methyl-4-(2-dimethylaminoethyl)piperazin, N,N,N',N",N"-Pentamethyldiethylentriamin, N,N,N',N",N"-Pentamethyldipropylentriamin, Polyethylenimine, erhältlich beispielsweise unter den Handelsnamen Lupasol® (von BASF) und Epomin® (von Nippon Shokubai); Etheramine wie insbesondere 2-Methoxyethylamin, 2-Ethoxyethylamin, 3-Methoxypropylamin, 3-Ethoxypropylamin, 3-(2-Ethylhexyloxy)propylamin, 3-(2-Methoxyethoxy)propylamin, Morpholin, N-Methylmorpholin, N-Ethylmorpholin, 2-Aminoethylmorpholin, Bis(2-aminoethyl)ether, Bis(dimethylaminoethyl)ether, Bis(morpholinoethyl)ether (DMDEE), N,N,N'-Trimethyl-N'-hydroxy-ethylbis(2-aminoethyl)ether, 3,6-Dioxaoctan-1,8-diamin, 4,7-Dioxadecan-1,10-diamin, 4,7-Dioxadecan-2,9-diamin, 4,9-Dioxadodecan-1,12-diamin, 5,8-Dioxadodecan-3,10-diamin, 4,7,10-Trioxatridecan-1,13-diamin oder 2-Aminopropylterminierte Glykole, wie sie beispielsweise unter dem Handelsnamen Jeffamin® (von Huntsman) erhältlich sind; Aminoalkohole, wie insbesondere Ethanolamin, Isopropanolamin, Diethanolamin, Diisopopanolamin, Triethanolamin, Triisopropanolamin, N-Butylethanolamin, Diglykolamin, N,N-Diethylethanolamin, N-Methyldiethanolamin, N-Methyldiisopropylamin, N,N,N'-Trimethylaminoethyl-ethanolamin, N-(3-Dimethylaminopropyl)-N,N-diisopropanolamin, N,N-Bis(3-dimethylaminopropyl)-N-isopropanolamin, 2-(2-Dimethylaminoethoxy)ethanolamin oder Addukte aus Mono- und Polyaminen mit Epoxiden oder Diepoxiden; Phenolgruppen-haltige Amine, wie insbesondere Kondensationsprodukte aus Phenolen, Aldehyden und Aminen (sogenannte Mannich-Basen und Phenalkamine) wie insbesondere 2-(Dimethylaminomethyl)phenol, 2,4,6-Tris(dimethylaminomethyl)phenol oder Polymere aus Phenol, Formaldehyd und N,N-Dimethyl-1,3-propandiamin sowie im Handel unter den Markennamen Cardolite® (von Cardolite), Aradur® (von Huntsman) und Beckopox® (von Cytec) erhältliche Phenalkamine; Amidgruppen-haltige Polyamine, sogenannte Polyamidoamine, wie sie im Handel erhältlich sind, beispielsweise unter den Markennamen Versamid® (von Cognis), Aradur® (von Huntsman), Euretek® (von Huntsman) oder Beckopox® (von Cytec); oder Aminosilane, wie insbesondere 3-Aminopropyltrimethoxysilan, 3-Aminopropyldimethoxymethylsilan, N-(2-Aminoethyl)-3-aminopropyltrimethoxysilan, N-(2-Aminoethyl)-3-aminopropyl-methyl-dimethoxysilan, N-(2-Aminoethyl)-N'-[3-(trimethoxysilyl)propyl]ethylendiamin oder deren Analoga mit Ethoxy- anstelle der Methoxygruppen am Siliciumatom.

Geeignete weitere Hexahydrotriazine sind insbesondere 1,3,5-Hexahydrotriazin, 1,3,5-Trimethylhexahydrotriazin oder 1,3,5-Tris(3-(dimethylamino)propyl)-hexahydrotriazin.

Geeignete Biguanide sind insbesondere Biguanid, 1-Butylbiguanid, 1,1-Dimethylbiguanid, 1-Butylbiguanid, 1-Phenylbiguanid oder 1-(o-Tolyl)biguanid (OTBG).

Geeignete weitere Guanidine sind insbesondere 1-Butylguanidin, 1,1-Dimethylguanidin, 1,3-Dimethylguanidin, 1,1,3,3-Tetramethylguanidin (TMG), 2-(3-(Tri-methoxysilyl)propyl)-1,1,3,3-tetramethylguanidin, 2-(3-(Methyldimethoxysilyl)-propyl)-1,1,3,3-tetramethylguanidin, 2-(3-(Triethoxysilyl)propyl)-1,1,3,3-tetramethylguanidin, 1,5,7-Triazabicyclo[4.4.0]dec-5-en (TBD), 7-Methyl-1,5,7-tri-azabicyclo[4.4.0]dec-5-en, 7-Cyclohexyl-1,5,7-triazabicyclo[4.4.0]dec-5-en, 1-Phenylguanidin, 1-(o-Tolyl)guanidin (OTG), 1,3-Diphenylguanidin, 1,3-Di(o-to-lyl)guanidin oder 2-Guanidinobenzimidazol.

Geeignete weitere Amidine sind insbesondere 1,8-Diazabicyclo[5.4.0]undec-7-en (DBU), 1,5-Diazabicyclo[4.3.0]non-5-en (DBN), 6-Dibutylamino-1,8-diazabi-cyclo[5.4.0]undec-7-en, 6-Dibutylamino-1,8-diazabicyclo[5.4.0]undec-7-en, N,N'-Di-n-hexylacetamidin (DHA), 2-Methyl-1,4,5,6-tetrahydropyrimidin, 1,2-Dimethyl-1,4,5,6-tetrahydropyrimidin, 2,5,5-Trimethyl-1,4,5,6-tetrahydropyri-midin, N-(3-Trimethoxysilylpropyl)-4,5-dihydroimidazol oder N-(3-Triethoxy-silylpropyl)-4,5-dihydroimidazol.

Weiterhin kann die Zusammensetzung als Co-Katalysator eine Säure enthalten, insbesondere eine Carbonsäure. Bevorzugt sind aliphatische Carbonsäuren wie Ameisensäure, Laurinsäure, Stearinsäure, Isostearinsäure, Ölsäure, 2-Ethyl-2,5-dimethylcapronsäure, 2-Ethylhexansäure, Neodecansäure, Fettsäuregemische aus der Verseifung von natürlichen Fetten und Ölen oder Di- und Polycarbonsäuren, insbesondere Poly(meth)acrylsäuren.

Die Zusammensetzung ist in einer bevorzugten Ausführungsform im Wesentlichen frei von Organozinn-Verbindungen. Organozinn-freie Zusammensetzungen sind hinsichtlich Gesundheitsschutz und Umweltschutz vorteilhaft. Insbesondere beträgt der Zinngehalt der härtbaren Zusammensetzung weniger als 0.1 Gew.-%, insbesondere weniger als 0.05 Gew.-%.

Die Zusammensetzung enthält in einer weiteren bevorzugten Ausführungsform eine Kombination aus mindestens einer Verbindung enthaltend mindestens eine Hexahydrotriazin-Einheit der Formel (I) und mindestens einer Organozinn-Verbindung, insbesondere einer Diorganozinn(IV)-Verbindung wie die vorgängig genannten. Eine solche Zusammensetzung weist bereits bei einem geringen Zinn-Gehalt eine hohe Aushärtungsgeschwindigkeit auf, was aus toxikologischen und ökologischen Gründen vorteilhaft ist.

In einer Ausführungsform enthält die Zusammensetzung neben der Verbindung enthaltend mindestens eine Hexahydrotriazin-Einheit der Formel (I) zusätzlich mindestens ein Organotitanat. Eine Kombination aus einer Verbindung enthaltend mindestens eine Hexahydrotriazin-Einheit der Formel (I) und einem Organotitanat weist eine besonders hohe katalytische Aktivität auf. Dadurch wird eine schnelle Aushärtung bei einer verhältnismässig geringen Einsatzmenge an Organotitanat ermöglicht.

Als Organotitanat geeignet sind insbesondere Titan(IV)-Komplexverbindungen. Bevorzugte Organoititanate sind insbesondere ausgewählt aus
- Titan(IV)-Komplexverbindungen mit zwei 1,3-Diketonat-Liganden, insbesondere 2,4-Pentandionat (=Acetylacetonat), und zwei Alkoholat-Liganden;
- Titan(IV)-Komplexverbindungen mit zwei 1,3-Ketoesterat-Liganden, insbesondere Ethylacetoacetat, und zwei Alkoholat-Liganden;
- Titan(IV)-Komplexverbindungen mit einem oder mehreren Aminoalkoholat-Liganden, insbesondere Triethanolamin oder 2-((2-Aminoethyl)amino)ethanol, und einem oder mehreren Alkoholat-Liganden;
- Titan(IV)-Komplexverbindungen mit vier Alkoholat-Liganden (Orthotitanate);
- sowie höherkondensierte Organotitanate, insbesondere oligomeres Titan(IV)-tetrabutanolat, auch als Polybutyltitanat bezeichnet;
wobei als Alkoholat-Liganden insbesondere Isobutoxy, n-Butoxy, Isopropoxy, Ethoxy und 2-Ethylhexoxy geeignet sind.

Insbesondere geeignet sind die kommerziell erhältlichen Typen Tyzor® AA, GBA, GBO, AA-75, AA-65, AA-105, DC, BEAT, BTP, TE, TnBT, KTM, TOT, TPT oder IBAY (alle von Dorf Ketal); Tytan PBT, TET, X85, TAA, ET, S2, S4 oder S6 (alle von Borica Company Ltd.) und Ken-React® KR® TTS, 7, 9QS, 12, 26S, 33DS, 38S, 39DS, 44, 134S, 138S, 133DS, 158FS oder LICA® 44 (alle von Kenrich Petrochemicals).

Ganz besonders geeignete Organotitanate sind ausgewählt aus Bis(ethylace-toacetato)diisobutoxy-titan(IV) (kommerziell erhältlich beispielsweise als Tyzor® IBAY von Dorf Ketal), Bis(ethylacetoacetato)diisopropoxy-titan(IV) (kommerziell erhältlich beispielsweise als Tyzor® DC von Dorf Ketal), Bis(acetylacetonato)diisopropoxy-titan(IV), Bis(acetylacetonato)diisobutoxy-titan(IV), Tris(oxyethyl)-amin-isopropoxy-titan(IV), Bis[tris(oxyethyl)amin]diisopropoxy-titan(IV), Bis(2-ethylhexan-1,3-dioxy)-titan(IV), Tris[2-((2-Aminoethyl)amino)ethoxy]ethoxy-titan(IV), Bis(neopentyl(diallyl)oxy)-diethoxy-titan(IV), Tetra(isopropoxy)titanat, Tetra(n-butoxy)titanat, Tetra(2-ethylhexyloxy)titanat und Polybutyltitanat. Am meisten bevorzugt sind Bis(ethylacetoacetato)diisobutoxy-titan(IV) oder Bis(ethylacetoacetato)diisopropoxy-titan(IV).

Die härtbare Zusammensetzung enthält bevorzugt mindestens einen weiteren Bestandteil ausgewählt aus der Gruppe bestehend aus Füllstoffen, Weichmachern, Rheologie-Additiven, Trocknungsmitteln, Haftvermittlern und Vernetzern. Besonders bevorzugt enthält sie eine beliebige Kombination von mehreren dieser Bestandteile.

Geeignete Füllstoffe sind insbesondere anorganische oder organische Füllstoffe, insbesondere natürliche, gemahlene oder gefällte Calciumcarbonate, welche gegebenenfalls mit Fettsäuren, insbesondere Stearinsäure, beschichtet sind, Baryt (Schwerspat), Talke, Quarzmehle, Quarzsand, Dolomite, Wollastonite, Kaoline, calcinierte Kaoline, Mica (Kalium-Aluminium-Silikat), Molekularsiebe, Aluminiumoxide, Aluminiumhydroxide, Magnesiumhydroxid, Kieselsäuren inklusive hochdisperse Kieselsäuren aus Pyrolyseprozessen, industriell hergestellte Russe, Graphit, Metall-Pulver wie Aluminium, Kupfer, Eisen, Silber oder Stahl, PVC-Pulver oder Hohlkugeln.

Geeignete Weichmacher sind insbesondere Trialkylsilyl-terminierte Polydialkylsiloxane, vorzugsweise Trimethylsilyl-terminierte Polydimethylsiloxane, insbesondere mit Viskositäten im Bereich von 10 bis 1'000 mPa·s, oder entsprechende Verbindungen, bei denen einige der Methylgruppen ersetzt sind durch andere organische Gruppen, insbesondere Phenyl-, Vinyl- oder Trifluorpropylgruppen, sogenannte Reaktivweichmacher in Form von monofunktionellen, also einseitig reaktiven, Polysiloxanen, Carbonsäureester wie Phthalate, insbesondere Dioctylphthalat, Bis(2-ethylhexyl)phthalat, Bis(3-propylheptyl)phthalat, Diisononylphthalat oder Diisodecylphthalat, Diester von ortho-Cyclohexandicarbonsäure, insbesondere Diisononyl-1,2-cyclohexandicarboxylat, Adipate, insbesondere Dioctyladipat, Bis(2-Ethylhexyl)adipat, Azelate, insbesondere Bis(2-ethylhexyl)acelat, Sebacate, insbesondere Bis(2-ethylhexyl)sebacat oder Diisononylsebacat, Polyole, insbesondere Polyoxyalkylenpolyole oder Polyesterpolyole, Glykolether, Glykolester, organische Phosphor- oder Sulfonsäureester, Sulfonsäureamide, Polybutene oder von natürlichen Fetten oder Ölen abgeleitete Fettsäuremethyl- oder -ethylester, auch "Biodiesel" genannt, wobei Siloxangruppen-haltige Weichmacher besonders geeignet sind für Silangruppen aufweisende Polymere in Form von Polyorganosiloxanen.

Geeignete Rheologie-Additive sind insbesondere Verdickungsmittel, insbesondere Schichtsilikate wie Bentonite, Derivate von Rizinusöl, hydriertes Rizinusöl, Polyamide, Polyurethane, Harnstoffverbindungen, pyrogene Kieselsäuren, Celluloseether oder hydrophob modifizierte Polyoxyethylene.

Geeignete Trocknungsmittel sind insbesondere Tetraethoxysilan, Vinyltrimethoxysilan, Vinyltriethoxysilan oder Organoalkoxysilane, welche in α-Stellung zur Silangruppe eine funktionelle Gruppe aufweisen, insbesondere N-(Methyl-dimethoxysilylmethyl)-O-methyl-carbamat, (Methacryloxymethyl)silane, Methoxymethylsilane, Orthoameisensäureester, Calciumoxid, Molekularsiebe, hochreaktive Isocyanate wie p-Tosylisocyanat, monomere Diisocyanate oder Mono-Oxazolidine wie Incozol® 2 (von Incorez), insbesondere Vinyltrimethoxysilan oder Vinyltriethoxysilan.

Geeignete Haftvermittler und/oder Vernetzer sind insbesondere Aminosilane wie insbesondere 3-Aminopropyltrimethoxysilan, 3-Aminopropyldimethoxymethylsilan, N-(2-Aminoethyl)-3-aminopropyltrimethoxysilan, N-(2-Aminoethyl)-3-aminopropyldimethoxymethylsilan, N-(2-Aminoethyl)-N'-[3-(trimethoxysilyl)propyl]ethylendiamin oder deren Analoga mit Ethoxy- anstelle von Methoxygruppen, weiterhin N-Phenyl-, N-Cyclohexyl- oder N-Alkylaminosilane, Mercaptosilane, Epoxysilane, (Meth)acrylosilane, Anhydridosilane, Carbamatosilane, Alkylsilane oder Iminosilane, oligomere Formen dieser Silane, Addukte aus primären Aminosilanen mit Epoxysilanen oder (Meth)acrylosilanen oder Anhydridosilanen, aminofunktionelle Alkylsilsesquioxane, insbesondere aminofunktionelles Methylsilsesquioxan oder aminofunktionelles Propylsilsesquioxan. Insbesondere geeignet sind 3-Aminopropyltrimethoxysilan, 3-Aminopropyltriethoxysilan, N-(2-Aminoethyl)-3-aminopropyltrimethoxysilan, N-(2-Aminoethyl)-3-aminopropyltriethoxysilan, 3-Glycidoxypropyltrimethoxysilan, 3-Glycidoxypropyltriethoxysilan oder 3-Ureidopropyltrimethoxysilan, oder oligomere Formen dieser Silane.

In einer bevorzugten Ausführungsform enthält die Zusammensetzung mindestens ein Trocknungsmittel und mindestens einen Haftvermittler und/oder Vernetzer.

In einer bevorzugten Ausführungsform enthält die Zusammensetzung keine Phthalate als Weichmacher. Solche Zusammensetzungen sind toxikologisch vorteilhaft und weisen gegebenenfalls weniger Probleme mit Migrationseffekten auf.

Für den Fall, dass die Zusammensetzung ein Polyisocyanat und/oder ein Isocyanatgruppen aufweisendes Polyurethanpolymer enthält, ist zusätzlich bevorzugt mindestens eine gegenüber Isocyanatgruppen reaktive mehrfunktionelle Verbindung vorhanden, wie insbesondere
- ein oder mehrere Polyole, insbesondere die als geeignet für die Herstellung eines Isocyanatgruppen aufweisendes Polyurethanpolymers genannten Polyole. Bevorzugt sind Polyetherpolyole, Polyesterpolyole, Polycarbonatpolyole, Poly(meth)acrylatpolyole oder Polybutadienpolyole. Besonders bevorzugt sind Polyetherpolyole, insbesondere Polyoxypropylenpolyole und/oder Ethylenoxid-terminierte Polyoxypropylenpolyole. Bevorzugt sind Polyole mit einem mittleren Molekulargewicht im Bereich von 400 bis 10'000 g/mol, insbesondere 500 bis 6'000 g/mol. Bevorzugt sind Polyole mit einer mittleren OH-Funktionalität im Bereich von 1.6 bis 4, insbesondere 1.8 bis 3, besonders bevorzugt 2.2 bis 3. Ebenfalls geeignet sind Polyetherpolyole mit darin dispergierten Polymerpartikeln, insbesondere solche mit Styrol-Acrylnitril-Partikeln (SAN) oder Polyharnstoff- bzw. Polyhydrazodicarbonamid-Partikeln (PHD).
- Kettenverlängerer, insbesondere 1,2-Ethandiol, 1,3-Propandiol, 2-Methyl-1,3-propandiol, 1,4-Butandiol, 1,5-Pentandiol, Neopentylglykol, 1,6-Hexandiol, 3-Methyl-1,5-pentandiol, 1,7-Heptandiol, 1,8-Octandiol, 1,3-Cyclohexandimethanol, 1,4-Cyclohexandimethanol, Diethylenglykol oder Triethylenglykol;
- Aminoalkohole, insbesondere 2-Aminoethanol, 2-(2-Aminoethoxy)ethanol oder 3-Aminomethyl-3,5,5-trimethylcyclohexanol oder Ether-, Ester- oder Urethangruppen aufweisende Derivate davon;
- blockierte Aminogruppen aufweisende Verbindungen, insbesondere Aldimine, Ketimine, Enamine, Oxazolidine, Imidazolidine oder Hexahydropyrimidine.;
- oder Polyamine.

Die Zusammensetzung kann weitere Bestandteile enthalten, insbesondere die folgenden Hilfs- und Zusatzmittel:
- Pigmente, insbesondere Titandioxid oder Eisenoxide;
- Farbstoffe;
- Stabilisatoren gegen Oxidation, Wärme, Licht oder UV-Strahlung;
- natürliche Harze, Fette oder Öle wie Kolophonium, Schellack, Leinöl, Rizinusöl oder Sojaöl;
- nicht-reaktive Polymere, wie insbesondere Homo- oder Copolymere von ungesättigten Monomeren, insbesondere aus der Gruppe umfassend Ethylen, Propylen, Butylen, Isobutylen, Isopren, Vinylacetat oder Alkyl(meth)acrylate, insbesondere Polyethylene (PE), Polypropylene (PP), Polyisobutylene, Ethylenvinylacetat-Copolymere (EVA) oder ataktische Poly-α-Olefine (APAO);
- flammhemmende Substanzen, insbesondere die bereits genannten Füllstoffe Aluminiumhydroxid und Magnesiumhydroxid, oder insbesondere organische Phosphorsäureester wie insbesondere Triethylphosphat, Trikresylphosphat, Triphenylphosphat, Diphenylkresylphosphat, Isodecyldiphenylphosphat, Tris(1,3-dichlor-2-propyl)phosphat, Tris(2-chlorethyl)phosphat, Tris(2-ethylhexyl)phosphat, Tris(chlorisopropyl)phosphat, Tris(chlorpropyl)-phosphat, isopropyliertes Triphenylphosphat, Mono-, Bis- oder Tris(isopropylphenyl)phosphate unterschiedlichen Isopropylierungsgrades, Resorcinol-bis(diphenylphosphat), Bisphenol-A-bis(diphenylphosphat) oder Ammoniumpolyphosphate;
- Fasern, insbesondere Glasfasern, Kohlefasern, Metallfasern, Keramikfasern oder Kunststofffasern wie Polyamidfasern oder Polyethylenfasern;
- oberflächenaktive Substanzen, insbesondere Netzmittel, Verlaufsmittel, Entlüftungsmittel oder Entschäumer;
- Lösemittel;
- Biozide, insbesondere Algizide, Fungizide oder das Pilzwachstum hemmende Substanzen;
sowie weitere üblicherweise in härtbaren Zusammensetzungen eingesetzte Substanzen.

Es kann sinnvoll sein, gewisse Bestandteile vor dem Einmischen in die Zusammensetzung chemisch oder physikalisch zu trocknen.

Die Zusammensetzung wird vorzugsweise unter Ausschluss von Feuchtigkeit hergestellt und aufbewahrt. Typischerweise ist sie in einer geeigneten Verpackung oder Anordnung, wie insbesondere einer Kartusche, einer Flasche, einer Büchse, einem Beutel, einem Eimer, einem Hobbock oder einem Fass, unter Ausschluss von Feuchtigkeit lagerstabil.

Die Zusammensetzung kann in Form einer einkomponentigen oder in Form einer mehrkomponentigen, insbesondere zweikomponentigen, Zusammensetzung vorliegen.

Als "einkomponentig" wird im vorliegenden Dokument eine Zusammensetzung bezeichnet, bei welcher alle Bestandteile der Zusammensetzung vermischt im gleichen Gebinde gelagert werden und welche mit Feuchtigkeit härtbar ist.

Als "zweikomponentig" wird im vorliegenden Dokument eine Zusammensetzung bezeichnet, bei welcher die Bestandteile der Zusammensetzung in zwei verschiedenen Komponenten vorliegen, welche in voneinander getrennten Gebinden gelagert werden. Erst kurz vor oder während der Applikation der Zusammensetzung werden die beiden Komponenten miteinander vermischt, worauf die vermischte Zusammensetzung aushärtet, gegebenenfalls unter Einwirkung von Feuchtigkeit.

Für den Fall, dass die Zusammensetzung ein Polyisocyanat und/oder ein Isocyanatgruppen aufweisendes Polyurethanpolymer enthält, ist sie bevorzugt zweikomponentig. Dabei enthält die eine Komponente das Polyisocyanat und/oder das Isocyanatgruppen aufweisende Polyurethanpolymer und die andere Komponente enthält die Verbindung enthaltend mindestens eine Hexahydrotriazin-Einheit der Formel (I) und zusätzlich mindestens eine gegenüber Isocyanatgruppen reaktive mehrfunktionelle Verbindung.

Für den Fall, dass die Zusammensetzung ein Silangruppen aufweisendes organisches Polymer enthält, ist sie bevorzugt einkomponentig.

Für den Fall, dass die Zusammensetzung ein Polyorganosiloxan mit endständigen Silangruppen enthält, ist sie bevorzugt einkomponentig, auch als RTV-1 bezeichnet, oder zweikomponentig, auch als RTV-2 bezeichnet. Im Fall einer RTV-2 Zusammensetzung ist das Polyorganosiloxan mit endständigen Silangruppen bevorzugt ein Bestandteil der ersten Komponente und ein Silanvernetzer, insbesondere ein Silan der Formel (VIII), ist bevorzugt ein Bestandteil der zweiten Komponente. Die Verbindung enthaltend mindestens eine Hexahydrotriazin-Einheit der Formel (I) kann dabei in der ersten und/oder in der zweiten Komponente enthalten sein.

Eine zweite oder gegebenenfalls weitere Komponenten wird bzw. werden mit der ersten Komponente vor oder bei der Applikation vermischt, insbesondere über einen Statikmischer oder über einen dynamischen Mischer.

Die Zusammensetzung wird insbesondere umgebungswarm, bevorzugt in einem Temperaturbereich zwischen 0°C und 45°C, insbesondere 5°C bis 35°C, appliziert und härtet auch bei diesen Bedingungen aus.

Bei der Applikation beginnt die Vernetzungsreaktion der funktionellen Gruppen, gegebenenfalls unter Einfluss von Feuchtigkeit.

Vorhandene Isocyanatgruppen reagieren mit Hydroxylgruppen, oder primären oder sekundären Aminogruppen, oder unter dem Einfluss von Feuchtigkeit mit blockierten Aminogruppen. Gegebenenfalls vorhandene weitere Isocyanatgruppen reagieren unter dem Einfluss von Feuchtigkeit untereinander. Vorhandene Silangruppen können mit vorhandenen Silanolgruppen zu Siloxangruppen (Si-O-Si-Gruppen) kondensieren. Vorhandene Silangruppen können bei Kontakt mit Feuchtigkeit auch zu Silanolgruppen (Si-OH-Gruppen) hydrolysieren und durch nachfolgende Kondensationsreaktionen Siloxangruppen (Si-O-Si-Gruppen) bilden.

Als Ergebnis dieser Reaktionen härtet die Zusammensetzung schliesslich aus. Die Verbindung enthaltend mindestens eine Hexahydrotriazin-Einheit der Formel (I) beschleunigt diese Aushärtung.

Falls für die Aushärtung Wasser benötigt wird, kann dieses entweder aus der Luft stammen (Luftfeuchtigkeit), oder aber die Zusammensetzung kann mit einer Wasser enthaltenden Komponente in Kontakt gebracht werden, zum Beispiel durch Bestreichen, beispielsweise mit einem Abglättmittel, oder durch Besprühen, oder es kann der Zusammensetzung bei der Applikation Wasser oder eine Wasser enthaltende Komponente zugesetzt werden, zum Beispiel in Form einer wasserhaltigen oder Wasser freisetzenden Flüssigkeit oder Paste. Eine Paste ist insbesondere geeignet für den Fall, dass die Zusammensetzung selber in Form einer Paste vorliegt.

Bei einer Aushärtung mittels Luftfeuchtigkeit härtet die Zusammensetzung von aussen nach innen durch, wobei zunächst eine Haut an der Oberfläche der Zusammensetzung gebildet wird. Die sogenannte Hautbildungszeit stellt ein Mass für die Aushärtungsgeschwindigkeit der Zusammensetzung dar. Die Geschwindigkeit der Aushärtung wird dabei im Allgemeinen von verschiedenen Faktoren, wie beispielsweise der Verfügbarkeit von Wasser, der Temperatur usw., bestimmt.

Die Zusammensetzung eignet sich für eine Vielzahl von Anwendungen, insbesondere als Anstrich, Lack oder Primer, als Harz zur Herstellung von Faserverbundwerkstoff (Composite), als Hartschaum, Weichschaum, Formteil, Elastomer, Faser, Folie oder Membran, als Vergussmasse, Dichtstoff, Klebstoff, Belag, Beschichtung oder Anstrich für Bau- und Industrieanwendungen, beispielsweise als Nahtabdichtung, Hohlraumversiegelung, Elektroisolationsmasse, Spachtelmasse, Fugendichtstoff, Schweiss- oder Bördelnahtdichtstoff, Montageklebstoff, Karrosserieklebstoff, Scheibenklebstoff, Sandwichelementklebstoff, Kaschierklebstoff, Laminatklebstoff, Verpackungsklebstoff, Holzklebstoff, Parkettklebstoff, Verankerungsklebstoff, Bodenbelag, Bodenbeschichtung, Balkonbeschichtung, Dachbeschichtung, Betonschutzbeschichtung, Parkhausbeschichtung, Versiegelung, Rohrbeschichtung, Korrosionsschutzbeschichtung, Textilbeschichtung, Dämpfungselement, Dichtungselement oder Spachtelmasse.

Besonders geeignet ist die Zusammensetzung als Klebstoff und/oder Dichtstoff, insbesondere für die Fugenabdichtung und für elastische Klebeverbindungen in Bau- und Industrieanwendungen, oder als elastische Beschichtung mit rissüberbrückenden Eigenschaften, insbesondere zum Schützen und/oder Abdichten von beispielsweise Dächern, Böden, Balkonen, Parkdecks oder Betonröhren.

Bevorzugt stellt die Zusammensetzung somit einen Klebstoff oder einen Dichtstoff oder eine Beschichtung dar.

Eine solche Zusammensetzung enthält typischerweise Füllstoffe, Weichmacher, Trocknungsmittel, Haftvermittler und/oder Vernetzer und gegebenenfalls weitere Hilfs- und Zusatzstoffe.

Für eine Anwendung als Klebstoff oder Dichtstoff weist die Zusammensetzung bevorzugt eine pastöse Konsistenz mit strukturviskosen Eigenschaften auf. Ein solcher pastöser Dichtstoff oder Klebstoff wird insbesondere aus handelsüblichen Kartuschen, welche manuell, mittels Druckluft oder Batterie betrieben werden, oder aus einem Fass oder Hobbock mittels einer Förderpumpe oder eines Extruders, gegebenenfalls mittels eines Applikationsroboters, auf ein Substrat aufgetragen.

Für eine Anwendung als Beschichtung weist die Zusammensetzung bevorzugt eine bei Raumtemperatur flüssige Konsistenz mit selbstverlaufenden Eigenschaften auf. Gegebenenfalls ist sie leicht thixotrop, so dass die Beschichtung an geneigten bis senkrechten Flächen applizierbar ist, ohne sofort wegzufliessen. Sie wird insbesondere appliziert mittels Rolle oder Pinsel oder durch Ausgiessen und Verteilen mittels beispielsweise einem Roller, einem Schaber oder einer Zahntraufel.

Bei der Applikation wird die Zusammensetzung bevorzugt auf mindestens ein Substrat appliziert.

Geeignete Substrate sind insbesondere
- Glas, Glaskeramik, Beton, Mörtel, Backstein, Ziegel, Gips oder Natursteine wie Kalkstein, Granit oder Marmor;
- Metalle und Legierungen, wie Aluminium, Eisen, Stahl oder Buntmetalle, sowie oberflächenveredelte Metalle oder Legierungen, wie verzinkte oder verchromte Metalle;
- Leder, Textilien, Papier, Holz, mit Harzen, beispielsweise Phenol-, Melamin-oder Epoxidharzen, gebundene Holzwerkstoffe, Harz-Textil-Verbundwerkstoffe und weitere sogenannte Polymer-Composites;
- Kunststoffe, wie Polyvinylchlorid (Hart- und Weich-PVC), Acrylonitril-Butadien-Styrol-Copolymere (ABS), Polycarbonat (PC), Polyamid (PA), Polyester, Poly(methylmethacrylat) (PMMA), Epoxidharze, Polyurethane (PUR), Polyoxymethylen (POM), Polyolefine (PO), Polyethylen (PE) oder Polypropylen (PP), Ethylen/Propylen-Copolymere (EPM) oder Ethylen/Propylen/Dien-Terpolymere (EPDM), oder faserverstärkte Kunststoffe wie Kohlefaserverstärkte Kunststoffe (CFK), Glasfaser-verstärkte Kunststoffe (GFK) oder Sheet Moulding Compounds (SMC), wobei die Kunststoffe bevorzugt mittels Plasma, Corona oder Flammen oberflächenbehandelt sein können;
- beschichtete Substrate, wie pulverbeschichtete Metalle;
- Farben oder Lacke, insbesondere Automobildecklacke, Metalllacke, Möbellacke oder Holzlacke.

Die Substrate können bei Bedarf vor dem Applizieren der Zusammensetzung vorbehandelt werden, insbesondere durch physikalische und/oder chemische Reinigungsverfahren oder durch das Aufbringen eines Haftvermittlers, einer Haftvermittlerlösung oder eines Primers.

Besonders geeignet ist die Zusammensetzung für den Kontakt mit Substraten, die besonders empfindlich sind auf Störungen durch migrierende Substanzen, insbesondere durch das Ausbilden von Verfärbungen oder Flecken. Dies sind insbesondere feinporige Substrate wie Marmor, Kalkstein oder andere Natursteine, Gips, Zementmörtel oder Beton, aber auch Kunststoffe. Insbesondere auf PVC werden bei der Anwesenheit von Katalysatoren wie beispielsweise DBU oder TMG starke Verfärbungen beobachtet, die sich durch Reinigung nicht entfernen lassen. Solche Effekte werden mit der Verbindung enthaltend mindestens eine Hexahydrotriazin-Einheit der Formel (I) nicht beobachtet.

Verklebt oder abgedichtet werden können zwei gleichartige oder zwei verschiedene Substrate, insbesondere die vorgängig genannten Substrate.

Nach der Aushärtung der Zusammensetzung wird eine ausgehärtete Zusammensetzung erhalten.

Aus der Anwendung der Zusammensetzung entsteht ein Artikel, welcher mit der Zusammensetzung insbesondere verklebt, abgedichtet oder beschichtet wurde. Beim Artikel handelt es sich insbesondere um ein Bauwerk, insbesondere ein Bauwerk des Hoch- oder Tiefbaus, um ein industriell gefertigtes Gut oder um ein Konsumgut, insbesondere ein Fenster, eine Haushaltmaschine oder ein Transportmittel wie insbesondere ein Automobil, ein Bus, ein Lastkraftwagen, ein Schienenfahrzeug, ein Schiff, ein Flugzeug oder ein Helikopter; oder der Artikel kann ein Anbauteil davon sein.

### Beispiele

Im Folgenden sind Ausführungsbeispiele aufgeführt, welche die beschriebene Erfindung näher erläutern sollen. Selbstverständlich ist die Erfindung nicht auf diese beschriebenen Ausführungsbeispiele beschränkt.

Als "Normklima" wird eine Temperatur von 23±1°C und eine relative Luftfeuchtigkeit von 50±5% bezeichnet.
**¹H- und ¹³C-NMR-Spektren** wurden bei Raumtemperatur auf einem Spektrometer des Typs Bruker Ascend bei 400.14 MHz (¹H) bzw. 100.63 MHz (¹³C) gemessen; die chemischen Verschiebungen δ sind angegeben in ppm relativ zu Tetramethylsilan (TMS). Kopplungskonstanten J sind angegeben in Hz. Echte und Pseudo-Kopplungsmuster wurden nicht unterschieden.

**Infrarotspektren** (FT-IR) wurden auf einem mit horizontaler ATR-Messeinheit mit Diamant-Kristall ausgestatteten FT-IR Gerät Nicolet iS5 von Thermo Scientific gemessen. Flüssige Proben wurden unverdünnt als Filme aufgetragen, feste Proben wurden in CH₂Cl₂ gelöst. Die Absorptionsbanden sind in Wellenzahlen (cm⁻¹) angegeben (Messfenster: 4000-650 cm⁻¹).

**Gaschromatogramme** (GC) wurden gemessen im Temperaturbereich von 60 bis 320°C mit einer Aufheizrate von 15°C/min und 10 min Verweilzeit bei 320°C. Die Injektortemperatur betrug 250°C. Es wurde eine Zebron ZB-5 Säule verwendet (L = 30 m, ID = 0.25 mm, dj = 0.5 µm) bei einem Gasfluss von 1.5 ml/Min. Die Detektion erfolgte mittels Flammenionisation (FID), wobei die Signale via Flächenprozent-Methode ausgewertet wurden.

Die **Hautbildungszeit** (HBZ) wurde dadurch bestimmt, dass einige Gramm der Zusammensetzung in einer Schichtdicke von ca. 2 mm auf Pappkarton aufgetragen wurden und im Normklima die Zeitdauer gemessen wurde, bis beim leichten Antippen der Oberfläche der Zusammensetzung mittels einer Pipette aus LDPE erstmals keine Rückstände auf der Pipette mehr zurückblieben. Die Beschaffenheit der **Oberfläche** wurde haptisch geprüft.

Die mechanischen Eigenschaften **Zugfestigkeit, Bruchdehnung** und **E-Modul** (bei 0-25% bzw. bei 0-5% und 0-50% Dehnung) wurden gemäss DIN EN 53504 bei einer Zuggeschwindigkeit von 200 mm/min. gemessen.

Die **Viskosität** wurde auf einem thermostatisierten Kegel-Platten-Viskosimeter Rheotec RC30 (Kegeldurchmesser 50 mm, Kegelwinkel 1°, Kegelspitze-Platten-Abstand 0.05 mm, Scherrate 10 U/min) gemessen.

### Verwendete kommerzielle Substanzen:

Jeffamine® D-230 (Huntsman), Polyoxypropylendiamin mit einem mittleren Molekulargewicht von ca. 240 g/mol, Aminzahl 465 mg KOH/g
Jeffamine® D-400 (Huntsman), Polyoxypropylendiamin mit einem mittleren Molekulargewicht von ca. 400 g/mol, Aminzahl 247 mg KOH/g
Wacker® Fluid NH 15 D (Wacker), α,ω-Bis(3-aminopropyl)-poly(dimethylsiloxan) mit einem mittleren Molekulargewicht von ca. 1050 g/mol, Aminzahl 106 mg KOH/g
Shin-Etsu Silicone® KF-8010 (Shin Etsu), α,ω-Bis(3-aminopropyl)-poly(dimethyl-siloxan) mit einem mittleren Molekulargewicht von ca. 860 g/mol
Wacker® Aminöl 446011-20 VP (Wacker), α-(3-Aminopropyl)-ω-isobutoxy-poly-(dimethylsiloxan) mit einem mittleren Molekulargewicht von ca. 1650 g/mol, Aminzahl 34 mg KOH/g
1,3-Diaminopropan (Sigma-Aldrich)
Bis(3-aminopropyl)amin (Baxxodur® EC 110 von BASF)
n-Hexylamin (Sigma-Aldrich)
Isopropylamin (Sigma-Aldrich)
3-Dimethylamino-1-propylamin (Sigma-Aldrich)
N,N'-Dicyclohexylcarbodiimid (Sigma-Aldrich)
n-Propylamin (Sigma-Aldrich)
3-Aminopropyltriethoxysilan (AMEO) (Sigma-Aldrich)
Trimethylorthoacetat (Sigma-Aldrich)
Lanthan(III)-trifluormethansulfonat (Sigma-Aldrich)
Poly bd® R-45HTLO (Cray Valley), Polybutadienpolyol mit OH-Funktionalität ca. 2.5, mittlerem Molekulargewicht ca. 2800 g/mol und OH-Zahl 47.1 mg KOH/g
Desmodur® CD (Covestro), modifiziertes Diphenylmethandiisocyanat enthaltend MDI-Carbodiimid-Addukte, bei Raumemperatur flüssig, 28 Gew-% NCO

### Herstellung von Amino-Amidinen oder -Guanidinen der Formel H₂N-A-Z: Amidin A1: 1-(3-Aminopropyl)-2-methyl-1,4,5,6-tetrahydropyrimidin

In einem Rundkolben wurden 62.97 g Trimethylorthoacetat, 62.12 g Bis(3-aminopropyl)amin und 2.80 g Lanthan(III)-trifluormethansulfonat unter Stickstoffatmosphäre vermischt und die Mischung unter Rühren am Rückfluss während 3 Tagen auf 120°C erwärmt. Darauf wurde das Reaktionsgemisch im Vakuum von den flüchtigen Bestandteilen befreit und der Rückstand im Vakuum destilliert. Man erhielt 26.65 g eines farblosen Öls mit einer Siedetemperatur von 85-88°C bei 0.1 mbar, welches gemäss GC-Spektrum einen Gehalt von 87% Amidin A1 enthielt.
¹H-NMR (CDCl₃) (nur Signale von Amidin A1): δ 1.05 (*s*, 2 H, NH₂), 1.57-1.70 (*m*, 2 H, C*H*₂CH₂NH₂), 1.74-1.86 (*m,* 2 H, C=NCH₂C*H*₂), 1.97 (*s*, 3 H, CH₃), 2.60-2.80 (*m*, 2 H, CH₂C*H*₂NH₂), 3.10-3.24 und 3.25-3.34 (2 x *m*, 4H und 2 H, CH₂N).
¹³C-NMR (CDCl₃): δ 22.02 (CH₂), 22.56 (CH₃), 32.10 (CH₂), 39.44 (CH₂), 44.50 (CH₂), 45.93 (CH₂), 49.09 (CH₂), 154.89 (C).
FT-IR: 3260, 2924, 2748, 1611, 1482, 1433, 1375, 1353, 1317, 1289, 1211, 1149, 1126, 1099, 1085, 1031,1014, 942, 879, 821, 752, 735, 692.

### Guanidin G1: Reaktionsprodukt enthaltend 1-(3-Aminopropyl)-2,3-dicyclohexylguanidin

In einem Rundkolben wurden 2.50 g 1,3-Diaminopropan und 6.89 g N,N'-Dicyclohexylcarbodiimid unter Stickstoffatmosphäre vermischt und die Mischung unter Rühren auf 120°C erwärmt. In regelmässigen Abständen wurde das Reaktionsgemisch mittels FT-IR-Spektroskopie untersucht. Nach 1 Stunde war die Carbodiimid-Bande bei ca. 2120 cm⁻¹ vollständig verschwunden. Darauf wurde das Reaktionsgemisch im Vakuum von den flüchtigen Bestandteilen befreit. Man erhielt 9.36 g eines hellgelben Öls.
¹H-NMR (CDCl₃): δ 1.05-1.2 und 1.25-1.40 (2 x *m*, 10 H), 1.54-1.78 (*m*, 10 H), 1.88-2.0 (*m*, 4 H), 2.73 (*m*, 2 H), 3.12 (*m*, 2 H), 3.22 (br s, 2 H).
¹³C-NMR (CDCl₃): δ 24.75 und 24.89 und 25.0 (CH₂), 25.47 (CH₂), 32.20 (CH₂), 33.88 (CH₂), 39.9 (*CH₂*NH), 41.94 (*CH₂*NH₂), 50.95 (CH), 151.5 (C).
FT-IR: 3371 (N-H), 2921, 2849, 1627 (C=N), 1502, 1447, 1324, 1238, 1147, 1111, 888, 713.

### Guanidin G2: Reaktionsprodukt enthaltend 1-(ω-3-Aminopropyl-α-1,3-propylen-poly(dimethylsiloxan))-2,3-dicyclohexylguanidin mit einem mittleren Molekulargewicht von ca. 1'260 g/mol

In einem Rundkolben wurden 30.23 g Wacker® Fluid NH 15 D und 6.00 g N,N'-Dicyclohexylcarbodiimid unter Stickstoffatmosphäre vermischt und die Mischung unter Rühren auf 115°C erwärmt. In regelmässigen Abständen wurde das Reaktionsgemisch mittels FT-IR-Spektroskopie untersucht. Nach 2 Stunden war die Carbodiimid-Bande bei ca. 2120 cm⁻¹ vollständig verschwunden. Man erhielt ein gelbliches Öl.
¹H-NMR (CDCl₃): δ 0.0 (*s*, 88 H, CH₃Si), 0.41-0.53 (*m*, 4 H, CH₂-Si), 1.05-1.18 und 1.20-1.30 (2 x *m*, 10 H), 1.33-1.5 (*m*, 6 H, CH₂), 1.6-1.78 (*m*, 4 H), 1.88-2.0 (*m*, 4 H), 2.57 (*t*, 2 H, J= 7 Hz, *CH₂*NH₂), 2.92 (*t*, 2 H, J= 7.2 Hz, *CH₂*-NH), 3.13 (br s, 2 H, CH-N).
¹³C-NMR (CDCl₃): δ 0.13 und 0.16 und 0.37 (CH₃Si), 14.89 und 14.23 (CH₂), 23.85 und 23.91 (CH₂), 24.87 (CH₂), 33.28 (CH₂), 44.16 (CH₂), 47.2 (CH₂), 50.45 (CH), 150.0 (C).
FT-IR: 2960, 2927, 2853, 1645, 1496, 1449, 1411, 1361, 1257, 1013, 789, 700.

### Guanidin G3: Reaktionsprodukt enthaltend 1-(ω-3-Aminopropyl-α-1,3-propylen-poly(dimethylsiloxan))-2,3-dicyclohexylguanidin mit einem mittleren Molekulargewicht von ca. 1'070 g/mol

In einem Rundkolben wurden 27.29 g Shin-Etsu Silicone® KF-8010 und 6.66 g N,N'-Dicyclohexylcarbodiimid unter Stickstoffatmosphäre vermischt und die Mischung unter Rühren auf 115°C erwärmt. In regelmässigen Abständen wurde das Reaktionsgemisch mittels FT-IR-Spektroskopie untersucht. Nach 2 Stunden war die Carbodiimid-Bande bei ca. 2120 cm⁻¹ vollständig verschwunden. Man erhielt ein gelbliches Öl.
¹H-NMR (CDCl₃): δ 0.0 (*s*, 88 H, CH₃Si), 0.41-0.53 (*m*, 4 H, CH₂-Si), 1.0-1.16 und 1.16-1.30 (2 x *m*, 10 H), 1.3-1.58 (*m*, 6 H, CH₂), 1.58-1.78 (*m*, 4 H), 1.88-2.0 (*m*, 4 H), 2.51-2.60 (*m*, 2 H, CH₂), 2.86-2.95 (*m*, 2 H, CH₂), 3.13 (br s, 2 H, CH).
¹³C-NMR (CDCl₃): δ 0.13 und 0.16 und 0.37 (CH₃Si), 14.24 und 14.91 (CH₂-Si), 24.26 (CH₂), 24.90 (CH₂), 26.57 (CH₂), 33.44 (CH₂), 44.18 (CH₂), 47.25 (CH₂), 50.38 (CH), 150.0 (C).
FT-IR: 2959, 2927, 2854, 1644 (C=N), 1450, 1412, 1257, 1015, 837, 789, 702.

### Guanidin G4: Reaktionsprodukt enthaltend 1-(ω-Aminopropyl-polyoxypropylen)-2,3-dicyclohexylguanidin mit einem mittleren Molekulargewicht von ca. 450 g/mol

In einem Rundkolben wurden 24.97 g Jeffamine® D-230 und 20.94 g N,N'-Dicyclohexylcarbodiimid unter Stickstoffatmosphäre vermischt und die Mischung unter Rühren auf 115°C erwärmt. In regelmässigen Abständen wurde das Reaktionsgemisch mittels FT-IR-Spektroskopie untersucht. Nach 4 Stunden war die Carbodiimid-Bande bei ca. 2120 cm⁻¹ vollständig verschwunden. Man erhielt ein gelbliches Öl.
¹³C-NMR (CDCl₃): 17.1-17.4 (mehrere CH₃), 19.66 (CH₃), 25.02 und 25.14 (CH₂), 25.84 (CH₂), 26.57 (CH₂), 34.09 und 34.40 (mehrere CH₂), 46.23 und 46.76 (CH-cy), 49.9 (CH-N), 51.6 (*CH*-NH₂), 74.26-75.35 (CH₂), 76.06-76.26 (CH), 78.08 (CH₂).
FT-IR: 3368 (N-H), 2922, 2850, 1637 (C=N), 1498, 1448, 1372, 1337, 1283, 1237, 1103, 1026, 977, 888, 860, 826, 718.

### Guanidin G5: Reaktionsprodukt enthaltend 1-(ω-Aminopropyl-polyoxypropylen)-2,3-dicyclohexylguanidin mit einem mittleren Molekulargewicht von ca. 650 g/mol

In einem Rundkolben wurden 24.31 g Jeffamine® D-400 und 11.46 g N,N'-Dicyclohexylcarbodiimid unter Stickstoffatmosphäre vermischt und die Mischung unter Rühren während 24 Stunden auf 120°C erwärmt. Die Carbodiimid-Bande bei ca. 2120 cm⁻¹ war dann vollständig verschwunden. Man erhielt ein gelbliches Öl.
¹³C-NMR (CDCl₃): 17.1-17.4 (CH₃), 19.65 (CH₃), 25.02 und 25.14 (CH₂), 25.84 (CH₂), 34.09 und 34.40 (CH₂), 26.57 (CH₂), 46.23 und 46.76 (CH-cy), 50.0 (CH-N), 51.6 (*CH*-NH₂), 74.26-75.35 (CH₂), 76.06-76.26 (CH), 78.08 (CH₂). FT-IR: 3366 (N-H), 2968, 2925, 2852, 1639 (C=N), 1496, 1448, 1372, 1341, 1256, 1238, 1100, 1018, 924, 888, 860, 826, 714, 666.

### Herstellung von Hexahydrotriazin-Zwischenprodukten: Zwischenprodukt HZ-1 @RCa917

In einem Rundkolben wurden 29.63 g n-Propylamin und 50 mL n-Heptan unter Stickstoffatmosphäre vermischt, 42.25 g wässrige Formaldehyd-Lösung (37 Gewichts-%) zugegeben und die Mischung anschliessend 1 Stunde bei Raumtemperatur gerührt. Die Reaktionsmischung wurde dann mit 10 mL Wasser gewaschen, mit Magnesiumsulfat getrocknet und filtriert. Darauf wurde das Reaktionsgemisch im Vakuum von den flüchtigen Bestandteilen befreit. Man erhielt 35.30 g eines geruchlosen, farblosen, sehr dünnflüssigen Öls.
¹H-NMR (CDCl₃): 0.83 (*t,* 9 H, J=7.4 Hz, CH₃), 1.41 (*sext,* 6 H, J=7.5 Hz, *CH*₂CH₃), 2.30 (*t*, 6 H, J=7.5 Hz, NCH₂CH₂), 3.20 (br s, 6 H, NCH₂N).
FT-IR: 2957, 2931, 2872, 2783, 1461, 1398, 1375, 1344, 1304, 1272, 1201, 1142, 1114, 1095, 1008, 976, 957, 927, 878, 852, 839, 779, 750.

### Zwischenprodukt HZ-2 @RCa925

In einem Rundkolben wurden 4.93 g Zwischenprodukt HZ-1 und 5.11 g 3-Aminopropyltriethoxysilan unter Stickstoffatmosphäre vermischt, 4 Stunden bei 150 °C am Rückfluss gekocht und anschliessend im Vakuum die flüchtigen Bestandteile entfernt. Man erhielt 8.39 g eines geruchlosen, farblosen, dünnflüssigen Öls.
¹H-NMR (CDCl₃): 0.56 (*m,* 2 H, CH₂Si), 0.83 (*t*, 6 H, J=7.4 Hz, CH₂CH₂C*H₃*), 1.15 (*t*, 9 H, J=7.2 Hz, OCH₂C*H₃*), 1.42 (*sext,* 4 H, J=7.5 Hz, CH₂C*H*₂CH₃), 1.50 (*m*, 2 H, J=7.5 Hz, C*H*₂CH₂Si), 2.34 (*m*, 4 H, NC*H*₂CH₂), 3.20 (br s, 6 H, NCH₂N), 3.74 (*quart,* 6 H, J=7.0 Hz, OCH₂).
FT-IR: 2969, 2929, 2875, 2798, 1462, 1389, 1377, 1364, 1345, 1296, 1275, 1202, 1166, 1101, 1075, 1008, 976, 953, 879, 768.

### Herstellung von erfindungsgemässen Hexahydrotriazin-Verbindungen: Katalysator HHT-1

In einem Rundkolben wurden 0.87 g Paraformaldehyd und 15 mL n-Heptan unter Stickstoffatmosphäre vermischt und mittels Eisbad gekühlt. Anschliessend wurde eine Mischung aus 11.52 g Guanidin G2 und 1.87 g n-Hexylamin portionenweise eingerührt und die Mischung dann unter Rühren während 3 Stunden auf 40°C erwärmt. Die Reaktionsmischung wurde dann auf Raumtemperatur abgekühlt, mit 10 mL Wasser gewaschen, mit Magnesiumsulfat getrocknet und filtriert. Darauf wurde das Reaktionsgemisch im Vakuum von den flüchtigen Bestandteilen befreit. Man erhielt 11.68 g eines geruchlosen, gelben, sehr dünnflüssigen Öls.
¹³C-NMR (CDCl₃): 0.13 (CH₃), 13.02 (CH₃), 14.86 (CH₂), 21.56 (CH₂), 23.31-24.75 (CH₂), 24.74 (CH₂), 25.82 (CH₂), 26.19 (CH₂), 26.59 (CH₂), 29.42 (CH₂), 30.76 (CH₂), 33.23 (CH₂), 51.88 (CH₂), 62.37 (CH₂), 73.71 (NCH₂N), 151.87 (C).
FT-IR: 2959, 2927, 2854, 1645, 1450, 1410, 1363, 1257, 1066, 1015, 862, 791, 699.

### Katalysator HHT-2

In einem Rundkolben wurden 2.03 g Guanidin G1, 4.57 g Guanidin G2 und 10 mL n-Heptan unter Stickstoffatmosphäre vermischt und mittels Eisbad gekühlt. Dann wurden 1.03 g wässrige Formaldehyd-Lösung (37 Gewichts-%) portionenweise eingerührt und die Mischung anschliessend 4 Stunden bei Raumtemperatur gerührt. Die Reaktionsmischung wurde dann mit 10 mL Wasser gewaschen, mit Magnesiumsulfat getrocknet und filtriert. Darauf wurde das Reaktionsgemisch im Vakuum von den flüchtigen Bestandteilen befreit. Man erhielt 3.64 g eines geruchlosen, farblosen Öls mit einer Viskosität bei 25°C von 2.5 Pa s.
¹³C-NMR (CDCl₃): 0.13 (CH₃), 14.09 (CH₃) 20.55 (CH₂), 21.43 (CH₂), 21.92 (CH₂), 23.9-24.8 (CH₂), 32.94 (CH₂), 33.31 (CH₂), 40.78 (CH₂), 46.68 (CH₂), 47.16 (CH₂), 47.78 (CH₂), 48.54 (CH₂), 49.39 (CH₂), 50.34 (CH), 73.12 (NCH₂N), 78.45 (CH₂), 151.7 (C).
FT-IR: 2958, 2925, 2852, 1634, 1504, 1449, 1410, 1361, 1346, 1257, 1015, 791, 701.

### Katalysator HHT-3

In einem Rundkolben wurden 3.27 g Guanidin G1, 2.62 g Guanidin G4 und 10 mL n-Heptan unter Stickstoffatmosphäre vermischt und mittels Eisbad gekühlt. Dann wurden 1.53 g wässrige Formaldehyd-Lösung (37 Gewichts-%) portionenweise eingerührt und die Mischung anschliessend 2 Stunden bei Raumtemperatur gerührt. Die Reaktionsmischung wurde dann mit 10 mL Wasser gewaschen, mit Magnesiumsulfat getrocknet und filtriert. Darauf wurde das Reaktionsgemisch im Vakuum von den flüchtigen Bestandteilen befreit. Man erhielt 4.95 g eines geruchlosen, gelben, dickflüssigen Öls mit einer Viskosität bei 25°C von 159.8 Pa s.
¹³C-NMR (CDCl₃): 13.9 (CH₃), 17.23 (CH₃), 20.55 (CH₂), 22.58 (CH₂), 21.92 (CH₂), 24.6-26.0 (CH₂), 31.84 (CH₂), 33.67-34.0 (CH₂), 35.39 (CH₂), 41.59 (CH₂), 47.64 (CH₂), 49.56 (CH₂), 51.38 (CH), 64.26 (CH₂), 74.89-75.15 (NCH₂N), 79.42 (CH₂), 151.7 (C).
FT-IR: 3355, 2922, 2850, 1633, 1498, 1447, 1363, 1336, 1256, 1146, 1107, 1052, 978, 888, 872, 800, 718.

### Katalysator HHT-4

In einem Rundkolben wurden 3.04 g Guanidin G1, 3.41 g Guanidin G5 und 10 mL Toluol unter Stickstoffatmosphäre vermischt. Dann wurden bei Raumtemperatur 0.50 g Paraformaldehyd portionenweise eingerührt und die Mischung dann während 1 Stunde bei 70 °C gerührt. Anschliessend wurde die Reaktionsmischung auf Raumtemperatur abgekühlt, mit 10 mL Toluol versetzt, mit Magnesiumsulfat getrocknet und filtriert. Darauf wurde das Reaktionsgemisch im Vakuum von den flüchtigen Bestandteilen befreit. Man erhielt 6.54 g eines geruchlosen, gelben Öls mit einer Viskosität bei 25°C von 27.8 Pa s.
¹³C-NMR (CDCl₃): 17.08-17.59 (CH₃), 19.61-19.65 (CH₃), 22.30 (CH₃), 24.6-26.0 (CH₂), 33.6-34.0 (CH₂), 35.39 (CH₂), 42.45 (CH₂), 47.64 (CH₂), 49.56 (CH₂), 51.38 (CH), 64.26 (CH₂), 72.91 und 73.37 (NCH₂N), 74.91-75.50 (CH), 76.1-76.37 (CH₂), 155.83 (C).
FT-IR: 3295, 2923, 2850, 1634, 1497, 1448, 1371, 1341, 1255, 1237, 1100, 1020, 977, 926, 906, 888, 845, 807, 730, 695.

### Katalysator HHT-5

In einem Rundkolben wurden 1.13 g Amidin A1 und 6.02 g Wacker® Aminöl 446011-20 VP unter Stickstoffatmosphäre vermischt und mittels Eisbad gekühlt. Dann wurden 0.36 g Paraformaldehyd portionenweise eingerührt und die Mischung anschliessend während 1 Stunde bei Raumtemperatur gerührt. Dann wurden 15 mL Tetrahydrofuran zugegeben, mit 10 mL Wasser gewaschen, mit Magnesiumsulfat getrocknet und filtriert. Darauf wurde das Reaktionsgemisch im Vakuum von den flüchtigen Bestandteilen befreit. Man erhielt 6.88 g eines geruchlosen, weissen Öls mit einer Viskosität bei 25°C von 2.8 Pa s.
¹³C-NMR (CDCl₃): 0.13 (CH₃), 14.69 (CH₂), 17.24 (CH₃), 21.0 (CH₂), 21.38 (CH₃), 23.29 (CH₂), 24.63 (CH₂), 28.07 (CH₂), 29.21 (CH), 29.79 (CH), 43.5 (CH₂), 44.93 (CH₂), 52.0 (CH₂), 59.26 (CH₂), 65.44 (CH₂), 66.95 (CH₂), 68.49 (NCH₂N), 152.8 (C).
FT-IR: 2962, 2906, 1445, 1412, 1258, 1011, 863, 795, 700.

### Katalysator HHT-6

In einem Rundkolben wurden 4.22 g Amidin A1, 1.53 g Jeffamine® D-230 und 12 mL Methanol unter Stickstoffatmosphäre vermischt und mittels Eisbad gekühlt. Dann wurden 1.11 g Paraformaldehyd portionenweise eingerührt und die Mischung anschliessend während 1 Stunde bei Raumtemperatur gerührt. Die Reaktionsmischung wurde dann mit 10 mL Ethylacetat versetzt, mit Magnesiumsulfat getrocknet und filtriert. Darauf wurde das Reaktionsgemisch im Vakuum von den flüchtigen Bestandteilen befreit. Man erhielt 6.74 g eines geruchlosen, farblosen Öls mit einer Viskosität bei 25°C von 34.7 Pa s.
¹³C-NMR (CDCl₃): 17.23 (CH₃), 18.37 (CH₃), 21.61-21.7 (CH₂), 21.84-22.02 (CH₃), 28.85 (CH₂), 29.04 (CH₂), 43.63-43.91 (CH₂), 45.76 (CH₂), 49.05-49.92 (CH), 50.77 (CH₂), 52.95 (CH₂), 53.38 (CH), 54.38 (CH), 55.41 (CH), 60.13 (CH₂), 74.94 (NCH₂N), 153.96 (C).
FT-IR: 3233, 2926, 2850, 1612, 1425, 1374, 1318, 1292, 1208, 1100, 1085, 1015, 943, 737, 654.

### Katalysator HHT-7

In einem Rundkolben wurden 5.28 g Amidin A1 unter Stickstoffatmosphäre vorgelegt und mittels Eisbad gekühlt. Dann wurden 1.02 g Paraformaldehyd portionenweise eingerührt und die Mischung anschliessend unter Rühren während 1 Stunde auf 40°C erwärmt. Die Reaktionsmischung wurde dann auf Raumtemperatur abgekühlt, mit 15 mL Ethylacetat versetzt, mit Magnesiumsulfat getrocknet und filtriert. Darauf wurde das Reaktionsgemisch im Vakuum von den flüchtigen Bestandteilen befreit. Man erhielt 4.69 g eines geruchlosen, gelben Öls mit einer Viskosität bei 25°C von 52.1 Pa s.
¹³C-NMR (CDCl₃): 0.13 (CH₃), 12.96 (CH₃), 14.69 (CH₂), 20.41 (CH₂), 21.56 (CH₂), 23.31 (CH₂), 25.82 (CH₂), 26.19 (CH₂), 26.59 (CH₂), 29.41 (CH₂), 30.57 (CH₂), 30.76 (CH₂), 44.90 (CH₂), 48.19 (CH₂), 48.64 (CH), 51.96 (CH₂), 55.20 (CH₂), 62.38 (CH₂), 65.44 (CH₂), 75.0 (NCH₂N), 151.81 (C).
FT-IR: 3260, 2927, 2849, 1643, 1609, 1466, 1426, 1376, 1317, 1292, 1210, 1086, 1031, 945, 890, 813, 737, 693.

### Katalysator HHT-8

In einem Rundkolben wurden 3.69 g Amidin A1, 1.06 g 3-Dimethylamino-1-propylamin und 15 mL Methylenchlorid unter Stickstoffatmosphäre vermischt. Dann wurden bei Raumtemperatur 0.97 g Paraformaldehyd portionenweise eingerührt und die Mischung dann während 1 Stunde bei 40°C gerührt. Anschliessend wurde die Reaktionsmischung auf Raumtemperatur abgekühlt, mit 10 mL Methylenchlorid versetzt, mit Magnesiumsulfat getrocknet und filtriert. Darauf wurde das Reaktionsgemisch im Vakuum von den flüchtigen Bestandteilen befreit. Man erhielt 4.13 g eines geruchlosen, gelben Öls mit einer Viskosität bei 25°C von 41.7 Pa s.
¹³C-NMR (CDCl₃): 14.1 (CH₂), 21.91 und 22.4 (CH₃), 25.9-26.43 (CH₂), 28.56 (CH₂), 29.13 (CH₂), 35.42 (CH₂), 44.15 (CH₂), 45.6 (CH₃), 49.08-49.48 (CH₂), 50.81 (CH₂), 57.77-57.85 (CH₂), 60.23 (CH₂), 61.28 (CH₂), 74.42-74.67 (NCH₂N), 153.29 und 153.87 (C).
FT-IR: 3255, 2926, 2850, 2812, 2762, 1612, 1551, 1457, 1424, 1374, 1317, 1292, 1206, 1098, 1084, 1061, 1040, 1012, 967, 941, 891, 835, 796, 746, 653.

### Katalysator HHT-9

In einem Rundkolben wurden 1.00 g Amidin A1 und 3.17 g Guanidin G3 unter Stickstoffatmosphäre vermischt und mittels Eisbad gekühlt. Dann wurden 0.29 g Paraformaldehyd portionenweise eingerührt und die Mischung anschliessend unter Rühren während 1 Stunde auf 40°C erwärmt. Die Reaktionsmischung wurde dann auf Raumtemperatur abgekühlt, mit 15 mL Ethylacetat versetzt, mit Magnesiumsulfat getrocknet und filtriert. Darauf wurde das Reaktionsgemisch im Vakuum von den flüchtigen Bestandteilen befreit. Man erhielt 3.96 g eines geruchlosen, hellgelben Öls mit einer Viskosität bei 25°C von 5.9 Pa s.
¹³C-NMR (CDCl₃): 0.15 (CH₃), 14.73 (CH₂), 20.02 (CH₃), 20.93 (CH₂), 21.50 (CH₃), 23.79, 24.06 und 24.71 (CH₂), 33.19 (CH₂), 43.30 (CH₂), 44.95 (CH₂), 59.36 (CH₂), 73.14-73.83 (NCH₂N).
FT-IR: 2961, 2927, 2854, 1615, 1448, 1430, 1373, 1318, 1257, 1078, 1016, 938, 861, 792, 701.

### Katalysator HHT-10

In einem Rundkolben wurden 2.38 g Amidin A1 und 3.23 g Guanidin G4 unter Stickstoffatmosphäre vermischt und mittels Eisbad gekühlt. Dann wurden 0.69 g Paraformaldehyd portionenweise eingerührt und die Mischung anschliessend unter Rühren während 1 Stunde auf 40°C erwärmt. Die Reaktionsmischung wurde dann auf Raumtemperatur abgekühlt, mit 15 mL Ethylacetat versetzt, mit Magnesiumsulfat getrocknet und filtriert. Darauf wurde das Reaktionsgemisch im Vakuum von den flüchtigen Bestandteilen befreit. Man erhielt 6.08 g eines geruchlosen, gelben Öls mit einer Viskosität bei 25 °C von 308.9 Pa s.
¹³C-NMR (CDCl₃): 14.15 (CH₃), 17.1-17.4 (CH₃), 19.25 (CH₃), 21.0 (CH₃), 21.96 (CH₂), 22.45 (CH₃), 24.97 und 25.10 (CH₂), 25.75 (CH₂), 28.94 und 29.07 (CH₂), 33.97-34.31 (CH₂), 44.33 (CH₂), 45.85 (CH₂), 49.12-49.31 (CH₂), 49.43 (CH), 51.65 (CH), 60.3 (CH₂), 75.15 (NCH₂N).
FT-IR: 3269, 2924, 2850, 1738, 1615, 1532, 1481, 1446, 1372, 1318, 1237, 1146, 1100, 1098, 1048, 943, 861, 785, 733.

### Katalysator HHT-11 @RCa928

In einem Rundkolben wurden 4.31 g Zwischenprodukt HZ-1 und 3.14 g Amidin A1 unter Stickstoffatmosphäre vermischt, 4 Stunden bei 150°C am Rückfluss gekocht und anschliessend im Vakuum die flüchtigen Bestandteile entfernt. Man erhielt 5.68 g eines geruchlosen, hellgelben, dünnflüssigen Öls.
¹H-NMR (CDCl₃): 0.83 (*t*, 6 H, J=7.4 Hz, CH₃), 1.41 (*sext,* 4 H, J=7.5 Hz, C*H*₂CH₃), 1.60 (*sept,* 2 H, J=7.1 Hz, NHₑₓₐₕCH₂C*H*₂CH₂N_{Amidin}), 1.75 (*m*, 2 H, N_{Amidin}CH₂C*H*₂CH₂N_{Amidin}), 1.92 (*s*, 3 H, N=CCH₃), 2.30 (*t*, 4 H, J=7.7 Hz, NC*H*₂CH₂CH₃), 2.64 (*t*, 2 H, J=7.0 Hz, N_{Hexah}C*H*₂CH₂CH₂), 3.10 und 3.14 (2 × *m*, 6 H, N_{Amidin}CH₂), 3.20 (br s, 6 H, NCH₂N).
FT-IR: 3272, 2926, 2850, 1612, 1422, 1376, 1317, 1292, 1203, 1114, 1100, 1085, 1009, 940, 878, 833, 780, 750.

### Katalysator HHT-12 @RCa927

In einem Rundkolben wurden 3.77 g Zwischenprodukt HZ-2 und 1.56 g Amidin A1 unter Stickstoffatmosphäre vermischt, 4 Stunden bei 150°C am Rückfluss gekocht und anschliessend im Vakuum die flüchtigen Bestandteile entfernt. Man erhielt 4.51 g eines geruchlosen, hellgelben, dünnflüssigen Öls.
¹H-NMR (CDCl₃): 0.56 (*m*, ca. 2.5 H, CH₂Si), 0.83 (*t*, ca. 4 H, J=7.4 Hz, CH₂CH₂C*H*₃), 1.15 (*t,* ca. 9 H, J=7.0 Hz, OCH₂C*H*₃), 1.40 (*quart,* ca. 2.5 H, J=7.5 Hz, CH₂C*H*₂CH₃), 1.50 (*m*, ca. 2.5 H, C*H*₂CH₂Si), 1.60 (*quint,* ca. 2 H, J=7.0 Hz, N_{Hexah}CH₂C*H*₂CH₂N_{Amidin}), 1.75 (*m*, ca. 2.5 H, N_{Amidin}CH₂C*H*₂CH₂-N_{Amidin}), 1.93 (*s*, ca. 3 H, N=CCH₃), 2.32 und 2.65 (*m*, ca. 5 H und *m*, ca. 1 H, N_{Hexah}C*H*₂CH₂), 3.1 (br *m*, ca. 5.5 H, N_{Amidin}CH₂), 3.20 (br *m*, ca. 6 H, NCH₂N), 3.74 (*quart,* ca. 6 H, J=7.0 Hz, OCH₂).
FT-IR: 3270, 2925, 2850, 1613, 1483, 1423, 1376, 1317, 1292, 1210, 1166, 1101, 1078, 1013, 943, 880, 769.

### Katalysator HHT-13 @RCa961

In einem Rundkolben wurden 3.59 g Zwischenprodukt HZ-2 und 2.89 g Guanidin G1 unter Stickstoffatmosphäre vermischt, 4 Stunden bei 150°C am Rückfluss gekocht und anschliessend im Vakuum die flüchtigen Bestandteile entfernt. Man erhielt 6.06 g eines geruchlosen, hellgelben, dünnflüssigen Öls.
FT-IR: 3270, 2924, 2851, 1613, 1504, 1448, 1388, 1363, 1344, 1236, 1202, 1102, 1076, 1009, 955, 888, 772.

### Herstellung einer nicht-erfindungsgemässen Hexahydrotriazin-Verbindung:

### Katalysator R-1

In einem Rundkolben wurden 7.62 g Paraformaldehyd unter Stickstoffatmosphäre vorgelegt und mittels Eisbad gekühlt. Dann wurden langsam 15.0 g Isopropylamin zugetropft und eingerührt und die Mischung anschliessend unter Rühren während 1 Stunde auf 40°C erwärmt. Die Reaktionsmischung wurde dann auf Raumtemperatur abgekühlt, mit 15 mL Ethylacetat versetzt, mit Magnesiumsulfat getrocknet und filtriert. Darauf wurde das Reaktionsgemisch im Vakuum von den flüchtigen Bestandteilen befreit. Man erhielt 13.14 g eines aminartig riechenden farblosen Öls.
¹H-NMR (CDCl₃): 0.99 (*d*, 18 H, J=6.3 Hz, CH₃), 2.79 (*sext,* 3 H, J=6.4 Hz, NCH), 3.46 (br s, 6 H, NCH₂N).
¹³C-NMR (CDCl₃): 10.85 (CH₃), 49.81 (CH), 68.46 (NCH₂N).
FT-IR: 2964, 2930, 2872, 2813, 1663, 1462, 1380, 1362, 1328, 1237, 1214, 1165, 1115, 1090, 1035, 1009, 988, 936, 888, 851, 785.

### Übersicht über die hergestellten Hexahydrotriazin-Verbindungen:

**Tabelle 1: Eingesetzte Edukte und ungefähre Molverhältnisse der Zwischenprodukte HZ-1 und HZ-2 und der Katalysatoren HHT-1 bis HHT-13 und R-1**

| **HZ-1** | 3 mol n-Propylamin | | 3 mol Formaldehyd |
|---|---|---|---|
| **HZ-2** | 1 mol Zwischenprodukt HZ-1 | | 1 mol AMEO |
| **HHT-1** | 1 mol Guanidin G2 | 2 mol Hexylamin | 3 mol Formaldehyd |
| **HHT-2** | 2 mol Guanidin G1 | 1 mol Guanidin G2 | 3 mol Formaldehyd |
| **HHT-3** | 2 mol Guanidin G1 | 1 mol Guanidin G4 | 3 mol Formaldehyd |
| **HHT-4** | 2 mol Guanidin G1 | 1 mol Guanidin G5 | 3 mol Formaldehyd |
| **HHT-5** | 2 mol Amidin A1 | 1 mol Wacker® Aminöl 446011-20 VP | 3 mol Formaldehyd |
| **HHT-6** | 4 mol Amidin A1 | 1 mol Jeffamine® D-230 | 6 mol Formaldehyd |
| **HHT-7** | 3 mol Amidin A1 | | 3 mol Formaldehyd |
| **HHT-8** | 2 mol Amidin A1 | 1 mol 3-Dimethylamino-1-propylamin | 3 mol Formaldehyd |
| **HHT-9** | 2 mol Amidin A1 | 1 mol Guanidin G3 | 3 mol Formaldehyd |
| **HHT-10** | 2 mol Amidin A1 | 1 mol Guanidin G4 | 3 mol Formaldehyd |
| **HHT-11** *@RCa928* | 1 mol Zwischenprodukt HZ-1 | | 1 mol Amidin A1 |
| **HHT-12** *@RCa927* | 1 mol Zwischenprodukt HZ-2 | | 1 mol Amidin A1 |
| **HHT-13** *@RCa961* | 1 mol Zwischenprodukt HZ-2 | | 1 mol Guanidin G1 |
| **R-1** | 3 mol Isopropylamin | | 3 mol Formaldehyd |

**Tabelle 2: Idealisierte Struktur¹ der Zwischenprodukte HZ-1 und HZ-2 und der Katalysatoren HHT-1 bis HHT-13 und R-1**

| | |
|---|---|
| **HZ-1** | 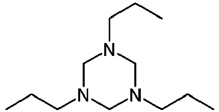 |
| **HZ-2** | 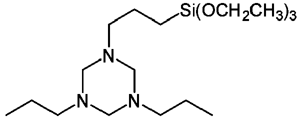 |
| **HHT-1** | 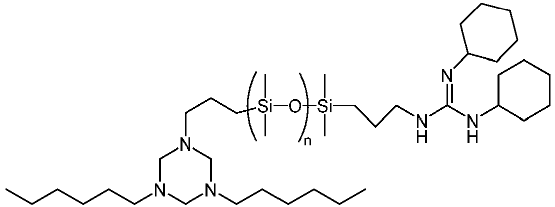 |
| **HHT-2** | 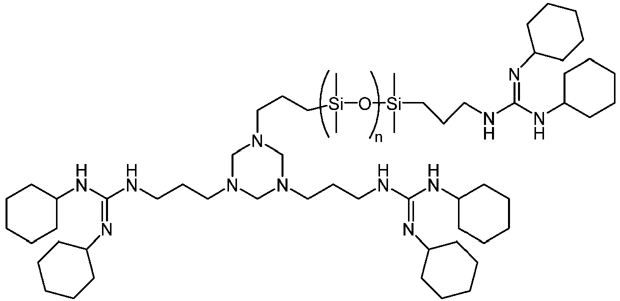 |
| **HHT-3** | 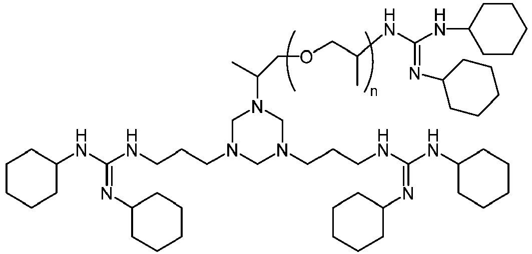 |
| **HHT-4** | 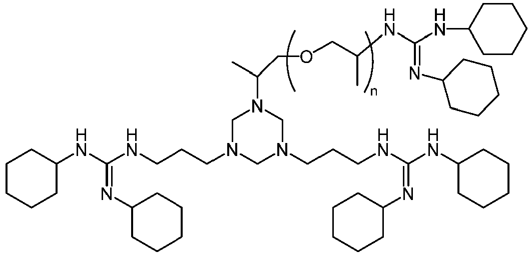 |
| **HHT-5** | 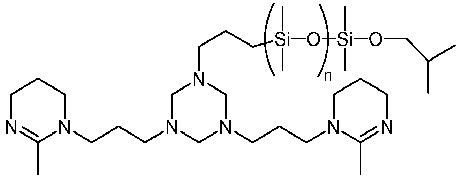 |
| **HHT-6** | 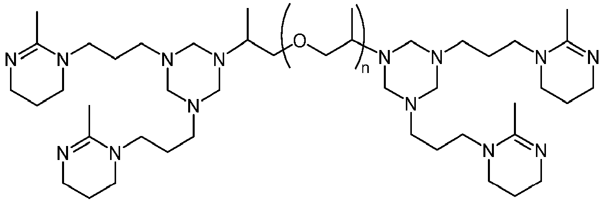 |
| **HHT-7** | 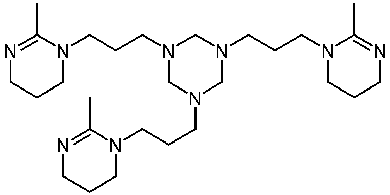 |
| **HHT-8** | 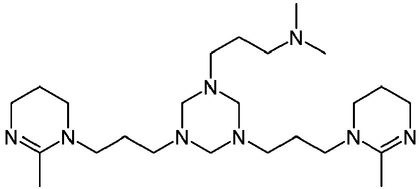 |
| **HHT-9** | 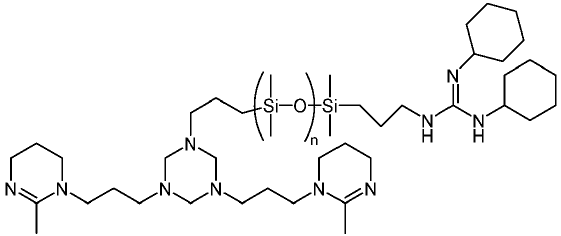 |
| **HHT-10** | 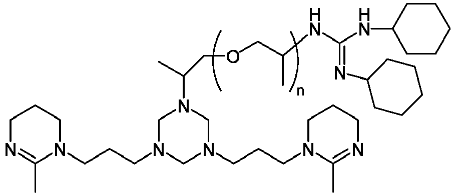 |
| **HHT-11** | 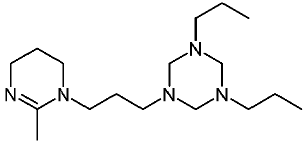 |
| **HHT-12** | 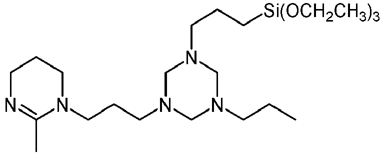 |
| **HHT-13** | 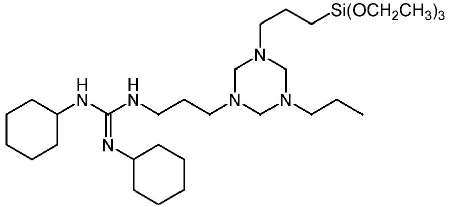 |
| **R-1** | 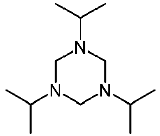 |

| | |
|---|---|
| ¹ Als "idealisierte Struktur" wird eine die stöchiometrischen Verhältnisse wiedergebende Struktur bezeichnet. Selbstverständlich enthält das Reaktionsprodukt neben dieser Verbindung Anteile von weiteren Reaktionsprodukten, insbesondere solchen, bei welchen die Substituenten an der Hexahydrotriazin-Einheit anders verteilt sind. | |

### Herstellung von Silangruppen aufweisenden Polyethern:

### Polymer STP-1:

Unter Feuchtigkeitsausschluss wurden 1000 g Polyol Acclaim® 12200 (Polyoxypropylendiol mit niedrigem Ungesättigtheitsgrad, von Covestro; OH-Zahl 11.0 mg KOH/g), 43.6 g Isophorondiisocyanat (IPDI; Vestanat® IPDI, von Evonik), 126.4 g Diisononyl-1,2-cyclohexandicarboxylat (DINCH) und 0.1 g Bismuttris(neodecanoat) (10 Gew.-% in DINCH) unter stetigem Rühren auf 90 °C aufgeheizt und auf dieser Temperatur belassen, bis der titrimetrisch bestimmte Gehalt an freien Isocyanatgruppen einen stabilen Wert von 0.63 Gew.-% erreicht hatte. Anschliessend wurden 63.0 g N-(3-Trimethoxysilylpropyl)aminobernsteinsäurediethylester (Addukt aus 3-Aminopropyltrimethoxysilan und Maleinsäurediethylester; hergestellt nach den Angaben in US 5,364,955) eingemischt und die Mischung bei 90°C solange gerührt, bis mittels FT-IR-Spektroskopie kein freies Isocyanat mehr nachgewiesen wurde. Der so erhaltene Trimethoxysilangruppen aufweisende Polyether mit einem Silan-Equivalentgewicht von ca. 6880 g/Eq (aus den Einsatzmengen berechnet) wurde auf Raumtemperatur abgekühlt und unter Ausschluss von Feuchtigkeit aufbewahrt.

### Verwendete kommerzielle Katalysatoren:

- Cat.41: Reaktionsprodukt von Kieselsäuretetraethylester mit Bis(acetyloxy)-dibutylstannan, Zinn-Gehalt ca. 23.8 Gewichts-% (Catalyst 41, von Wacker)
- DBU: 1,8-Diazabicyclo[5.4.0]undec-7-en (Lupragen® N 700, von BASF)
- TMHHT: 1,3,5-Trimethylhexahydro-1,3,5-triazin (Sigma-Aldrich)

### Zusammensetzungen auf Basis von Silangruppen aufweisenden Polymeren:

Vergleichsbeispiele sind in den Tabellen 3 bis 6 mit "(Ref)" gekennzeichnet.

### Zusammensetzungen Z1 bis Z11:

In einem Rundkolben wurden 71.1 g eines OH-terminierten linearen Polydimethylsiloxans mit einer Viskosität von ca. 50'000 mPas (Wacker® Silicone Rubber Polymer FD 50, von Wacker) bei Raumtemperatur mit 2.6 g Vinyl-tris(methylethylketoximo)silan vermengt und während 15 Minuten unter Vakuum gerührt. In das so erhaltene Polydimethylsiloxan mit Vinyl-bis(methylethylketoximo)silyl-Endgruppen wurden 26.3 g Trimethylsilyl-terminiertes Polydimethylsiloxan (Wacker® AK 100 Siliconöl, von Wacker) eingerührt. Diese Mischung wurde mit verschiedenen Katalysatoren gemäss nachstehender Tabelle 3 vermengt und die Mischung auf Viskosität bei 25°C und Hautbildungszeit (HBZ) im Normklima, vor und nach Lagerung, geprüft. Die Hautbildungszeit dient dabei als Mass für die Aktivität des Katalysators in Bezug auf die Vernetzungsreaktion der Silangruppen, d.h. für die Vernetzungsgeschwindigkeit; die Veränderung der Viskosität und der Hautbildungszeit nach Lagerung sind ein Mass für die Lagerstabilität der Zusammensetzung. Weiterhin wurde die applizierte Mischung nach 24 Stunden im Normklima daraufhin geprüft, ob die Oberfläche wie gewünscht trocken war oder sich ein schmieriger Film gebildet hatte, was ein Anzeichen für das Ausschwitzen des Katalysators aufgrund schlechter Verträglichkeit mit dem ausgehärteten Kunststoff ist, und/oder ob die Oberfläche klebrig war, was ein Anzeichen für eine unvollständige Aushärtung ist. Weiterhin wurde von der Mischung ein Film von 2 mm Dicke hergestellt, während 7 Tagen im Normklima ausgehärtet und auf mechanische Eigenschaften geprüft. Die Ergebnisse sind in Tabelle 3 und 4 wiedergegeben.

**Tabelle 3:**

| **Zusammensetzung** | **Katalysator** | **Menge** | **Konzentration¹** | **Viskosität [Pa·s]** | | **HBZ** | |
|---|---|---|---|---|---|---|---|
| | | | | **frisch** | **gelagert²** | **frisch** | **gelagert²** |
| **Z1** | HHT-1 | 0.57 g | 0.5 | 11.8 | 12.3 | 18' | 15' |
| **Z2** | HHT-4 | 0.15 g | 0.5 | 12.2 | 12.7 | 16' | 17' |
| **Z3** | HHT-6 | 0.09 g | 0.5 | 11.9 | 12.1 | 20' | 18' |
| **Z4** | HHT-7 | 0.06 g | 0.5 | 11.6 | 12.1 | 65' | 70' |
| **Z5** | HHT-8 | 0.09 g | 0.5 | 12.0 | 12.0 | 35' | 30' |
| **Z6** | HHT-9 | 0.18 g | 0.5 | 13.6 | 14.5 | 20' | 14' |
| **Z7** | HHT-10 | 0.10 g | 0.5 | 13.0 | 13.7 | 25' | 16' |
| **Z8** (Ref) | R-1 | 0.08 g | - | 12.6 | 12.8 | 2h 25' | 1h 56' |
| **Z9** (Ref) | TMHHT | 0.05 g | - | 11.9 | 18.4 | 2h 30' | 1h 58' |
| **Z10** (Ref) | - | - | - | 11.9 | 12.2 | 2h 36' | 2h 10' |
| **Z11** (Ref) | Cat.41 | 0.33 g | 1.1 | 11.7 | 48.1 | 27' | 32' |

| | | | | | | | |
|---|---|---|---|---|---|---|---|
| ¹ mmol Amidin- oder Guanidin-Gruppen oder Metallatome auf 100 g Ketoximato-Polydimethylsiloxan Polymer. ² während 7 Tagen bei 70 °C in geschlossenem Gebinde. | | | | | | | |

**Tabelle 4:**

| **Zusammensetzung** | **Oberfläche nach 24h** | **Zugfestigkeit** | **Bruchdehnung** | **E-Modul 0-25% Dehnung** |
|---|---|---|---|---|
| **Z1** | trocken | n.b. | n.b. | n.b. |
| **Z2** | trocken | 0.22 MPa | 183% | 0.18 MPa |
| **Z3** | trocken | 0.17 MPa | 126% | 0.18 MPa |
| **Z4** | trocken | 0.18 MPa | 103% | 0.18 MPa |
| **Z5** | trocken | 0.14 MPa | 81% | 0.17 MPa |
| **Z6** | trocken | 0.23 MPa | 234% | 0.18 MPa |
| **Z7** | trocken | 0.22 MPa | 129% | 0.20 MPa |
| **Z8** (Ref) | trocken | n.b. | n.b. | n.b. |
| **Z9** (Ref) | trocken | n.b. | n.b. | n.b. |
| **Z10** (Ref) | trocken | n.b. | n.b. | n.b. |
| **Z11** (Ref) | trocken | 0.24 MPa | 256% | 0.19 MPa |

| | | | | |
|---|---|---|---|---|
| "n.b." steht für "nicht bestimmt" | | | | |

### Zusammensetzungen Z12 bis Z25:

Eine Zusammensetzung aus 97.6 g Polymer STP-1, 2.0 g Vinyltrimethoxysilan und 0.4 g 3-Aminopropyltrimethoxysilan wurde mit verschiedenen Katalysatoren in der angegebenen Menge gemäss Tabelle 5 vermengt und wie für Zusammensetzung Z1 beschrieben auf Viskosität, Hautbildungszeit (HBZ), Oberflächenbeschaffenheit sowie mechanische Eigenschaften geprüft.

Die Ergebnisse sind in Tabelle 5 und 6 wiedergegeben.

**Tabelle 5:**

| **Zusammensetzung** | **Katalysator** | **Menge** | **Konzentration¹** | **Viskosität [Pa·s]** | | **HBZ** | |
|---|---|---|---|---|---|---|---|
| | | | | **frisch** | **gelagert²** | **frisch** | **gelagert²** |
| **Z12** | HHT-1 | 2.81 g | 1.9 | 28.3 | 44.0 | 17' | 11' |
| **Z13** | HHT-2 | 1.16 g | 1.9 | 29.5 | 58.9 | 12' | 8' |
| **Z14** | HHT-3 | 0.66 g | 1.9 | 28.4 | 61.3 | 12' | 8' |
| **Z15** | HHT-5 | 1.88 g | 1.9 | 28.8 | 45.3 | 25' | 31' |
| **Z16** | HHT-6 | 0.44 g | 1.9 | 51.5 | 53.5 | 25' | 22' |
| **Z17** | HHT-7 | 0.31 g | 1.9 | 40.9 | 50.0 | 26' | 30' |
| **Z18** | HHT-9 | 0.88 g | 1.9 | 40.8 | 52.9 | 14' | 16' |
| **Z19** | HHT-10 | 0.50 g | 1.9 | 40.8 | 52.9 | 18' | 20' |
| **Z20** | HHT-11 | 0.58 g | 1.9 | 17.9 | 24.2 | 24' | 26' |
| **Z21** | HHT-12 | 0.89 g | 1.9 | 17.6 | 24.0 | 24' | 27' |
| **Z22** | HHT-13 | 1.11 g | 1.9 | 29.9 | 52.1 | 8' | 6' |
| **Z23** (Ref) | R-1 | 0.40 g | - | 28.2 | 35.4 | 77' | 90' |
| **Z24** (Ref) | TMHHT | 0.24 g | - | 29.5 | 34.2 | 80' | 90' |
| **Z25** (Ref) | DBU | 0.36 g | 2.4 | 26.3 | 31.0 | 29' | 31' |

| | | | | | | | |
|---|---|---|---|---|---|---|---|
| ¹ mmol Amidin- oder Guanidin-Gruppen auf 100 g Silangruppen aufweisender Polyether. ² während 7 Tagen bei 70°C in geschlossenem Gebinde. | | | | | | | |

**Tabelle 6:**

| **Zusammensetzung** | **Oberfläche nach 24h** | **Zugfestigkeit** | **Bruchdehnung** | **E-Modul** | |
|---|---|---|---|---|---|
| | | | | **0-5%** | **0-50%** |
| **Z12** | trocken | 0.77 MPa | 126% | 1.22 MPa | 0.84 MPa |
| **Z13** | trocken | 0.75 MPa | 110% | 1.23 MPa | 0.85 MPa |
| **Z14** | trocken | 0.75 MPa | 104% | 1.25 MPa | 0.88 MPa |
| **Z15** | leicht schmierig | 0.68 MPa | 97% | 1.09 MPa | 0.82 MPa |
| **Z16** | trocken | 0.67 MPa | 96% | 1.20 MPa | 0.82 MPa |
| **Z17** | trocken | 0.75 MPa | 108% | 1.15 MPa | 0.87 MPa |
| **Z18** | trocken | 0.73 MPa | 100% | 1.11 MPa | 0.87 MPa |
| **Z19** | trocken | 0.72 MPa | 91% | 1.18 MPa | 0.86 MPa |
| **Z20** | trocken | 0.80 MPa | 102% | 1.30 MPa | 0.87 MPa |
| **Z21** | trocken | 0.73 MPa | 88% | 1.28 MPa | 0.87 MPa |
| **Z22** | trocken | 0.64 MPa | 76% | 1.37 MPa | 0.84 MPa |
| **Z23** (Ref) | trocken | 0.67 MPa | 94% | 1.30 MPa | 0.86 MPa |
| **Z24** (Ref) | trocken | 0.66 MPa | 88% | 1.21 MPa | 0.88 MPa |
| **Z25** (Ref) | schmierig | 0.70 MPa | 95% | 0.98 MPa | 0.79 MPa |

### Zweikomponentige Polyurethan-Zusammensetzungen:

### Vergleichsbeispiele sind in Tabelle 7 mit "(Ref)" gekennzeichnet.

### Zusammensetzungen Z26 bis Z29

Eine erste Komponente wurde hergestellt, indem 20.00 g Poly bd® R-45HTLO mit der in Tabelle 7 angegebenen Menge Katalysator vermischt wurde. Als zweite Komponente wurden 2.65 g Desmodur® CD eingesetzt.

Die beiden Komponenten wurden vermischt und sofort die Hautbildungszeit bestimmt.

Die Ergebnisse sind in Tabelle 7 wiedergegeben.

| **Zusammensetzung** | **Katalysator** | **Menge** | **Konzentration¹** | **HBZ** |
|---|---|---|---|---|
| **Z26** | HHT-8 | 0.08 g | 1.8 | 26' |
| **Z27** (Ref) | DBU | 0.03 g | 1.2 | 25' |
| **Z28** (Ref) | TMHHT | 0.03 g | - | 62' |
| **Z29** (Ref) | - | - | - | 65' |

| | | | | |
|---|---|---|---|---|
| ¹ mmol Amidin- oder Guanidin-Gruppen auf 100 g Polyol. | | | | |

## Patentansprüche

1. Verbindung enthaltend mindestens eine Hexahydrotriazin-Einheit der Formel (I), 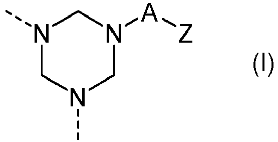 wobei
A für einen zweiwertigen Kohlenwasserstoff-Rest, welcher gegebenenfalls Heteroatome enthält, steht, und
Z für eine über ein Stickstoffatom gebundene Amidin- oder Guanidingruppe steht,
wobei der Hexahydrotriazin-Ring und die Gruppe Z durch eine Kette von mindestens zwei C-Atomen voneinander getrennt sind.

2. Verbindung gemäss Anspruch 1, **dadurch gekennzeichnet, dass** A für einen zweiwertigen Kohlenwasserstoff-Rest mit 2 bis 50 C-Atomen, welcher gegebenenfalls Heteroatome in Form von Ether-Sauerstoff oder sekundärem oder tertiärem Amin-Stickstoff oder Siloxan-Einheiten enthält, steht.

3. Verbindung gemäss einem der Ansprüche 1 oder 2, **dadurch gekennzeichnet, dass** Z für 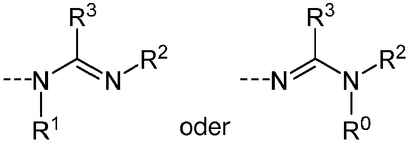 steht, wobei
R⁰ für einen Wasserstoff-Rest oder für einen Alkyl- oder Cycloalkyl- oder Aralkyl-Rest mit 1 bis 8 C-Atomen steht,
R¹ für einen Wasserstoff-Rest oder für einen Alkyl- oder Cycloalkyl- oder Aralkyl-Rest mit 1 bis 8 C-Atomen oder zusammen mit R² für R⁶ steht,
R² für einen Wasserstoff-Rest oder für einen Alkyl-, Cycloalkyl- oder Aralkyl-Rest mit 1 bis 18 C-Atomen, welcher gegebenenfalls Ether-Sauerstoff oder tertiären Amin-Stickstoff enthält, oder zusammen mit R¹ für R⁶ steht, R³ für -NR⁴R⁵ oder für einen Wasserstoff-Rest oder für einen Alkyl- oder Cycloalkyl- oder Aralkyl-Rest mit 1 bis 12 C-Atomen steht,
wobei
R⁴ und R⁵ unabhängig voneinander jeweils für einen Wasserstoff-Rest oder für einen Alkyl-, Cycloalkyl- oder Aralkyl-Rest mit 1 bis 18 C-Atomen, welcher gegebenenfalls Ether-Sauerstoff oder tertiären Amin-Stickstoff enthält, stehen,
R⁶ für einen gegebenenfalls substituierten 1,2-Ethylen-, 1,3-Propylen- oder 1,4-Butylen-Rest mit 2 bis 12 C-Atomen steht,
R² und R⁰ auch zusammen für einen Alkylen-Rest mit 3 bis 6 C-Atomen, welcher gegebenenfalls Ether-Sauerstoff oder tertiären Amin-Stickstoff enthält, stehen können,
R² und R³ auch zusammen für einen Alkylen-Rest mit 3 bis 6 C-Atomen stehen können,
R⁴ und R⁵ auch zusammen für einen Alkylen-Rest mit 4 bis 7 C-Atomen, welcher gegebenenfalls Ether-Sauerstoff oder tertiären Amin-Stickstoff enthält, stehen können, und
R² und R⁵ auch zusammen für einen Alkylen-Rest mit 2 bis 12 C-Atomen stehen können.

4. Verbindung gemäss Anspruch 3, **dadurch gekennzeichnet, dass** R³ für einen Wasserstoff-Rest oder für einen Alkyl-Rest mit 1 bis 4 C-Atomen steht, und R¹ und R² zusammen für R⁶ stehen.

5. Verbindung gemäss Anspruch 3, **dadurch gekennzeichnet, dass** R³ für -NR⁴R⁵ steht, R¹, R⁰ und R⁴ jeweils für einen Wasserstoff-Rest stehen, und R² und R⁵ unabhängig voneinander jeweils für einen Alkyl-, Cycloalkyl- oder Aralkyl-Rest mit 1 bis 12 C-Atomen, welcher gegebenenfalls einen Ether-Sauerstoff oder tertiären Amin-Stickstoff enthält, stehen.

6. Verbindung gemäss einem der Ansprüche 1 bis 5, **dadurch gekennzeichnet, dass** sie ausgewählt ist aus Verbindungen der Formel (II) und Verbindungen der Formel (III), 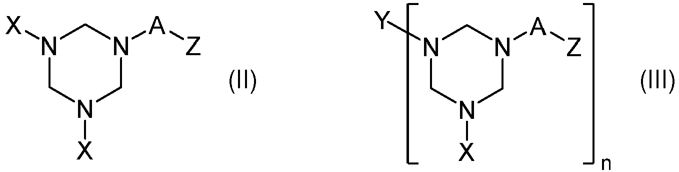 wobei
X für -A-Z oder für einen einwertigen Kohlenwasserstoff-Rest mit 1 bis 30 C-Atomen, welcher gegebenenfalls Heteroatome enthält, steht,
Y für einen n-wertigen Kohlenwasserstoff-Rest mit 2 bis 30 C-Atomen, welcher gegebenenfalls Heteroatome enthält, steht, und
n für 2 oder 3 steht,
wobei im Fall von Verbindungen der Formel (III) die Hexahydrotriazin-Ringe jeweils durch eine Kette von mindestens zwei C-Atomen voneinander getrennt sind.

7. Verbindung gemäss Anspruch 6, **dadurch gekennzeichnet, dass** X für einen Rest ausgewählt aus -A-Z, Methyl, Ethyl, Propyl, Isopropyl, Butyl, Isobutyl, sec-Butyl, tert.Butyl, Hexyl, Octyl, 2-Ethylhexyl, Cyclohexyl, Benzyl, Methoxyethyl, Methoxyethoxyethyl, Trimethoxysilylpropyl, Triethoxysilylpropyl und ω-Alkoxy-poly(dimethylsiloxan)prop-3-yl mit einem mittleren Molekulargewicht im Bereich von etwa 350 bis 2'000 g/mol steht.

8. Verfahren zur Herstellung der Verbindung gemäss einem der Ansprüche 1 bis 7, **dadurch gekennzeichnet, dass** mindestens ein Amin der Formel H₂N-A-Z und gegebenenfalls mindestens ein weiteres primäres Amin mit Formaldehyd oder einer Formaldehyd freisetzenden Verbindung, unter Entfernung von Wasser umgesetzt wird.

9. Verwendung der Verbindung gemäss einem der Ansprüche 1 bis 7 als Katalysator für die Vernetzung einer funktionellen Verbindung.

10. Verwendung gemäss Anspruch 9, **dadurch gekennzeichnet, dass** die funktionelle Verbindung ein Polyisocyanat oder ein Isocyanatgruppen aufweisendes Polyurethanpolymer oder ein Silangruppen aufweisendes Polymer ist.

11. Verwendung gemäss Anspruch 10, **dadurch gekennzeichnet, dass** die funktionelle Verbindung ein Silangruppen aufweisendes Polymer ausgewählt aus der Gruppe bestehend aus Polyorganosiloxanen mit endständigen Silangruppen und Silangruppen aufweisenden organischen Polymeren ist.

12. Härtbare Zusammensetzung enthaltend mindestens eine Verbindung gemäss einem der Ansprüche 1 bis 7 als Katalysator.

13. Härtbare Zusammensetzung gemäss Anspruch 12, **dadurch gekennzeichnet, dass** sie Isocyanatgruppen und/oder Silangruppen enthält.

14. Härtbare Zusammensetzung gemäss einem der Ansprüche 12 oder 13, **dadurch gekennzeichnet, dass** sie einen Klebstoff oder einen Dichtstoff oder eine Beschichtung darstellt.

15. Härtbare Zusammensetzung gemäss einem der Ansprüche 12 bis 14, **dadurch gekennzeichnet, dass** sie mindestens einen weiteren Bestandteil ausgewählt aus der Gruppe bestehend aus Füllstoffen, Weichmachern, Rheologie-Additiven, Trocknungsmitteln, Haftvermittlern und Vernetzern enthält.

## Claims

1. Compound containing at least one hexahydrotriazine unit of the formula (I) 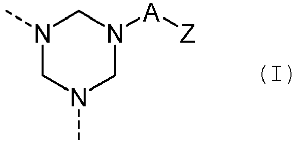 where
A is a divalent hydrocarbyl radical optionally containing heteroatoms, and
Z is an amidine or guanidine group bonded via a nitrogen atom,
where the hexahydrotriazine ring and the Z group are separated from one another by a chain of at least two carbon atoms.

2. Compound according to Claim 1, **characterized in that** A is a divalent hydrocarbyl radical which has 2 to 50 carbon atoms and optionally contains heteroatoms in the form of ether oxygen or secondary or tertiary amine nitrogen or siloxane units.

3. Compound according to either of Claims 1 and 2, **characterized in that** Z is 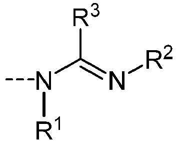 or 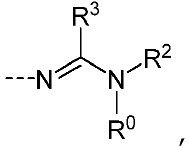 where
R⁰ is a hydrogen radical or an alkyl or cycloalkyl or aralkyl radical having 1 to 8 carbon atoms,
R¹ is a hydrogen radical or an alkyl or cycloalkyl or aralkyl radical having 1 to 8 carbon atoms or together with R² is R⁶,
R² is a hydrogen radical or an alkyl, cycloalkyl or aralkyl radical which has 1 to 18 carbon atoms and optionally contains ether oxygen or tertiary amine nitrogen, or together with R¹ is R⁶,
R³ is -NR⁴R⁵ or a hydrogen radical or an alkyl or cycloalkyl or aralkyl radical having 1 to 12 carbon atoms,
where
R⁴ and R⁵ are each independently a hydrogen radical or an alkyl, cycloalkyl or aralkyl radical which has 1 to 18 carbon atoms and optionally contains ether oxygen or tertiary amine nitrogen,
R⁶ is an optionally substituted 1,2-ethylene, 1,3-propylene or 1,4-butylene radical having 2 to 12 carbon atoms,
R² and R⁰ together may also be an alkylene radical which has 3 to 6 carbon atoms and optionally contains ether oxygen or tertiary amine nitrogen, R² and R³ together may also be an alkylene radical having 3 to 6 carbon atoms,
R⁴ and R⁵ together may also be an alkylene radical which has 4 to 7 carbon atoms and optionally contains ether oxygen or tertiary amine nitrogen, and
R² and R⁵ together may also be an alkylene radical having 2 to 12 carbon atoms.

4. Compound according to Claim 3, **characterized in that** R³ is a hydrogen radical or an alkyl radical having 1 to 4 carbon atoms, and R¹ and R² together are R⁶.

5. Compound according to Claim 3, **characterized in that** R³ is -NR⁴R⁵, R¹, R⁰ and R⁴ are each a hydrogen radical, and R² and R⁵ are each independently an alkyl, cycloalkyl or aralkyl radical which has 1 to 12 carbon atoms and optionally contains an ether oxygen or tertiary amine nitrogen.

6. Compound according to any of Claims 1 to 5, **characterized in that** it is selected from compounds of the formula (II) and compounds of the formula (III) 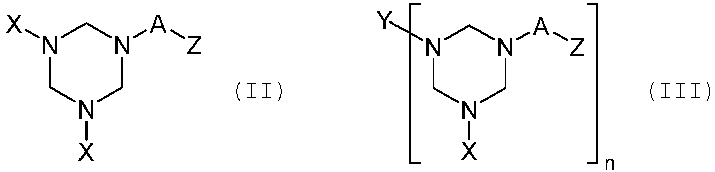 where
X is -A-Z or a monovalent hydrocarbyl radical which has 1 to 30 carbon atoms and optionally contains heteroatoms,
Y is an n-valent hydrocarbyl radical which has 2 to 30 carbon atoms and optionally contains heteroatoms, and
n is 2 or 3,
where, in the case of compounds of the formula (III), the hexahydrotriazine rings are each separated from one another by a chain of at least two carbon atoms.

7. Compound according to Claim 6, **characterized in that** X is a radical selected from -A-Z, methyl, ethyl, propyl, isopropyl, butyl, isobutyl, sec-butyl, tert-butyl, hexyl, octyl, 2-ethylhexyl, cyclohexyl, benzyl, methoxyethyl, methoxyethoxyethyl, trimethoxysilylpropyl, triethoxysilylpropyl and □ - alkoxypoly(dimethylsiloxane)prop-3-yl having an average molecular weight in the range from about 350 to 2'000 g/mol.

8. Process for preparing the compound according to any of Claims 1 to 7, **characterized in that** at least one amine of the formula H₂N-A-Z and optionally at least one further primary amine are reacted with formaldehyde or a formaldehyde-releasing compound with removal of water.

9. Use of the compound according to any of Claims 1 to 7 as catalyst for the crosslinking of a functional compound.

10. Use according to Claim 9, **characterized in that** the functional compound is a polyisocyanate or a polyurethane polymer having isocyanate groups or a polymer having silane groups.

11. Use according to Claim 10, **characterized in that** the functional compound is a polymer having silane groups selected from the group consisting of polyorganosiloxanes having terminal silane groups and organic polymers having silane groups.

12. Curable composition comprising at least one compound according to any of Claims 1 to 7 as catalyst.

13. Curable composition according to Claim 12, **characterized in that** it contains isocyanate groups and/or silane groups.

14. Curable composition according to either of Claims 12 and 13, **characterized in that** it is an adhesive or a sealant or a coating.

15. Curable composition according to any of Claims 12 to 14, **characterized in that** it comprises at least one further constituent selected from the group consisting of fillers, plasticizers, rheology additives, desiccants, adhesion promoters and crosslinkers.

## Revendications

1. Composé contenant au moins un motif de type hexahydrotriazine de formule (I), 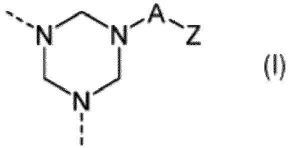
A représentant un radical hydrocarboné divalent, qui contient éventuellement des hétéroatomes, et
Z représentant un groupe amidine ou guanidine lié par l'intermédiaire d'un atome d'azote,
le cycle hexahydrotriazine et le groupe Z étant séparés l'un de l'autre par une chaîne d'au moins deux atomes de C.

2. Composé selon la revendication 1, **caractérisé en ce que** A représente un radical hydrocarboné divalent comportant 2 à 50 atomes de C, qui contient éventuellement des hétéroatomes sous forme d'oxygène d'éther ou d'azote d'amine secondaire ou tertiaire ou de motifs de type siloxane.

3. Composé selon l'une quelconque des revendications 1 et 2, **caractérisé en ce que** Z représente 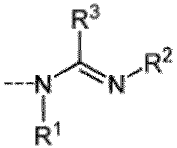 ou 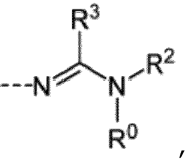
R⁰ représentant un radical hydrogène ou un radical alkyle ou cycloalkyle ou aralkyle comportant 1 à 8 atomes de C,
R¹ représentant un radical hydrogène ou un radical alkyle ou cycloalkyle ou aralkyle comportant 1 à 8 atomes de C ou, conjointement avec R², représentant R6,
R² représentant un radical hydrogène ou un radical alkyle ou cycloalkyle ou aralkyle comportant 1 à 18 atomes de C, qui contient éventuellement un oxygène d'éther ou un azote d'amine tertiaire ou, conjointement avec R¹, représentant R⁶,
R³ représentant -NR⁴R⁵ ou un radical hydrogène ou un radical alkyle ou cycloalkyle ou aralkyle comportant 1 à 12 atomes de C,
R⁴ et R⁵ représentant indépendamment l'un de l'autre à chaque fois un radical hydrogène ou un radical alkyle ou cycloalkyle ou aralkyle comportant 1 à 18 atomes de C, qui contient éventuellement un oxygène d'éther ou un azote d'amine tertiaire,
R⁶ représentant un radical 1,2-éthylène, 1,3-propylène ou 1,4-butylène éventuellement substitué comportant 2 à 12 atomes de C,
R² et R⁰ pouvant également représenter ensemble un radical alkylène comportant 3 à 6 atomes de C, qui contient éventuellement un oxygène d'éther ou un azote d'amine tertiaire,
R² et R³ pouvant également représenter ensemble un radical alkylène comportant 3 à 6 atomes de C,
R⁴ et R⁵ pouvant également représenter ensemble un radical alkylène comportant 4 à 7 atomes de C, qui contient éventuellement un oxygène d'éther ou un azote d'amine tertiaire, et
R² et R⁵ pouvant également représenter ensemble un radical alkylène comportant 2 à 12 atomes de C.

4. Composé selon la revendication 3, **caractérisé en ce que** R³ représente un radical hydrogène ou un radical alkyle comportant 1 à 4 atomes de C, et R¹ et R² représentent ensemble R⁶.

5. Composé selon la revendication 3, **caractérisé en ce que** R³ représente -NR⁴R⁵, R¹, R⁰ et R⁴ représentant à chaque fois un radical hydrogène, et R² et R⁵ représentent indépendamment l'un de l'autre à chaque fois un radical alkyle, cycloalkyle ou aralkyle comportant 1 à 12 atomes de C, qui contient éventuellement un oxygène d'éther ou un azote d'amine tertiaire.

6. Composé selon l'une quelconque des revendications 1 à 5, **caractérisé en ce qu'**il est choisi parmi des composés de formule (II) et les composés de formule (III) 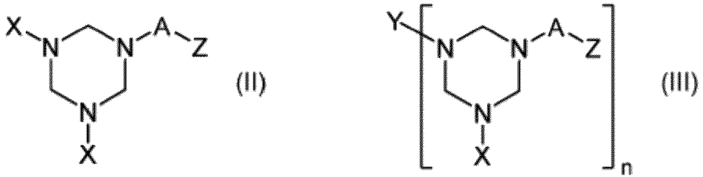
X représentant -A-Z ou un radical hydrocarboné monovalent comportant 1 à 30 atomes de C, qui contient éventuellement des hétéroatomes,
Y représentant un radical hydrocarboné n-valent comportant 2 à 30 atomes de C, qui contient éventuellement des hétéroatomes, et
n représentant 2 ou 3,
dans lequel dans le cas de composés de formule (III), les cycles hexahydrotriazine sont à chaque fois séparés par une chaîne d'au moins deux atomes de C.

7. Composé selon la revendication 6, **caractérisé en ce que** X représente un radical choisi parmi -A-Z, méthyle, éthyle, propyle, isopropyle, butyle, isobutyle, sec-butyle, tert-butyle, hexyle, octyle, 2-éthylhexyle, cyclohexyle, benzyle, méthoxyéthyle, méthoxyéthoxyéthyle, triméthoxysilylpropyle, triéthoxysilylpropyle et ω-alcoxy-poly(diméthylsiloxane)prop-3-yle doté d'un poids moléculaire moyen dans la plage d'environ 350 à 2 000 g/mole.

8. Procédé pour la préparation du composé selon l'une quelconque des revendications 1 à 7, **caractérisé en ce qu'**au moins une amine de formule H₂N-A-Z et éventuellement au moins une amine primaire supplémentaire est transformée avec du formaldéhyde ou avec un composé libérant du formaldéhyde, avec l'élimination d'eau.

9. Utilisation du composé selon l'une quelconque des revendications 1 à 7 en tant que catalyseur pour la réticulation d'un composé fonctionnel.

10. Utilisation selon la revendication 9, **caractérisée en ce que** le composé fonctionnel est un polyisocyanate ou un polymère de type polyuréthane présentant des groupes isocyanate ou un polymère présentant des groupes silane.

11. Utilisation selon la revendication 10, **caractérisée en ce que** le composé fonctionnel est un polymère présentant des groupes silane choisi dans le groupe constitué par des polyorganosiloxanes comportant des groupes silane terminaux et des polymères organiques présentant des groupes silane.

12. Composition durcissable contenant au moins un composé selon l'une quelconque des revendications 1 à 7 en tant que catalyseur.

13. Composition durcissable selon la revendication 12, **caractérisée en ce qu'**elle contient des groupes isocyanate et/ou des groupes silane.

14. Composition durcissable selon l'une quelconque des revendications 12 et 13, **caractérisée en ce qu'**elle représente un adhésif ou un agent d'étanchéité ou un revêtement.

15. Composition durcissable selon l'une quelconque des revendications 12 à 14, **caractérisée en ce qu'**elle contient au moins un ingrédient supplémentaire choisi dans le groupe constitué par des charges, des plastifiants, des additifs de rhéologie, des agents desséchants, des promoteurs d'adhérence et des agents de réticulation.
